# EUROPEAN PATENT APPLICATION

(11) **EP 4 696 705 A1**
(43) Date of publication of application: **18.02.2026**
(21) Application number: 24194393.5
(22) Date of filing: 13.08.2024
(51) Int. Cl.: C07K 14/47, A61K 38/17, A61K 48/00, A61P 25/00, A61P 25/14, A61P 43/00, C12N 9/10, C12N 9/22, C12N 15/113

(54) **ENGINEERED TRANSCRIPTION REPRESSORS AND USES THEREOF**

(71) Applicant: Fondazione Telethon ETS, 00185 Roma (IT); Ospedale San Raffaele S.r.l., 20132 Milano (MI) (IT)
(72) Inventor: Lombardo, Angelo, 20132 Milano (IT); Cappelluti, Martino Alfredo, 20132 Milano (IT)
(74) Representative: Manfredi, Irene

(57) **Abstract**

The present invention aims to treat polyglutamine diseases by silencing the expression of pathogenic alleles using engineered transcription repressors (ETRs). The provided ETRs deposit one or more epigenetic marks that can silence a pathogenic allele.

## Description

### Technical field

The present invention relates to the development of novel therapeutics as well as their use in the treatment of polyglutamine diseases such a Huntington's disease (HD) and Spinocerebellar ataxia (SCA).

### Background art

Trinucleotide repeat disorders consist of more than 30 human diseases caused by trinucleotide repeat expansion (Boivin & Charlet-Berguerand, 2022. Front Genet. 13:843014), a kind of mutation in which repeats of three nucleotides (trinucleotide repeats) increase in copy numbers until they cross a threshold above which they cause developmental, neurological, or neuromuscular disorders. Depending on its location, the unstable trinucleotide repeat may cause protein defects, changes in gene expression, and/or produce a toxic RNA or protein. In over half of trinucleotide repeat disorders, the repeated trinucleotide is a CAG. In a coding region, CAG codes for glutamine (Q), so CAG repeats result in an expanded polyglutamine tract. These diseases are commonly referred to as polyglutamine (or polyQ) diseases (see **Table 1** as well as Estevam et al., 2023. Database (Oxford). 2023:baad060).

**Table 1: Polyglutamine (PolyQ) diseases.**

| **Type** | **Gene name** | **Normal PolyQ repeats** | **Pathogenic PolyQ repeats** |
|---|---|---|---|
| DRPLA (Dentatorubropallidoluysian atrophy) | *ATN1* | 6 - 35 | 49 - 88 |
| HD (Huntington's disease) | *HTT* | 6 - 35 | 36 - 250 |
| SBMA (Spinal and bulbar muscular atrophy) | *AR* | 4 - 34 | 35 - 72 |
| SCA1 (Spinocerebellar ataxia Type 1) | *ATXN1* | 6 - 35 | 49 - 88 |
| SCA2 (Spinocerebellar ataxia Type 2) | *ATXN2* | 14 - 32 | 33 - 77 |
| SCA3 (Spinocerebellar ataxia Type 3 or Machado-Joseph disease) | *ATXN3* | 12 - 40 | 55 - 86 |
| SCA6 (Spinocerebellar ataxia Type 6) | *CACNA1A* | 4 - 18 | 21 - 30 |
| SCA7 (Spinocerebellar ataxia Type 7) | *ATXN7* | 7 - 17 | 38 - 120 |
| SCA17 (Spinocerebellar ataxia Type 17) | *TBP* | 25 - 42 | 47 - 63 |

Huntington's disease (HD) is an autosomal-dominant neurodegenerative disease caused by the expansion of a CAG repeat in exon 1 of the Huntingtin gene (*HTT*) (see Ross et al., 2014. Nat Rev Neurol. 10(4):204-16). In healthy individuals, *HTT* is polymorphic, containing a number of CAGs ranging from 10 to 35, with an average of 20 often interrupted by a CAA trinucleotide. Conversely, pathologically expanded *HTT* alleles present CAG expansion longer than 37, with the most prevalent ones ranging around 37 to 39 (Reiner et al., 2011. Int Rev Neurobiol. 98:325-72). While the exact pathogenetic role of the mutated *HTT* is still unclear (as well as the function of the wild-type protein), a substantial body of evidence indicates that the poly-glutamine stretch encoded by the extended repeat triggers toxic protein aggregation, finally leading to neural dysfunction and cell death (Tabrizi et al., 2020. Nat Rev Neurol. 16(10):529-546).

Spinocerebellar ataxia Type 2 (SCA2) is an autosomal dominant disease caused by the expansion of a CAG repeat in exon 1 of the *Ataxin-2* gene (*ATXN2*). In non-pathologic conditions, *ATXN2* harbors the sequence (CAG)₁₃(CAA)₁(CAG)₉ - referred to as (CAG)₂₂ -, which encodes for a 22-long polyglutamine stretch. Conversely, pathologically expanded alleles present expansion longer than 31 CAGs, not interrupted by the CAA trinucleotide, with the most prevalent ones harboring 37 to 39 CAG repetitions (Paulson et al., 2017. Nat Rev Neurosci. 18(10):613-626). Although the pathogenetic mechanism induced by CAG expansion is still unclear, it has been postulated that the poly-glutamine stretch triggers protein aggregation, finally leading to neural cell death (Velázquez-Pérez et al., 2017. Front Neurol. 8:472).

In the last two decades, several gene therapy approaches have been proposed for the treatment of HD and SCA2. Among these, post-transcriptional knock-down (KD) with Antisense Oligonucleotide (ASO) represents the most advanced one (Aguiar et al., 2017. Transl Neurodegener. 6:30). Indeed, pre-clinical testing in small and large animal models resulted in significant reduction of *HTT* and sustained amelioration of disease-associated phenotypes (Kordasiewicz et al., 2012. Neuron. 74(6):1031-44). Similar studies demonstrated that post-transcriptional KD of *Atxn2* by ASO can improve motor functions in SCA2 mouse models (Scoles et al., 2017. Nature. 544(7650):362-366). Overall, these data support clinical translation of ASOs for both HD and SCA2.

A general problem of the ASO technology is the need for frequent administration (on a monthly base) through invasive and potentially risky intrathecal medical procedures. A potential solution to this problem may come from the use of viral-derived vectors that stably express RNA interfering molecules against *HTT.* Supported by promising pre-clinical data, these approaches are now entering clinical testing (Vall6s et al., 2021. Sci TranslMed. 13(588):eabb8920).

Given the limitations associated with ASOs, there is a continuous quest in the field for more efficient and precise gene therapy approaches for polyQ diseases.

Targeted Epigenetic silencing (hereafter referred as to epi-silencing) with Engineered Transcription repressors (ETRs) allows for durable silencing of a gene (Amabile et al., 2016. Cell. 167(1):219-232; Mlambo et al., 2018. Nucleic Acids Res. 46(9):4456-4468; Nuñez et al., 2021. Cell. 184(9):2503-2519.e17; Cappelluti et al., 2024. Nature. 627(8003):416-423). Indeed, this approach allows to achieve permanent repression of genes of therapeutic relevance upon transient delivery of the ETRs.

Three relevant features of the Epi-silencing technology are (i) its hit-and-run mode of action, (ii) its high specificity profile (a feature enforced by the combinatorial nature of the technology), and (iii) its modularity (which allows specific gene targeting by programming their DNA-binding domains).

The present invention aims to provide safe and effective medicaments for the treatment of polyQ diseases.

### Summary of the invention

The present invention provides a composition comprising one or more nucleic acid encoding at least a transcriptional repressor domain (TRD) and a polynucleotide-binding domain, wherein the TRD is operably linked to the polynucleotide-binding domain when expressed, and wherein the polynucleotide-binding domain is a DNA-binding domain (e.g., a ZFP or TALE) that specifically binds to a sequence contained in a polyglutamine disease-related gene, or the polynucleotide-binding domain is a dCas protein and the composition further comprises a gRNA that specifically binds to a sequence contained in a polyglutamine disease-related gene, or a nucleic acid encoding a gRNA that specifically binds to a sequence contained in a polyglutamine disease-related gene. The polyglutamine disease-related gene may be *ATN1*, *HTT*, *AR*, *ATAN1*, *ATXN2*, *ATXN3*, *CACNA1A*, *ATXN7*, or *TBP.*

In some embodiments, the composition comprises one or more nucleic acid encoding at least:
(a) one or more TRD;
(b) a first DNA-binding domain (e.g., a ZFP or TALE), wherein one or more TRD is operably linked to the first DNA-binding domain when expressed; and
(c) a second DNA-binding domain (e.g., a ZFP or TALE), wherein one or more TRD is operably linked to the second DNA-binding domain when expressed,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene.

In some embodiments, the composition comprises one or more nucleic acids encoding at least:
(a) two or more TRDs;
(b) a first DNA-binding domain (e.g., a ZFP or TALE), wherein one or more TRD is operably linked to the first DNA-binding domain when expressed; and
(c) a second DNA-binding domains (e.g., a ZFP or TALE), wherein one or more TRD is operably linked to the second DNA-binding domains when expressed,
wherein the first and second DNA-binding domains specifically bind to sequences contained in a regulatory region of the polyglutamine disease-related gene or to sequences contained in the poly(CAG) tract of the polyglutamine disease-related gene. In some embodiments, all DNA-binding domains encoded by the one or more nucleic acid of the composition specifically bind to sequences contained in a regulatory region of the polyglutamine disease-related gene. In some embodiments, all DNA-binding domains encoded by the one or more nucleic acid of the composition specifically bind to sequences contained in the poly(CAG) tract of the polyglutamine disease-related gene.

In some embodiments, the composition comprises:
(a) one or more nucleic acid encoding at least a TRD and a dCas protein, wherein the TRD is operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene.

In some embodiments, the composition comprises:
(a) one or more nucleic acid encoding at least a TRD and a dCas protein, wherein the TRD is operably linked to the dCas protein when expressed;
(b) a first guide RNA (gRNA) or a nucleic acid encoding the first gRNA; and
(c) a second gRNA or a nucleic acid encoding the second gRNA,
wherein the first and second gRNAs specifically bind to sequences contained in a regulatory region of the polyglutamine disease-related gene or to sequences contained in the poly(CAG) tract of the polyglutamine disease-related gene. In some embodiments, all gRNAs encoded by a nucleic acid of the composition or comprised in the composition specifically bind to sequences contained in a regulatory region of the polyglutamine disease-related gene. In some embodiments, all gRNAs encoded by a nucleic acid of the composition or comprised in the composition specifically bind to sequences contained in the poly(CAG) tract of the polyglutamine disease-related gene.

In some embodiments, the composition comprises one or more nucleic acid encoding at least a TRD that increases repressive histone modifications, a TRD that increases DNA methylation, and a first DNA-binding domain (e.g., a ZFP or TALE) that specifically binds to a sequence contained in a polyglutamine disease-related gene, wherein the TRDs are operably linked to the first DNA-binding domain when expressed.

In some embodiments, the composition comprises:
(a) one or more nucleic acid encoding at least a TRD that increases repressive histone modifications, a TRD that increases DNA methylation, and a dCas protein, wherein the TRDs are operably linked to the dCas protein when expressed; and
(b) a first gRNA that specifically binds to a sequence contained in a polyglutamine disease-related gene, or a nucleic acid encoding the first gRNA.

In some embodiments, the composition comprises one or more nucleic acid encoding at least:
(a) a TRD that increases repressive histone modifications (e.g., a KRAB domain) and a first DNA-binding domain (e.g., a ZFP or TALE) that specifically binds to a sequence contained in a polyglutamine disease-related gene, wherein the TRD is operably linked to the first DNA-binding domain when expressed, and
(b) a TRD that increases DNA methylation (e.g., DNMT3L).

In some embodiments, the composition comprises:
(a) one or more nucleic acid encoding at least:
   (i) a TRD that increases repressive histone modifications (e.g., a KRAB domain) and a dCas protein, wherein the TRD is operably linked to the dCas protein when expressed, and
   (ii) a TRD that increases DNA methylation (e.g., DNMT3L); and
(b) a first gRNA that specifically binds to a sequence contained in a polyglutamine disease-related gene, or a nucleic acid encoding the first gRNA.

The present invention also provides a composition of the present invention for use as a medicament. The present invention also provides a composition of the present invention for use in the treatment of a polyglutamine disease.

The present invention also provides a method of reducing the expression of a polyglutamine disease-related gene (e.g., a pathogenic allele), the method comprising contacting a biological system (e.g., a cell, organ or subject) with a composition of the present invention.

The present invention also provides a method of treating a subject having a polyglutamine disease, the method comprising administering a therapeutically effective amount of the composition to the subject.

### Figures

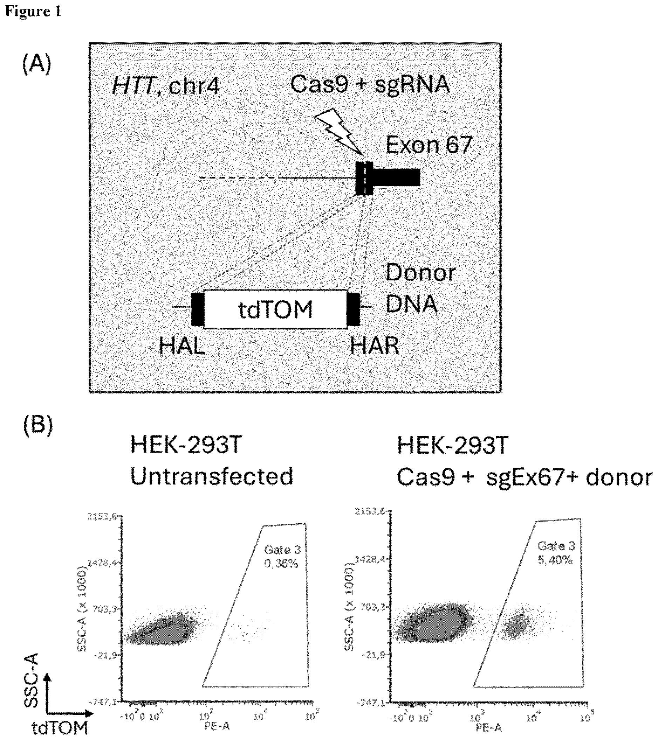

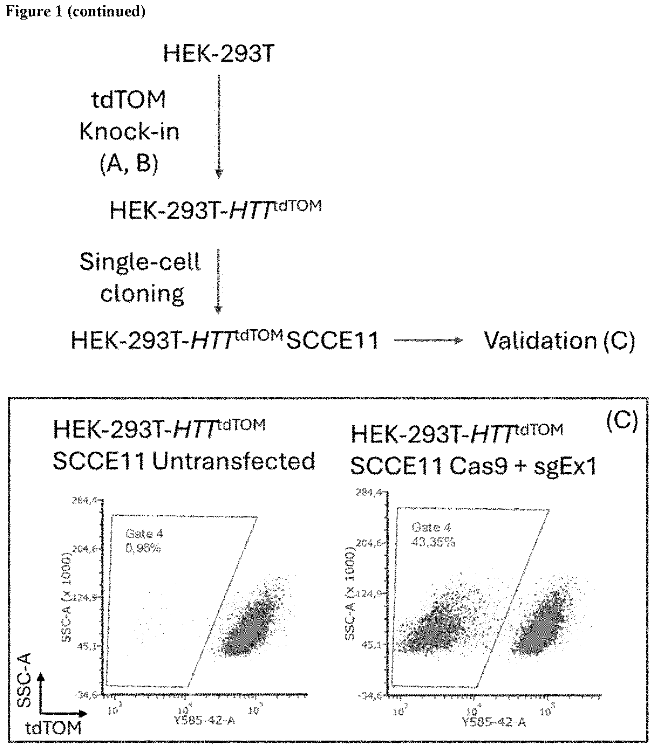

**Figure 1** **| Generation of a transcription reporter cell line for HTT expression.** (A) Schematic of the CRISPR/Cas9-mediated insertion of tdTomato (tdTOM) reporter gene into exon 67 of the HTT gene on chromosome 4 (HTT chr4). The donor DNA containing homology arms (HAL and HAR) flanking the tdTOM gene is introduced into the cells alongside Cas9 and sgRNA to facilitate targeted insertion. (B) Flow cytometry plots illustrating the gating strategy used to identify and sort tdTOM-positive cells. (C) Flow cytometry plots showing results of cell line validation experiment. Transient expression of Cas9 and sgRNA targeting the first exon of HTT determines the formation of tdTOM-negative cells, indicating successful reporter integration in the last exon of the gene.

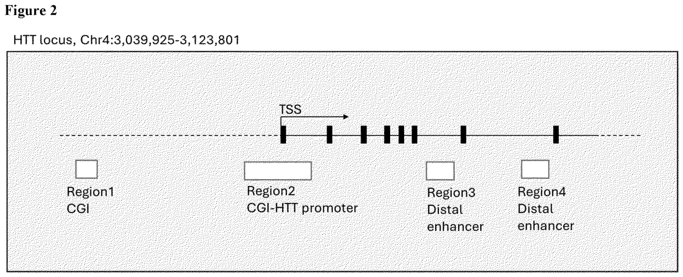

**Figure 2** **| Schematic representation of regulatory regions in the HTT gene.** The diagram depicts putative key regulatory elements in the HTT gene locus targeted by the sgRNA library. Region 1 corresponds to a genomic region annotated as a CpG island (CGI) upstream of the primary gene promoter. Region 2 corresponds to the promoter and contains a second CGI and the transcription start site (TSS) is indicated with an arrow. Finally, Regions 3 and 4 are distal enhancers downstream of the gene promoter.

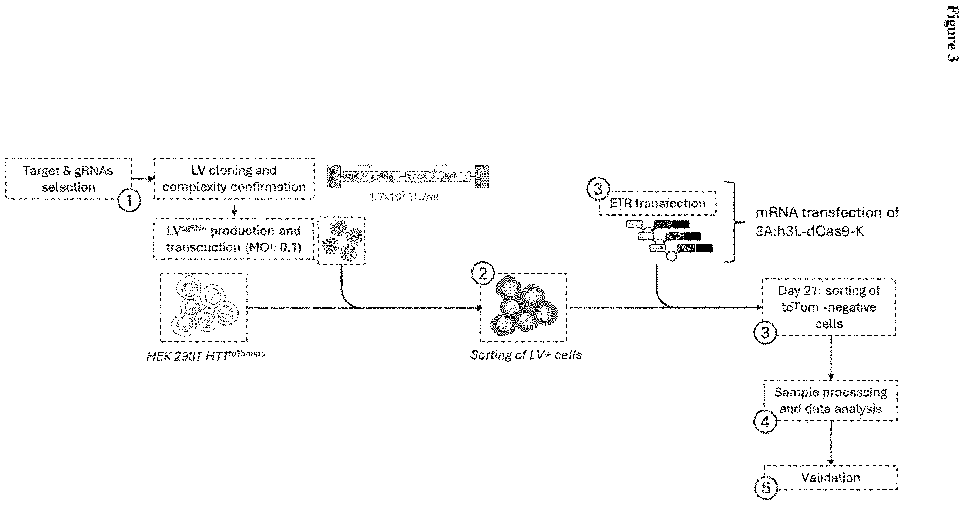

**Figure 3** **| Workflow for sgRNA library screening.** Schematic representation of the experimental procedure: (1) Selection of sgRNA target sites (see Figure 2) and design of corresponding guide RNAs (gRNAs). Guides are then cloned into a lentiviral vector (LV), and the library is sequenced to verify composition and complexity. LVs are then produced and used to transduce HEK-293T HTTtdTomato at a multiplicity of infection (MOI) of 0.1. (2) Transduced cells are purified by FAC sorting and (3) transfected with Engineered Transcriptional Repressors (ETRs). HTT-silenced cells are sorted 21 days post-transfection, isolating tdTomato-negative cells. (4) DNA is extracted from sorted cells, and the integrated guides are identified by sequencing followed by data analysis to identify and validate enriched sgRNAs.

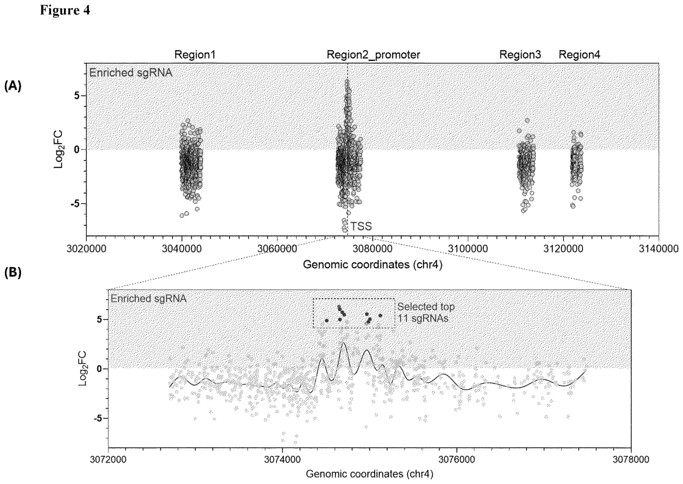

**Figure 4** **| Identification of enriched sgRNAs.** (A) Schematic representation of enriched sgRNAs mapped onto the genomic regions (see Figure 1). Each dot represents a sgRNA with its position on the genome (X-axis) and the relative frequency enrichment in silenced cells over non-silenced ones (Y-axis, Log2FC). (B) Zoom in proximity to the TSS of the HTT gene. The top 11 sgRNAs with the highest enrichment are highlighted within the dashed box.

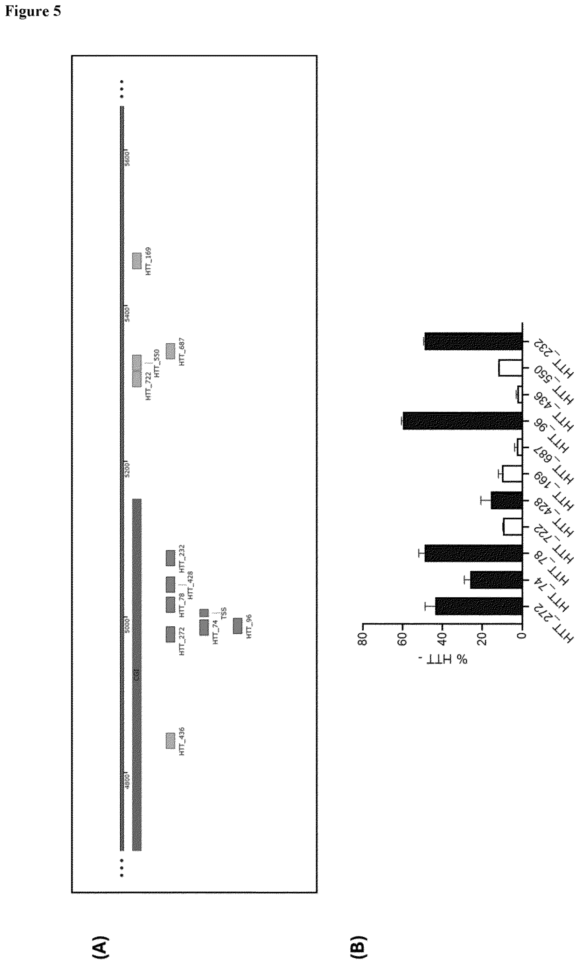

**Figure 5** **| Validation of top 11 sgRNAs targeting the HTT gene in HEK-293T HTTtdTomato.** (Top) Schematic map illustrating the location of selected sgRNAs (gray boxes) within the HTT promoter region. The transcription start site (TSS) is marked along with the CpG island (CGI) of the gene. sgRNAs are labelled with their corresponding identifiers. (Bottom) Bar graph representing the activity of each sgRNA in terms of silencing efficiency.

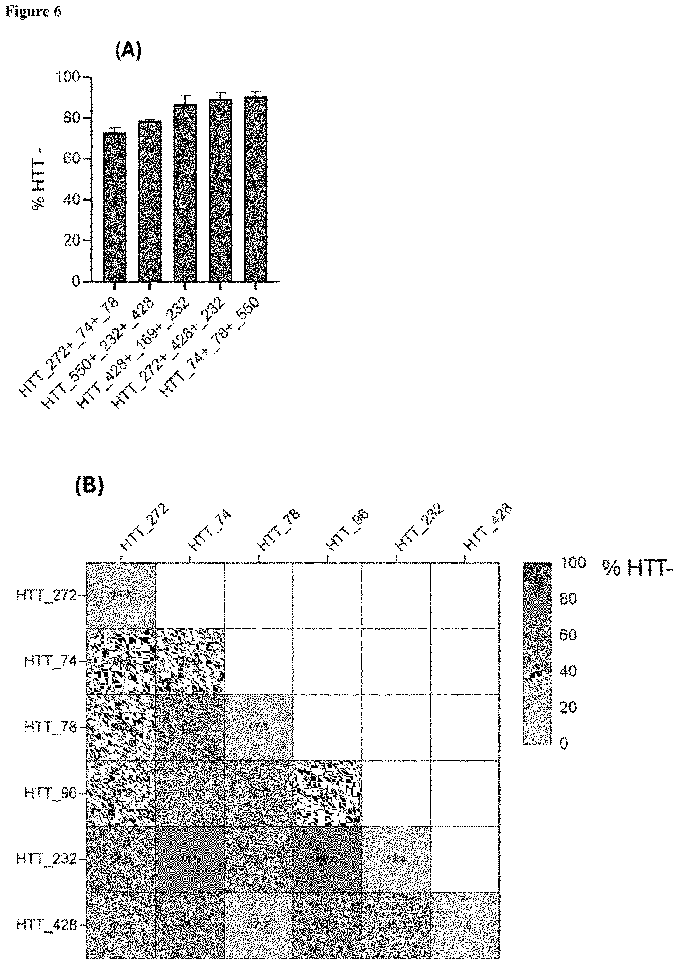

**Figure 6** **| Silencing efficiency of sgRNA combinations in HEK-293T HTTtdTomato.** (A) Bar graph representing the silencing efficiency of some triple combinations of the top 6 sgRNAs targeting the HTT gene. The y-axis reports the percentage of silenced cells, while the x-axis lists the different triple sgRNA combinations. Higher bars indicate greater silencing efficiency. (B) Heatmap showing the silencing activity of all possible double combinations of the top 6 sgRNAs. Each cell contains the percentage of HTT-negative cells, with darker shades representing higher efficiency.

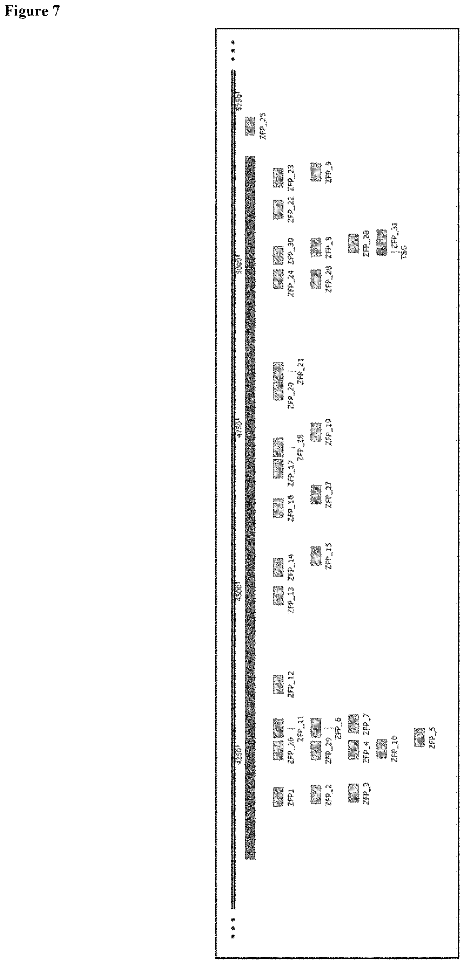

**Figure 7** **| Distribution of Zinc Finger Proteins (ZFPs) targeting the HTT gene region.** Genomic map illustrating the location of selected ZFPs (gray boxes) within the HTT gene region. The transcription start site (TSS) is marked along with the CpG island (CGI). Each ZFP is labeled with its corresponding identifier.

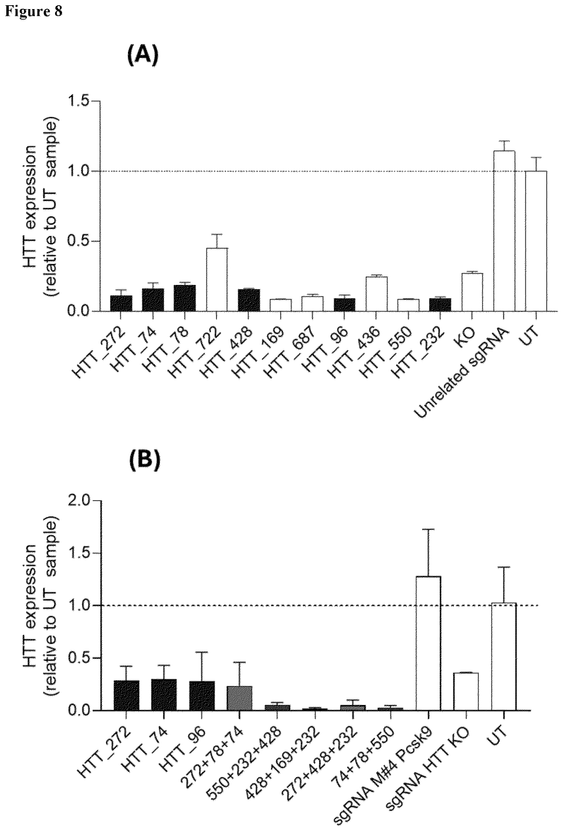

**Figure 8** **| Silencing efficiency of individual sgRNAs and combinations in Neural Stem Cells (NSCs).** (A) Bar graph representing the silencing efficiency of the top 11 sgRNAs targeting the HTT gene in NSCs. (B) Bar graph representing the silencing efficiency of some triple combinations of sgRNAs from the top 6 from the sgRNA library screening. The y-axis indicates HTT expression upon delivery of ETRs and the indicated sgRNA as the fraction of the expression over the UT sample.

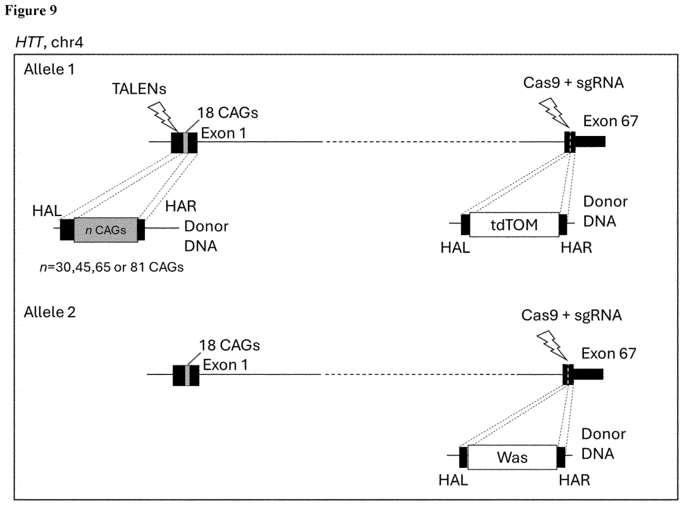

**Figure 9** **| Schematic representation of the gene editing strategy for the construction of HTT allele-specific transcriptional reporter cell lines.** Step 1: Knock-in of two different fluorophores in the two HTT alleles. Cas9 and an sgRNA targeting the last exon of the HTT gene are co-delivered along with a donor DNA containing homology arms (HAL and HAR) flanking the tdTomato (tdTOM) or Wasabi (Was) reporter gene. Step 2: Monoallelic introduction of CAG repeat expansions of different lengths into the first exon by TALEN-mediated knock-in.

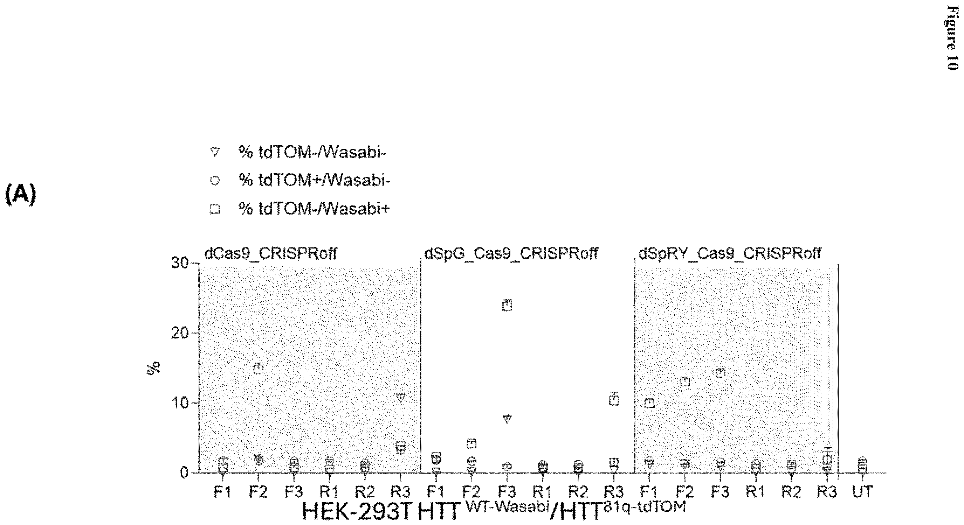

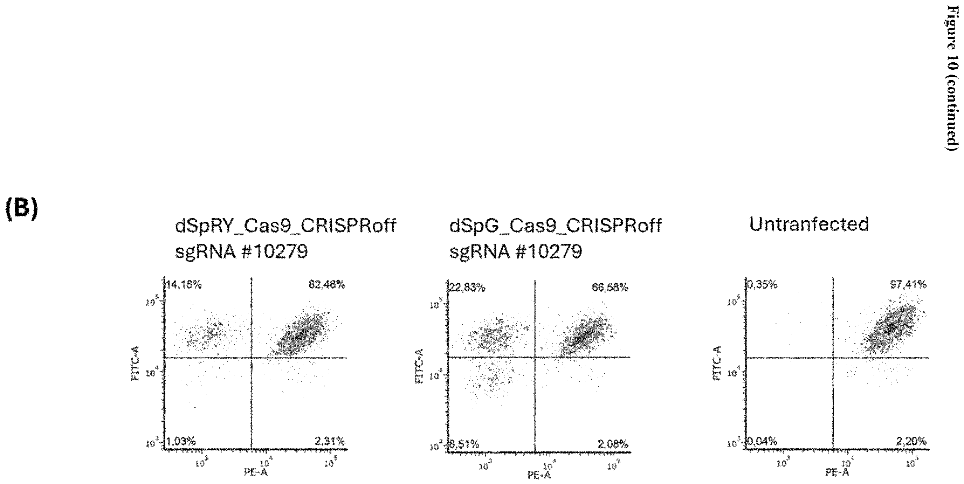

**Figure 10** **| Allele-selective silencing of the CAG-expanded HTT gene in HEK-293T HTT WT-Wasabi/HTT81q-tdTOM.** (A) Dot plots showing the percentage of cells negative for tdTomato (tdTOM) and/or Wasabi (Was) following plasmid delivery of ETRs (dSpRY_CRISPRoff, dSpG CRISPRoff, or dCas9_CRISPRoff) and an sgRNA targeting the forward (F1, 2, or 3) and the reverse (R1, 2, or 3) strand of the CAG expansion. (B) Representative flow cytometry plots illustrating the expression of tdTOM (expanded allele) and Was (wild-type allele) upon delivery of sgRNA F3 and either dSpG_CRISPRoff or dSpRY_CRISPRoff.

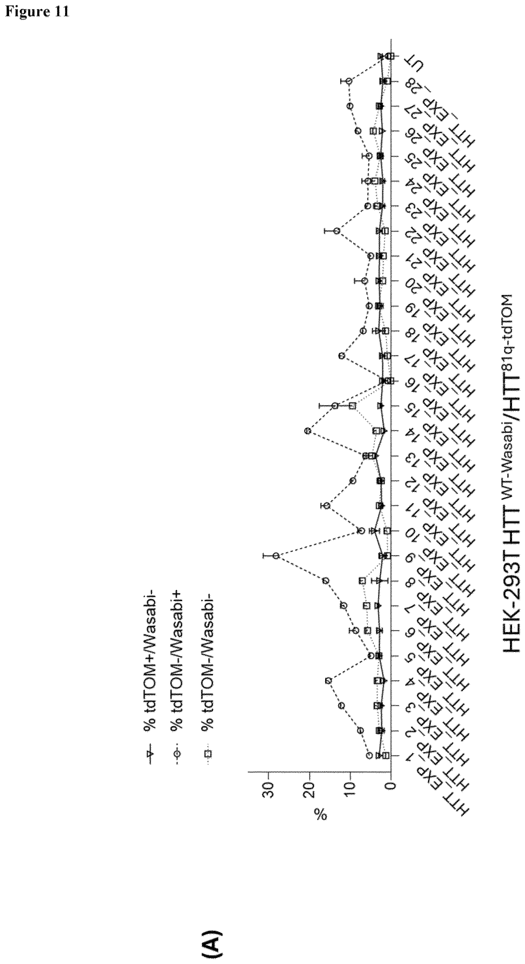

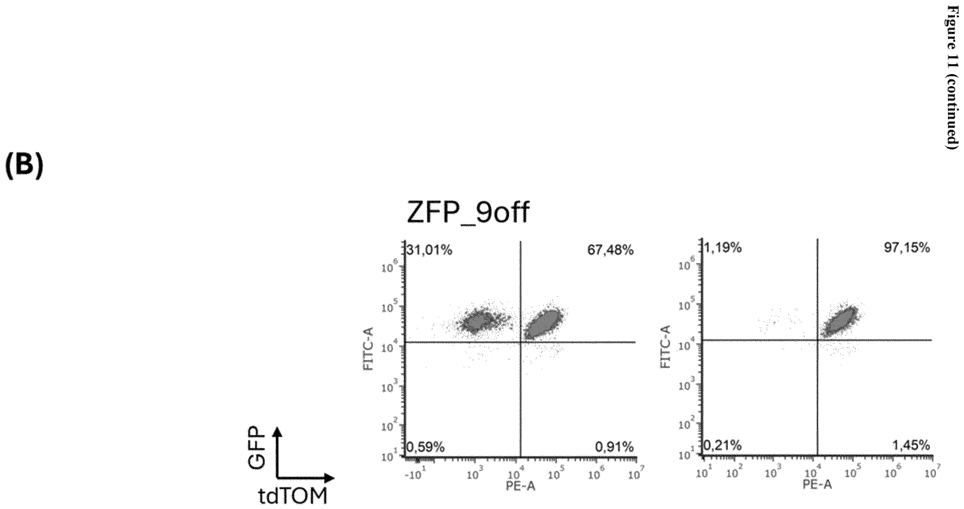

**Figure 11** **| Allele-selective silencing of the CAG-expanded HTT gene in HEK-293T HTT WT-Wasabi/HTT81q-tdTOM.** (A) Dot plots showing the percentage of cells negative for tdTomato (tdTOM) and/or Wasabi (Was) following plasmid delivery of ETRs comprising CAG-targeting and allele-selective ZFPs (herein defined as ZFPoff). (B) Representative flow cytometry plots illustrating the expression of tdTOM (expanded allele) and Was (wild-type allele) upon delivery of ETR containing the ZFP_9.

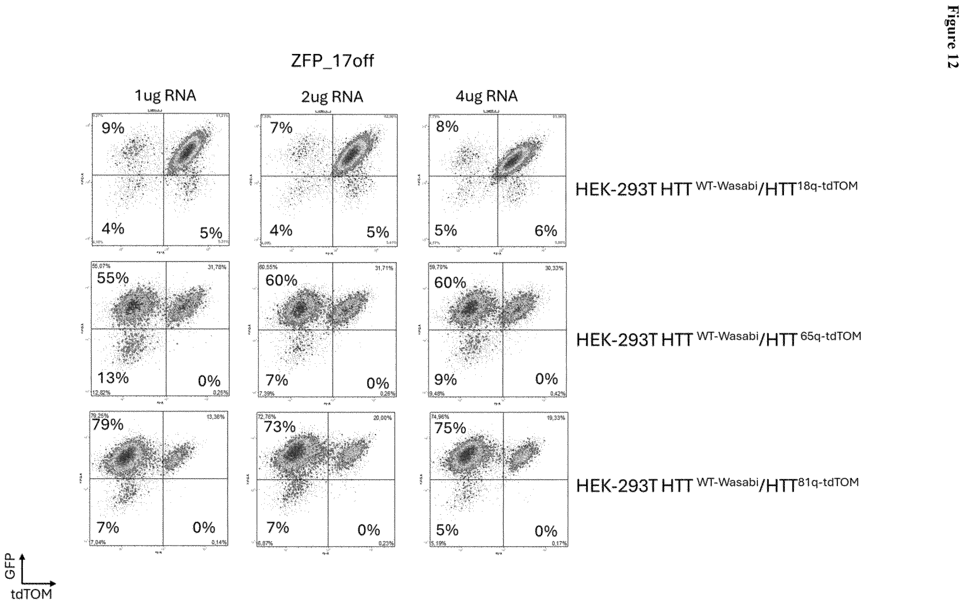

**Figure 12** **| Allele-selective silencing of the CAG-expanded HTT gene in HEK-293T with 18, 65, and 81q.** Representative flow cytometry plots illustrating the expression of tdTOM (expanded allele) and Was (wild-type allele) upon mRNA delivery of the ETR containing the ZFP_17 (three doses: 1, 2, or 4 µg) in HEK-293T with 18, 65, and 81 CAGs.

### Detailed description of the invention

The present invention is based on the following contributions to the art:
1. Efficient and durable biallelic silencing of a polyglutamine disease-related gene can be achieved by targeting one or more sequence contained in a regulatory region of the polyglutamine disease-related gene using one of the compositions of the present invention (see, for example, Figures 5B, 6 and 8B).
2. Stable and durable allele-specific silencing of a pathogenic allele of a polyglutamine disease-related gene can be achieved by targeting a sequence contained in the poly(CAG) tract of the pathogenic allele using one of the compositions of the present invention (see, for example, Figure 10 or 11).
3. The combination of a TRD that increases repressive histone modifications with a TRD that increases DNA methylation can result in robust and long-lasting silencing (e.g., more than 20 days) of a polyglutamine disease-related gene (see, for example, Figures 5B, 6, 8B, 10 and 11).
4. A dCas protein can effectively and specifically silence the pathogenic allele of a polyglutamine disease-related gene by targeting the poly(CAG) tract (see, for example, Figure 11).
5. Co-targeting of the regulatory region and the poly(CAG) tract of the polyglutamine disease-related gene with two or more ETRs can lead to higher silencing efficiencies of the pathogenic allele as compared to individual targeting of the two sequences.

The present invention provides a composition comprising one or more nucleic acid encoding at least a TRD and a polynucleotide-binding domain, wherein the TRD is operably linked to the polynucleotide-binding domain when expressed, and wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

Further embodiments of the invention are discussed in more detail below.

The invention is not limited in its application to the details of construction and the arrangement of components set forth in the present description or illustrated in the drawings. The invention is capable of other embodiments and of being practiced or of being carried out in various ways. Also, the phraseology and terminology used herein is for the purpose of description and should not be regarded as limiting. The use of "including", "comprising", "having", "containing", "involving" and variations thereof herein, is meant to encompass the items listed thereafter and equivalents thereof as well as additional items.

Unless otherwise defined herein, scientific, and technical terms used in connection with the present disclosure shall have the meanings that are commonly understood by those of ordinary skill in the art. The methods and techniques of the present disclosure are generally performed according to conventional methods well-known in the art. Generally, nomenclatures used in connection with, and techniques of biochemistry, enzymology, molecular and cellular biology, microbiology, virology, cell or tissue culture, genetics, and protein and nucleic acid chemistry described herein are those well-known and commonly used in the art. The methods and techniques of the present disclosure are generally performed according to conventional methods well known in the art and as described in various general and more specific references that are cited and discussed throughout the present specification unless otherwise indicated.

### Definitions

The term "*composition*" refers to a combination, aggregate or mixture. The composition may be a combination product as defined in 21 Code of Federal Regulation (CFR) 3.2(e). According to 21 CFR 3.2(e), a combination product includes (1) a product comprised of two or more regulated components, i.e., drug/device, biologic/device, drug/biologic, or drug/device/biologic, that are physically, chemically, or otherwise combined or mixed and produced as a single entity, (2) two or more separate products packaged together in a single package or as a unit and comprised of drug and device products, device and biological products, or biological and drug products, (3) a drug, device, or biological product packaged separately that according to its investigational plan or proposed labeling is intended for use only with an approved individually specified drug, device, or biological product where both are required to achieve the intended use, indication, or effect and where upon approval of the proposed product the labeling of the approved product would need to be changed, e.g., to reflect a change in intended use, dosage form, strength, route of administration, or significant change in dose, or (4) any investigational drug, device, or biological product packaged separately that according to its proposed labeling is for use only with another individually specified investigational drug, device, or biological product where both are required to achieve the intended use, indication, or effect.

The term "*construct*" as used herein can refer to a protein or nucleic acid construct. A protein construct according to the present invention comprises a polynucleotide-binding domain and one or more TRD, wherein the one or more TRD is operably linked to the polynucleotide-binding domain. A nucleic acid construct comprises one or more nucleic acid encoding the protein construct.

The term "*dCas*" or "*dCas protein*" refers to a group of well-known catalytically dead CRISPR-associated proteins (see Xu & Qi, 2019. J Mol Biol. 431(1):34-47; Brezgin et al., 2019. Int J Mol Sci. 20(23): 6041). In some embodiments, the dCas protein is a type II dCas protein (e.g., dCas9). Wild-type Cas9 can be rendered catalytically dead by introducing point mutations in the RuvC1 and HNH nuclease domains (e.g., mutating D10 and H840). Well-known point mutations include D10A and H840A (see Jinek et al., 2012. Science. 337(6096): 816-821). dCas9 can be further rendered more flexible by mutating the amino acids D1135, S1136, G1218, E1219, R1335, and T1337 (e.g., D1135L/S1136W/G1218K/E1219Q/R1335Q/T1337R) to allow NGN PAM recognition. Moreover, PAM flexibility can be further extended by adding additional mutations at the following amino acids: L1111, A1322, A61, N1317 and R1333 (e.g., D1135L/S1136W/G1218K/E1219Q/R1335Q/T1337R/L1111R/A1322R/A61R/N1317R/R1333P) (see Walton et al., 2020. Science. 368(6488):290-296). Further examples of CRISPR-associated (Cas) proteins include predecessors such as TnpB and IscB (Kato et al., 2022. Nat Commun. 13(1):6719; Karvelis et al., 2021. Nature. 599(7886):692-696). These predecessors can be made catalytically dead to obtain further dCas proteins.

The term "*DNMT3L*" refers to a known member of the DNMT3 family that has no DNA methyltransferase activity. However, DNMT3L is known to induce *de novo* methylation by recruitment or activation of DNA methyltransferases such as DNMT3A (Copped6, F. (2012). Epigenetics in Human Disease, Chapter 9 (Editor: Tollefsbol, T.O.) Academic Press). In some embodiments, the DNMT3L is operably linked to a histone fragment when expressed. The histone fragment is capable of recruiting an endogenously expressed DNA methyltransferase. The histone fragment may be a histone tail, such as a histone H3 tail. Histone tails are known to recruit DNA methyltransferases (Li et al., 2011. Cell Res. 21(8):1172-81). The DNMT3L may be directly or indirectly fused to the histone fragment (see, for example, Neumann et al., 2024. Science. 384(6703):ado7082). The DNMT3L may be human DNMT3L or non-human DNMT3L. The non-human DNMT3L may be *Apodemus sylvaticus* DNMT3L. In some embodiments, the DNMT3L comprises or consists of SEQ ID NO: 2 or a sequence that has at least 70, 75, 80, 85, 90, 95, 97, 98 or 99% sequence identity with SEQ ID NO: 2. In some embodiments, the DNMT3L comprises or consists of SEQ ID NO: 112 or a sequence that has at least 70, 75, 80, 85, 90, 95, 97, 98 or 99% sequence identity with SEQ ID NO: 112.

As used herein, the term "*effective amount*" of an agent, e.g., a therapeutic agent such as a nucleic acid encoding an engineered transcription repressor (ETR), is that amount sufficient to effect beneficial or desired results, for example, clinical results, and, as such, an "*effective amount*" depends upon the context in which it is being applied. The term "*effective amount*" can be used interchangeably with "*effective dose*", "*therapeutically effective amount*", or "*therapeutically effective dose*".

The expression "*EZH2 domain*" refers to the Enhancer of zeste (EZH) 2, a methyltransferase and component of the polycomb repressive complex 2 (see, for example, Yoo & Henninghausen, 2012. Int JBiol Sci. 8(1):59-65).

The term "*gene"* encompasses the coding region and regulatory regions, the latter may contain one or more non-coding regulatory element. The expression "*non-coding regulatory element*" is given its normal meaning in the art and refers to any non-coding element that modulates the transcription or translation of a particular gene. The DNA-binding domains or gRNAs of the present invention that specifically bind to a sequence contained in a regulatory region of the polyglutamine disease-related gene may specifically bind to a sequence contained in a regulatory region of the polyglutamine disease-related gene. The regulatory region bound by said DNA-binding domains or gRNAs of the present invention may be upstream or downstream of the transcription start site (TSS). In some embodiments, the regulatory region bound by said DNA-binding domain or gRNAs of the present invention spans a 2 kb region centered on the TSS of the gene. In other words, the regulatory region may be a region that spans from 1 kb upstream of the TSS to 1 kb downstream of the TSS. In some embodiments, the regulatory region bound by said DNA-binding domain or gRNAs of the present invention spans a 1 kb region centered on the TSS of the gene. In other words, the regulatory region may be a region that spans from 0.5 kb upstream of the TSS to 0.5 kb downstream of the TSS.

The terms "*individual*", "*patient*" or "*subject*" are used interchangeably in the present application to designate a human being and are not meant to be limiting in any way. The "*individual*", "*patient*" or "*subject*" can be of any age, sex and physical condition.

The expression "*KRAB domain*" refers to any Krüppel associated box (KRAB) domain that is suitable for repressing transcription of a gene. The KRAB domain is known to directly interact with KAP-1 which recruits histone-modifying enzymes including the histone H3K9-specific methyltransferase SETDB1 (see, for example, Zhu et al., 2020. Transl Psychiatry. 10(1):115). Methods for determining whether a KRAB domain would be suitable for repressing transcription are known in the art and many suitable KRAB domains have already been identified (see, for example, Alerasool et al., 2020. Nat Methods. 17(11):1093-1096). In some embodiments, the KRAB domain is a ZIM KRAB domain (e.g., a ZIM3 KRAB domain) or a KOX1 KRAB domain (e.g., SEQ ID NO: 1). In some embodiments, the KRAB domain comprises or consists of SEQ ID NO: 1 or a sequence that has at least 70, 75, 80, 85, 90, 95, 97, 98 or 99% sequence identity with SEQ ID NO: 1.

The expressions "*is operably linked to the dCas protein when expressed*" and "*is operably linked to the DNA-binding domain when expressed*" mean that when the one or more TRD and the polynucleotide-binding domain (e.g., a DNA-binding domain or dCas protein) are expressed, the one or more TRD is covalently or non-covalently linked to the polynucleotide-binding domain. A noncovalent link can be achieved by, for example, encoding a SunTag in the same open reading frame (ORF) as the polynucleotide-binding domain, and encoding a single chain variable fragment (scFv) that is specific for a repeating peptide epitope found on the SunTag in the same ORFs as each TRD (see Tanenbaum et al., 2014. Cell. 159(3): 635-646). A covalent link can be achieved by encoding one or more TRD in the same ORF as the polynucleotide-binding domain. These are commonly referred to as fusion proteins and, in any embodiment of the present invention, the one or more nucleic acid of the invention may encode a fusion protein comprising the components that are operably linked. A covalent link can also be achieved by using any isopeptide-forming Catcher/Tag pair discussed in Fan & Aranko, 2024. Chembiochem. 25(1):e202300600.

As used herein, "*pharmaceutically acceptable carrier*" or "*pharmaceutically acceptable diluent*" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington: The Science and Practice of Pharmacy 22nd edition, Pharmaceutical press (2012), ISBN-13: 9780857110626 may also be included.

The expression "*polyglutamine disease*" (can be abbreviated as "*polyQ disease*") is well understood in the art and refers to a group of diseases caused by abnormal expansion of CAG triplet sequences in the coding region of a gene (see, for example, Estevam et al., 2023. Database (Oxford). 2023:baad060 and the PolyQ Database disclosed therein). The polyglutamine disease may be Dentatorubropallidoluysian atrophy, Huntington's disease, Spinal and bulbar muscular atrophy or a Spinocerebellar ataxia (e.g., Type 1, 2, 3, 6, 7 or 17). Polyglutamine disease-related genes are those containing the abnormal expansion of CAG triplet sequences that cause a polyglutamine disease. Such genes are known in the art (see Table 1). In any embodiment of the present invention, the polyglutamine disease-related gene is *ATN1*, *HTT*, *AR*, *ATXN1*, *ATXN2*, *ATXN3*, *CACNA1A*, *ATXN7*, or *TBP.* Further details on polyglutamine disease-related genes can be found in the UniProt database (see Table 2).

**Table 2: UniProt Accession Numbers for Polyglutamine Disease-related Genes**

| **Gene name** | **UniProt Accession Number** |
|---|---|
| *ATN1* | P54259 |
| *HTT* | P42858 |
| *AR* | P10275 |
| *ATXN1* | P54253 |
| *ATXN2* | Q99700 |
| *A TAN3* | P54252 |
| *CACNA1A* | O00555 |
| *ATXN7* | O15265 |
| *TBP* | P20226 |

The term "*polynucleotide-binding domain*" refers to an independently folded protein domain that contains at least one structural motif that recognizes double- or single-stranded nucleic acids. The term "*polynucleotide*" is used interchangeably with the expression "*nucleic acid*" herein. In some embodiments, the polynucleotide-binding domain is a DNA-binding domain. Exemplary DNA-binding domains include Transcription Activator-like Effectors (TALE) and Zing Finger Proteins (ZFP). In some embodiments, the polynucleotide-binding domain is a gRNA-binding domain. An exemplary gRNA-binding domain is dCas.

The expression "*poly(CAG) tract*" is given its normal meaning in the art and refers to a region of a gene containing CAG repeats that encode for a polyglutamine tract (see, for example, Stoyas & Spada, 2018. Handb Clin Neurol. 143-170). A DNA-binding domain or gRNA that specifically binds to a sequence contained in the poly(CAG) tract of a polyglutamine disease-related gene allows the constructs of the present invention to downregulate the expression of the pathogenic allele of the polyglutamine disease-related gene to a higher degree than a healthy allele (also commonly referred to as a "wild-type" allele) of the polyglutamine disease-related gene. Pathogenic alleles contain expanded poly(CAG) tracts which allow for a greater degree of multimerization of transcription repressors and thus a more effective silencing of the pathogenic allele. In any embodiment of the present invention, the construct comprising DNA-binding domain or gRNA that specifically binds to a sequence contained in the poly(CAG) tract of the polyglutamine disease-related gene specifically downregulates the expression of the pathogenic allele. A sequence contained in the poly(CAG) tract of the polyglutamine disease-related gene may comprise SEQ ID NO: 99 or SEQ ID NO: 105.

The expression "*Transcription Activator-like Effectors*" (can be abbreviated as "*TALE*") refers to a group of well-known modular DNA-binding proteins that are based on repeat-containing proteins expressed by the bacterial genus *Xanthomonas.* Custom TALEs can be designed using web-based tools such as TALE-NT 2.0 (Doyle et al., 2012. Nucleic Acids Res. 40(Web Server issue):W117-22).

The expression "*transcriptional repressor domain*" (abbreviated as "*TRD*") refers to any polypeptide that deposits an epigenetic mark or recruits one or more endogenous polypeptide that deposits an epigenetic mark. An "*epigenetic mark*" may be a histone modification or DNA methylation. Thus, a TRD of the present invention may increase repressive histone modifications and/or increase DNA methylation. It has been established in the art that the combination of a TRD that increases repressive histone modifications and a TRD that increases DNA methylation is more effective than each repressor on their own (Amabile et al., 2016. Cell. 167(1):219-232; Mlambo et al., 2018. Nucleic Acids Res. 46(9):4456-4468; O'Geen et al., 2019. Epigenetics Chromatin. 12(1):26; Nuñez et al., 2021. Cell. 184(9):2503-2519.e17; Cappelluti et al., 2024. Nature. 627(8003):416-423). Thus, in any of the disclosed embodiments, one or more nucleic acid of any one of the compositions of the present invention encodes a TRD that increases repressive histone modifications and a TRD that increases DNA methylation. In some embodiments, the TRD that increases repressive histone modifications is a TRD that increases histone methylation (e.g., KRAB domain, SETDB1 domain or EZH2 domain). In some embodiments, the TRD that increases repressive histone modifications is a KRAB domain. In some embodiments, the TRD that increases DNA methylation is DNMT3L or a DNA methyltransferase (e.g., DNMT3A, DNMT3B, DNMT3C, or DNMT1). In some embodiments, the DNA methyltransferase is DNMT3A.

The expression "*Zinc Finger Protein*" (can be abbreviated as "*ZFP*") refers to a group of DNA-binding proteins that are based on DNA-binding motifs found in mammalian transcription factors (see Negi et al., 2023. J Biol Inorg Chem. 28(3): 249-261). Machine learning models such as ZFDesign have been developed and can be used to design custom ZFPs (Ichikawa et al., 2023. Nat Biotechnol. 41(8):1117-1129). Phage display has also been applied to select for ZFPs (see Negi et al., 2023. J Biol Inorg Chem. 28(3): 249-261).

The terms "*treatment*" and "*therapy*", as used in the present application, refer to a set of hygienic, pharmacological, surgical and/or physical means used with the intent to cure and/or alleviate a disease and/or symptom with the goal of remediating the health problem. The terms "*treatment*" and "*therapy*" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

### Compositions targeting the regulatory element

In some embodiments, the composition comprises one or more nucleic acids encoding at least:
(a) a TRD; and
(b) a DNA-binding domain (e.g., a ZFP or a TALE), wherein at least one TRD is operably linked to the DNA-binding domain when expressed;
wherein the DNA-binding domain specifically binds to a sequence contained in a regulatory region of the polyglutamine disease-related gene.

In another embodiment, the composition comprises:
(a) one or more nucleic acids encoding at least a TRD and a dCas protein, wherein at least one TRD is operably linked to the dCas protein when expressed; and
(b) a guide RNA (gRNA) or a nucleic acid encoding said gRNA;
wherein the gRNA specifically binds to a sequence contained in a regulatory region of the polyglutamine disease-related gene.

In another embodiment, the composition comprises one or more nucleic acid encoding at least:
(a) one or more TRD; and
(b) one or more DNA-binding domain (e.g., a ZFP or TALE), wherein at least one TRD is operably linked to each DNA-binding domain when expressed;
wherein the one or more DNA-binding domain specifically binds to one or more sequences contained in a regulatory region of the polyglutamine disease-related gene.

In another embodiment, the composition comprises:
(a) one or more nucleic acid encoding one or more TRD and one or more dCas protein, wherein at least one TRD is operably linked to each dCas protein when expressed; and
(b) one or more guide RNA (gRNA) or one or more nucleic acid encoding said one or more gRNA;
wherein the one or more gRNAs specifically bind to one or more sequences contained in a regulatory region of the polyglutamine disease-related gene.

### Compositions targeting the poly(CAG) tract

In some embodiments, the composition comprises one or more nucleic acids encoding at least:
(a) a TRD;
(b) a DNA-binding domain (e.g., a ZFP or a TALE), wherein at least one TRD is operably linked to the DNA-binding domain when expressed;
   wherein the DNA-binding domain specifically binds to a sequence contained in the poly(CAG) tract of the polyglutamine disease-related gene. The one or more nucleic acid may encode two or more DNA-binding domains that specifically bind to one, two or more sequences contained in the poly(CAG) tract of the polyglutamine disease-related gene, wherein each DNA-binding domain is operably linked to one or more TRD. The one or more TRD may be the same or different. In some embodiments, the composition further comprises:
(c) one or more nucleic acids encoding at least a TRD and a dCas protein, wherein at least one TRD is operably linked to the dCas protein when expressed; and
(d) a guide RNA (gRNA) or a nucleic acid encoding said gRNA;
   wherein the gRNA specifically binds to a sequence contained in the poly(CAG) tract of the polyglutamine disease-related gene. More than one TRD may be operably linked to each of the polynucleotide-binding domains. The TRDs linked to the DNA-binding domain and the dCas protein may be the same or different.

In another embodiment, the composition comprises:
(a) one or more nucleic acids encoding at least a TRD and a dCas protein, wherein at least one TRD is operably linked to the dCas protein when expressed; and
(b) a guide RNA (gRNA) or a nucleic acid encoding said gRNA;
wherein the gRNA specifically binds to a sequence contained in the poly(CAG) tract of the polyglutamine disease-related gene.

In another embodiment, the composition comprises one or more nucleic acid encoding at least:
(a) one or more TRD;
(b) one or more DNA-binding domain (e.g., a ZFP or TALE), wherein at least one TRD is operably linked to each DNA-binding domain when expressed;
wherein the one or more DNA-binding domain specifically binds to one or more sequences contained in the poly(CAG) tract of the polyglutamine disease-related gene.

In another embodiment, the composition comprises:
(a) one or more nucleic acid encoding one or more TRD and one or more dCas protein, wherein at least one TRD is operably linked to each dCas protein when expressed; and
(b) one or more guide RNA (gRNA) or one or more nucleic acid encoding said one or more gRNA;
wherein the one or more gRNAs specifically bind to one or more sequences contained in the poly(CAG) tract of the polyglutamine disease-related gene.

### Compositions targeting the regulatory region and the poly(CAG) tract

In some embodiments, the composition comprises one or more nucleic acid encoding at least:
(a) two or more TRDs;
(b) a first DNA-binding domain (e.g., a ZFP or TALE), wherein one or more TRD is operably linked to the first DNA-binding domain when expressed; and
(c) a second DNA-binding domain (e.g., a ZFP or TALE), wherein one or more TRD is operably linked to the second DNA-binding domain when expressed,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene.

In another embodiment, the composition comprises:
(a) one or more nucleic acid encoding at least a TRD and a dCas protein, wherein the TRD is operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene.

In another embodiment, the composition comprises one or more nucleic acids encoding for:
(a) one or more TRDs;
(b) a first DNA-binding domain (e.g., a ZFP or TALE), wherein the one or more TRDs are operably linked to the first DNA-binding domain when expressed; and
(c) a second or more DNA-binding domains (e.g., a ZFP or TALE), wherein the one or more TRDs are operably linked to the second or more DNA-binding domains when expressed,
wherein the first DNA-binding domain specifically binds to a sequence contained in the poly(CAG) tract of the polyglutamine disease-related gene, and the second or more DNA-binding domains specifically bind to one or more sequences contained in a regulatory region of the polyglutamine disease-related gene.

In another embodiment, the composition comprises one or more nucleic acids encoding for:
(a) one or more TRDs;
(b) a first DNA-binding domain (e.g., a ZFP or TALE), wherein the one or more TRDs are operably linked to the first DNA-binding domain when expressed; and
(c) a second or more DNA-binding domains (e.g., a ZFP or TALE), wherein the one or more TRDs are operably linked to the second or more DNA-binding domains when expressed,
wherein the first DNA-binding domain specifically binds to a sequence contained in a regulatory region of the polyglutamine disease-related gene, and the second or more DNA-binding domains specifically bind to one or more sequences contained in the poly(CAG) tract of the polyglutamine disease-related gene

In another embodiment, the composition comprises one or more nucleic acids encoding for:
(a) one or more TRDs;
(b) a first or more DNA-binding domains (e.g., a ZFP or TALE), wherein the one or more TRDs are operably linked to the one or more DNA-binding domains when expressed; and
(c) a second or more DNA-binding domains (e.g., a ZFP or TALE), wherein the one or more TRDs are operably linked to the second or more DNA-binding domains when expressed,
wherein the first or more DNA-binding domains specifically bind to one or more sequences contained in a regulatory region of the polyglutamine disease-related gene, and the second or more DNA-binding domains specifically bind to one or more sequences contained in the poly(CAG) tract of the polyglutamine disease-related gene.

In another embodiment, the composition comprises one or more nucleic acids encoding for:
(a) one or more TRDs;
(b) a dCas protein, wherein the one or more TRDs are operably linked to the dCas protein when expressed;
(c) a first guide RNA (gRNA) or a nucleic acid encoding the first gRNA; and
(d) a second or more gRNAs or nucleic acids encoding the second or more gRNAs,
wherein the first gRNA specifically binds to a sequence contained in the poly(CAG) tract of the polyglutamine disease-related gene, and the second or more gRNAs specifically bind to one or more sequences contained in a regulatory region of the polyglutamine disease-related gene.

In another embodiment, the composition comprises one or more nucleic acids encoding for:
(a) one or more TRDs;
(b) a dCas protein, wherein the one or more TRDs are operably linked to the dCas protein when expressed;
(c) a first guide RNA (gRNA) or a nucleic acid encoding the first gRNA; and
(d) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene.

In another embodiment, the composition comprises one or more nucleic acids encoding for:
(a) one or more TRDs;
(b) a dCas protein, wherein the one or more TRDs are operably linked to the dCas protein when expressed;
(c) a first guide RNA (gRNA) or a nucleic acid encoding the first gRNA; and
(d) a second or more gRNAs or nucleic acids encoding the second or more gRNAs,
wherein the first gRNA specifically binds to a sequence contained in a regulatory region of the polyglutamine disease-related gene, and the second or more gRNAs specifically bind to one or more sequences contained in the poly(CAG) tract of the polyglutamine disease-related gene.

In another embodiment, the composition comprises one or more nucleic acids encoding for:
(a) one or more TRDs;
(b) a dCas protein, wherein the one or more TRDs are operably linked to the dCas protein when expressed;
(c) a first or more guide RNAs (gRNAs) or nucleic acids encoding the first and more gRNAs; and
(d) a second or more gRNAs or nucleic acids encoding the second or more gRNAs,
wherein the first or more gRNAs specifically bind to one or more sequences contained in a regulatory region of the polyglutamine disease-related gene, and the second or more gRNAs specifically bind to one or more sequences contained in the poly(CAG) tract of the polyglutamine disease-related gene.

In another embodiment, the composition comprises one or more nucleic acid encoding at least:
(a) two or more TRDs;
(b) a DNA-binding domain (e.g., a ZFP or TALE), wherein one or more TRD is operably linked to the DNA-binding domain when expressed; and
(c) a dCas protein, wherein one or more TRD is operably linked to the dCas protein when expressed,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene.

In another embodiment, the composition comprises one or more nucleic acids encoding for:
(a) one or more TRDs;
(b) a DNA-binding domain (e.g., a ZFP or TALE), wherein the one or more TRDs are operably linked to the DNA-binding domain when expressed; and
(c) a dCas protein, wherein the one or more TRDs are operably linked to the dCas protein when expressed,
wherein the composition further comprises a gRNA or a nucleic acid encoding for the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene.

In another embodiment, the composition comprises one or more nucleic acid encoding at least:
(a) two or more TRDs;
(b) a DNA-binding domain (e.g., a ZFP or TALE), wherein one or more TRD is operably linked to the DNA-binding domain when expressed; and
(c) a dCas protein, wherein one or more TRD is operably linked to the dCas protein when expressed,
wherein the composition further comprises a gRNA or a nucleic acid encoding the gRNA, and wherein the gRNA specifically binds to a sequence contained in a regulatory region of the polyglutamine disease-related gene, and the DNA-binding domain specifically binds to a sequence contained in the poly(CAG) tract of the polyglutamine disease-related gene.

In another embodiment, the composition comprises one or more nucleic acids encoding for:
(a) two or more TRDs;
(b) a DNA-binding domain (e.g., a ZFP or TALE), wherein one or more TRD is operably linked to the DNA-binding domain when expressed; and
(c) a dCas protein, wherein one or more TRD is operably linked to the dCas protein when expressed,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene.

In any of the above embodiments, one or more TRD may be a TRD that increases repressive histone modifications. In any of the above embodiments, one or more TRD may be a TRD that increases DNA methylation. In any of the above embodiments, the one or more nucleic acid encodes a TRD that increases repressive histone modifications and a TRD that increases DNA methylation.

In some embodiments, the composition comprises one or more nucleic acid encoding at least:
(a) one or more TRD that increases repressive histone modifications;
(b) one or more TRD that increases DNA methylation;
(c) a first DNA-binding domain (e.g., a ZFP or TALE), wherein a TRD according to (a) and/or a TRD according to (b) is operably linked to the first DNA-binding domain when expressed; and
(d) a second DNA-binding domain (e.g., a ZFP or TALE), wherein a TRD according to (a) and/or a TRD according to (b) is operably linked to the second DNA-binding domain when expressed,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene. In some embodiments, a TRD according to (a) or a TRD according to (b) is operably linked to the first DNA-binding domain when expressed, and a TRD according to (a) or a TRD according to (b) is operably linked to the second DNA-binding domain when expressed. In some embodiments, a TRD according to (a) and a TRD according to (b) are operably linked to the first DNA-binding domain when expressed, and a TRD according to (a) or a TRD according to (b) is operably linked to the second DNA-binding domain when expressed. In some embodiments, a TRD according to (a) or a TRD according to (b) is operably linked to the first DNA-binding domain when expressed, and a TRD according to (a) and a TRD according to (b) are operably linked to the second DNA-binding domain when expressed. In some embodiments, a TRD according to (a) and a TRD according to (b) are operably linked to the first DNA-binding domain when expressed, and a TRD according to (a) and a TRD according to (b) are operably linked to the second DNA-binding domain when expressed.

In some embodiments, the composition comprises one or more nucleic acid encoding at least:
(a) two or more TRDs that increase repressive histone modifications;
(b) two or more TRDs that increase DNA methylation;
(c) a first DNA-binding domain (e.g., a ZFP or TALE), wherein a TRD according to (a) and a TRD according to (b) are operably linked to the first DNA-binding domain when expressed; and
(d) a second DNA-binding domain (e.g., a ZFP or TALE), wherein a TRD according to (a) and a TRD according to (b) are operably linked to the second DNA-binding domain when expressed,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene. In some embodiments, the two or more TRDs that increase repressive histone modifications are KRAB domains and the two or more TRDs that increase DNA methylation are each DNMT3A and/or DNMT3L. In some embodiments, the first and second DNA-binding domains are each operably linked to a KRAB domain and DNMT3L when expressed (e.g., the one or more nucleic acid encodes a first fusion protein comprising the first DNA-binding domain, a KRAB domain and DNMT3L, and a second fusion protein comprising the second DNA-binding domain, a KRAB domain and DNMT3L). In some embodiments, DNMT3L is fused to a histone fragment that recruits an endogenously expressed DNA methyltransferase.

In some embodiments, the composition comprises one or more nucleic acid encoding at least:
(a) two or more TRDs that increase repressive histone modifications;
(b) four or more TRDs that increase DNA methylation;
(c) a first DNA-binding domain (e.g., a ZFP or TALE), wherein a TRD according to (a) and two TRDs according to (b) are operably linked to the first DNA-binding domain when expressed; and
(d) a second DNA-binding domain (e.g., a ZFP or TALE), wherein a TRD according to (a) and two TRDs according to (b) are operably linked to the second DNA-binding domain when expressed,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene. In some embodiments, the first and second DNA-binding domains are each operably linked to a KRAB domain, DNMT3L and DNMT3A when expressed (e.g., the one or more nucleic acid encodes a first fusion protein comprising the first DNA-binding domain, a KRAB domain, DNMT3A and DNMT3L, and a second fusion protein comprising the second DNA-binding domain, a KRAB domain, DNMT3A and DNMT3L). In some embodiments, DNMT3L is fused to a histone fragment that recruits an endogenously expressed DNA methyltransferase.

In some embodiments, the composition comprises:
(a) one or more nucleic acid encoding at least:
   (i) a TRD that increases repressive histone modifications and a TRD that increases DNA methylation; and
   (ii) a dCas protein, wherein the one or more (e.g., two) TRDs are operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene. In some embodiments, the TRD that increases repressive histone modifications (e.g., KRAB) and the TRD that increases DNA methylation (e.g., DNMT3A or DNMT3L) are operably linked to the dCas protein. In some embodiments, the TRD that increases repressive histone modifications is a KRAB domain and the TRD that increases DNA methylation is DNMT3L. In some embodiments, the one or more nucleic acid encodes a fusion protein comprising the dCas protein, the TRD that increases repressive histone modifications and the TRD that increases DNA methylation. For example, the fusion protein may comprise the dCas protein, a KRAB domain and DNMT3L. In some embodiments, DNMT3L is fused to a histone fragment that recruits an endogenously expressed DNA methyltransferase.

In some embodiments, the composition comprises:
(a) one or more nucleic acid encoding at least:
   (i) a TRD that increases repressive histone modifications and two TRD that increase DNA methylation; and
   (ii) a dCas protein, wherein the one or more (e.g., two or three) TRDs are operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene. In some embodiments, the TRD that increases repressive histone modifications (e.g., KRAB) and one TRD that increases DNA methylation (e.g., DNMT3A or DNMT3L) are operably linked to the dCas protein. In some embodiments, a KRAB domain and DNMT3A are operably linked to the dCas protein when expressed and the one or more nucleic acid further encodes DNMT3L. In some embodiments, a KRAB domain, DNMT3L and DNMT3A are operably linked to the dCas protein when expressed (e.g., the dCas protein, KRAB domain, DNMT3L and DNMT3A are comprised in a fusion protein encoded by the one or more nucleic acid).

In some embodiments, the composition comprises:
(a) one or more nucleic acid encoding at least:
   (i) one or more TRD that increase repressive histone modifications and one or more TRD that increases DNA methylation;
   (ii) a first dCas protein, wherein the one or more TRD is operably linked to the dCas protein when expressed; and
   (iii) a second dCas protein, wherein the one or more TRD is operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene. The first dCas protein may be operably linked to a TRD that increases repressive histone modifications (e.g., KRAB) when expressed and the second dCas protein may be operably linked to a TRD that increases DNA methylation (e.g., DNMT3L or DNMT3A) when expressed. In some embodiments, the first dCas protein is operably linked to a KRAB domain when expressed and the second dCas protein is operably linked to DNMT3L when expressed (e.g., the one or more nucleic acid encodes a first fusion protein comprising the first dCas protein and a KRAB domain, and a second fusion protein comprising the second dCas protein and DNMT3L). In some embodiments, the first dCas protein may be operably linked to a TRD that increases DNA methylation (e.g., DNMT3L or DNMT3A) when expressed and the second dCas protein may be operably linked to a TRD that increases histone repressive modifications (e.g., KRAB). In some embodiments, the first dCas protein is operably linked to a DNMT3L domain when expressed and the second dCas protein is operably linked to a KRAB domain when expressed (e.g., the one or more nucleic acids encode a first fusion protein comprising the first dCas protein and a DNMT3L domain, and a second fusion protein comprising the second dCas protein and KRAB).

In some embodiments, the composition comprises:
(a) one or more nucleic acid encoding at least:
   (i) one or more TRD that increases repressive histone modifications and two or more TRDs that increase DNA methylation;
   (ii) a first dCas protein, wherein the one or more TRD is operably linked to the dCas protein when expressed; and
   (iii) a second dCas protein, wherein the one or more TRD is operably linked to the dCas protein when expressed;
   (iv) a third dCas protein, wherein the one or more TRD is operably linked to the dCas protein when expressed;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene. The first dCas protein may be operably linked to a TRD that increases repressive histone modifications (e.g., KRAB) when expressed, the second dCas protein may be operably linked to a TRD that increases DNA methylation (e.g., DNMT3L) when expressed and the third dCas protein may be operably linked to a TRD that increases DNA methylation (e.g., DNMT3A) when expressed. In some embodiments, the first dCas protein is operably linked to a KRAB domain when expressed, the second dCas protein is operably linked to DNMT3L when expressed and the third dCas protein is operably linked to DNMT3A when expressed (e.g., the one or more nucleic acid encodes a first fusion protein comprising the first dCas protein and a KRAB domain, a second fusion protein comprising the second dCas protein and DNMT3L, and a third fusion protein comprising the third dCas protein and DNMT3A).

In some embodiments, the composition comprises one or more nucleic acid encoding at least:
(a) one or more TRD that increases repressive histone modifications;
(b) one or more TRD that increases DNA methylation;
(c) a DNA-binding domain (e.g., a ZFP or TALE), wherein a TRD according to (a) and/or a TRD according to (b) is operably linked to the DNA-binding domain when expressed; and
(d) a dCas protein, wherein a TRD according to (a) and/or a TRD according to (b) is operably linked to the dCas protein when expressed,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene. In some embodiments, a TRD according to (a) and a TRD according to (b) are operably linked to the DNA-binding domain when expressed, and a TRD according to (a) or a TRD according to (b) is operably linked to the dCas protein when expressed. In some embodiments, the one or more nucleic acid encodes a first dCas protein and a second dCas protein, and a TRD according to (a) is operably linked to the first dCas protein when expressed and a TRD according to (b) is operably linked to the second dCas protein when expressed. In some embodiments, the one or more nucleic acid encodes a first dCas protein and a second dCas protein, and a KRAB domain is operably linked to the first dCas protein when expressed and DNMT3L is operably linked to the second dCas protein when expressed. In some embodiments, the one or more nucleic acid encodes a first dCas protein, a second dCas protein, and a third dCas protein, and a TRD according to (a) is operably linked to the first dCas protein when expressed, a TRD according to (b) is operably linked to the second dCas protein when expressed and a TRD according to (b) is operably linked to the third dCas protein when expressed. In some embodiments, the one or more nucleic acid encodes a first dCas protein, a second dCas protein, and a third dCas protein, and a KRAB domain is operably linked to the first dCas protein when expressed, DNMT3L is operably linked to the second dCas protein when expressed and DNMT3A is operably linked to the third dCas protein when expressed. In some embodiments, a KRAB domain and DNMT3L are operably linked to the DNA-binding domain. In some embodiments, a DNMT3A domain and DNMT3L are operably linked to the DNA-binding domain. In some embodiments, a KRAB domain, DNMT3A and DNMT3L are operably linked to the DNA-binding domain. In some embodiments, a TRD according to (a) or a TRD according to (b) is operably linked to the DNA-binding domain when expressed, and a TRD according to (a) and a TRD according to (b) are operably linked to the dCas protein when expressed. In some embodiments, a TRD according to (a) and a TRD according to (b) are operably linked to the DNA-binding domain when expressed, and a TRD according to (a) and a TRD according to (b) are operably linked to the dCas protein when expressed. In some embodiments, DNMT3L is fused to a histone fragment that recruits an endogenously expressed DNA methyltransferase.

In some embodiments, the composition comprises one or more nucleic acid encoding at least:
(a) one or more TRD that increases repressive histone modifications;
(b) one or more TRD that increases DNA methylation;
(c) a DNA-binding domain (e.g., a ZFP or TALE), wherein a TRD according to (a) and/or a TRD according to (b) is operably linked to the DNA-binding domain when expressed; and
(d) a dCas protein, wherein a TRD according to (a) and/or a TRD according to (b) is operably linked to the dCas protein when expressed,
wherein the composition further comprises a gRNA or a nucleic acid encoding the gRNA, and wherein the gRNA specifically binds to a sequence contained in a regulatory region of the polyglutamine disease-related gene, and the DNA-binding domain specifically binds to a sequence contained in the poly(CAG) tract of the polyglutamine disease-related gene. In some embodiments, a TRD according to (a) and a TRD according to (b) are operably linked to the DNA-binding domain when expressed, and a TRD according to (a) or a TRD according to (b) is operably linked to the dCas protein when expressed. In some embodiments, the one or more nucleic acid encodes a first dCas protein and a second dCas protein, and a TRD according to (a) is operably linked to the first dCas protein when expressed and a TRD according to (b) is operably linked to the second dCas protein when expressed. In some embodiments, the one or more nucleic acid encodes a first dCas protein and a second dCas protein, and a KRAB domain is operably linked to the first dCas protein when expressed and DNMT3L is operably linked to the second dCas protein when expressed. In some embodiments, the one or more nucleic acid encodes a first dCas protein, a second dCas protein, and a third dCas protein, and a TRD according to (a) is operably linked to the first dCas protein when expressed, a TRD according to (b) is operably linked to the second dCas protein when expressed and a TRD according to (b) is operably linked to the third dCas protein when expressed. In some embodiments, the one or more nucleic acid encodes a first dCas protein, a second dCas protein, and a third dCas protein, and a KRAB domain is operably linked to the first dCas protein when expressed, DNMT3L is operably linked to the second dCas protein when expressed and DNMT3A is operably linked to the third dCas protein when expressed. In some embodiments, a KRAB domain and DNMT3L are operably linked to the DNA-binding domain. In some embodiments, a DNMT3A domain and DNMT3L are operably linked to the DNA-binding domain. In some embodiments, a KRAB domain, DNMT3A and DNMT3L are operably linked to the DNA-binding domain. In some embodiments, a TRD according to (a) or a TRD according to (b) is operably linked to the DNA-binding domain when expressed, and a TRD according to (a) and a TRD according to (b) are operably linked to the dCas protein when expressed. In some embodiments, a TRD according to (a) and a TRD according to (b) is operably linked to the DNA-binding domain when expressed, and a TRD according to (a) and a TRD according to (b) are operably linked to the dCas protein when expressed.

In some embodiments, the composition comprises one or more nucleic acid encoding at least:
(a) two or more TRDs that increase repressive histone modifications;
(b) two or more TRDs that increase DNA methylation;
(c) a DNA-binding domain (e.g., a ZFP or TALE), wherein a TRD according to (a) and a TRD according to (b) are operably linked to the DNA-binding domain when expressed; and
(d) a dCas protein, wherein a TRD according to (a) and a TRD according to (b) are operably linked to the dCas protein when expressed,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene. In some embodiments, the two or more TRDs that increase repressive histone modifications are KRAB domains and the two or more TRDs that increase DNA methylation are each DNMT3A and/or DNMT3L. In some embodiments, the DNA-binding domain and dCas protein are each operably linked to a KRAB domain and DNMT3L when expressed (e.g., the one or more nucleic acid encodes a first fusion protein comprising the DNA-binding domain, a KRAB domain and DNMT3L, and a second fusion protein comprising the dCas protein, a KRAB domain and DNMT3L).

In some embodiments, the composition comprises one or more nucleic acid encoding at least:
(a) two or more TRDs that increase repressive histone modifications;
(b) two or more TRDs that increase DNA methylation;
(c) a DNA-binding domain (e.g., a ZFP or TALE), wherein a TRD according to (a) and a TRD according to (b) are operably linked to the DNA-binding domain when expressed; and
(d) a dCas protein, wherein a TRD according to (a) and a TRD according to (b) are operably linked to the dCas protein when expressed,
wherein the composition further comprises a gRNA or a nucleic acid encoding the gRNA, and wherein the gRNA specifically binds to a sequence contained in a regulatory region of the polyglutamine disease-related gene, and the DNA-binding domain specifically binds to a sequence contained in the poly(CAG) tract of the polyglutamine disease-related gene. In some embodiments, the two or more TRDs that increase repressive histone modifications are KRAB domains and the two or more TRDs that increase DNA methylation are each DNMT3A and/or DNMT3L. In some embodiments, the DNA-binding domain and dCas protein are each operably linked to a KRAB domain and DNMT3L when expressed (e.g., the one or more nucleic acid encodes a first fusion protein comprising the DNA-binding domain, a KRAB domain and DNMT3L, and a second fusion protein comprising the dCas protein, a KRAB domain and DNMT3L).

In some embodiments, the composition comprises one or more nucleic acid encoding at least:
(a) two or more TRDs that increase repressive histone modifications;
(b) four or more TRDs that increase DNA methylation;
(c) a DNA-binding domain (e.g., a ZFP or TALE), wherein a TRD according to (a) and two TRDs according to (b) are operably linked to the DNA-binding domain when expressed; and
(d) a dCas protein, wherein a TRD according to (a) and two TRDs according to (b) are operably linked to the dCas protein when expressed,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene. In some embodiments, the DNA-binding domain and dCas protein are each operably linked to a KRAB domain, DNMT3L and DNMT3A when expressed (e.g., the one or more nucleic acid encodes a first fusion protein comprising the DNA-binding domain, a KRAB domain, DNMT3A and DNMT3L, and a second fusion protein comprising the dCas protein, a KRAB domain, DNMT3A and DNMT3L).

In some embodiments, the composition comprises one or more nucleic acid encoding at least:
(a) two or more TRDs that increase repressive histone modifications;
(b) four or more TRDs that increase DNA methylation;
(c) a DNA-binding domain (e.g., a ZFP or TALE), wherein a TRD according to (a) and two TRDs according to (b) are operably linked to the DNA-binding domain when expressed; and
(d) a dCas protein, wherein a TRD according to (a) and two TRDs according to (b) are operably linked to the dCas protein when expressed,
wherein the composition further comprises a gRNA or a nucleic acid encoding the gRNA, and wherein the gRNA specifically binds to a sequence contained in a regulatory region of the polyglutamine disease-related gene, and the DNA-binding domain specifically binds to a sequence contained in the poly(CAG) tract of the polyglutamine disease-related gene. In some embodiments, the DNA-binding domain and dCas protein are each operably linked to a KRAB domain, DNMT3L and DNMT3A when expressed (e.g., the one or more nucleic acid encodes a first fusion protein comprising the DNA-binding domain, a KRAB domain, DNMT3A and DNMT3L, and a second fusion protein comprising the dCas protein, a KRAB domain, DNMT3A and DNMT3L).

In any of the above embodiments, the composition comprises at least two gRNAs that specifically bind to at least two different sequences contained in the regulatory region of the polyglutamine disease-related gene, or one or more nucleic acids encoding the at least two gRNAs. In any of the above embodiments, the one or more nucleic acid encodes at least two DNA-binding domains that bind to at least two different sequences contained in the regulatory region of the polyglutamine disease-related gene, wherein each DNA-binding domain is operably linked to one or more (e.g., 1, 2 or 3) TRD. Each of the two or more constructs may comprise the same TRD(s) or different TRD(s). Each DNA-binding domain may be a ZFP or TALE. In some cases, the DNA-binding domains are ZFPs. In other cases, the DNA-binding domains are TALEs.

In our experience, the combination of a KRAB domain with DNMT3L and, optionally, DNMT3A allows for the most robust transcriptional repression. Thus, embodiments including one or more nucleic acid encoding a KRAB domain, DNMT3L and, optionally, DNMT3A may be most effective. In some embodiments, DNMT3L can be fused to a histone fragment that recruits an endogenously expressed DNA methyltransferase. In some embodiments, the one or more nucleic acid may encode any one of the amino acid sequences provided in Table 3 or a sequence having at least 70, 75, 80, 85, 90, 95, 97, 98 or 99% sequence identity thereto.

**Table 3: Sequences of exemplary TRDs**

| **TRD** | **Amino acid sequence** | **SEQ ID NO.** |
|---|---|---|
| KRAB | RTLVTFKDVFVDFTREEWKLLDTAQQIVYRNVMLENYKNL VSLGYQLTKPDVILRLEKGEEPWLV | 1 |
| Human DNMT3L | AAIPALDPEAEPSMDVILVGSSELSSSVSPGTGRDLIAYE VKANQRNIEDICICCGSLQVHTQHPLFEGGICAPCKDKFL DALFLYDDDGYQSYCSICCSGETLLICGNPDCTRCYCFEC VDSLVGPGTSGKVHAMSNWVCYLCLPSSRSGLLQRRRKWR SQLKAFYDRESENPLEMFETVPVWRRQPVRVLSLFEDIKK ELTSLGFLESGSDPGQLKHVVDVTDTVRKDVEEWGPFDLV YGATPPLGHTCDRPPSWYLFQFHRLLQYARPKPGSPRPFF WMFVDNLVLNKEDLDVASRFLEMEPVTIPDVHGGSLQNAV | 2 |
| | RVWSNIPAIRSRHWALVSEEELSLLAQNKQSSKLAAKWPT KLVKNCFLPLREYFKYFSTELTSSL | |
| DNMT3A | NHDQEFDPPKVYPPVPAEKRKPIRVLSLFDGIATGLLVLK DLGIQVDRYIASEVCEDSITVGMVRHQGKIMYVGDVRSVT QKHIQEWGPFDLVIGGSPCNDLSIVNPARKGLYEGTGRLF FEFYRLLHDARPKEGDDRPFFWLFENVVAMGVSDKRDISR FLESNPVMIDAKEVSAAHRARYFWGNLPGMNRPLASTVND KLELQECLEHGRIAKFSKVRTITTRSNSIKQGKDQHFPVF MNEKEDILWCTEMERVFGFPVHYTDVSNMSRLARQRLLGR SWSVPVIRHLFAPLKEYFACV | 3 |
| *Apodemus sylvaticus* DNMT3L | SPVEIYKTVSAWKRQPIRVLSLFGNIDKELKSLGFLESGS HSEGGTLKYVEDVTNVVRRDVEKWGPFDLLYGSTQPLGGS CDRCPGWYMFQFHRILQYARPRQESQPFFWIFMDNLLLTE DDQMTTVRFLQTEAVTLRDVRSRVLQNAVRVWSNIPGLKS KHEALSPKEEESLQGHVRTRSKLAAPKVDPLVKNCLLPLR EYFKYFSQSSLPL | 112 |

In some embodiments, the composition comprises one or more nucleic acid encoding at least:
(a) a first fusion protein comprising, optionally from N-terminus to the C-terminus, DNMT3A, DNMT3L, a first DNA-binding domain, and a KRAB domain;
(b) a second fusion protein comprising, optionally from N-terminus to the C-terminus, DNMT3A, DNMT3L, a second DNA-binding domain, and a KRAB domain,
wherein the first DNA-binding domain specifically binds to a sequence contained in a regulatory region of the polyglutamine disease-related gene, and the second DNA-binding domain specifically binds to a sequence contained in the poly(CAG) tract of the polyglutamine disease-related gene.

In some embodiments, the composition comprises one or more nucleic acid encoding at least:
(a) a first fusion protein comprising, optionally from N-terminus to the C-terminus, DNMT3A, DNMT3L, a first ZFP, and a KRAB domain;
(b) a second fusion protein comprising, optionally from N-terminus to the C-terminus, DNMT3A, DNMT3L, a second ZFP, and a KRAB domain,
wherein the first ZFP specifically binds to a sequence contained in a regulatory region of the polyglutamine disease-related gene, and the second ZFP specifically binds to a sequence contained in the poly(CAG) tract of the polyglutamine disease-related gene.

In some embodiments, the composition comprises:
(a) a nucleic acid encoding at least a fusion protein comprising, optionally from N-terminus to the C-terminus, DNMT3A, DNMT3L, a dCas protein, and a KRAB domain;
(b) a first guide RNA (gRNA) or a nucleic acid encoding the first gRNA; and
(c) a second gRNA or a nucleic acid encoding the second gRNA,
wherein the first gRNA specifically binds to a sequence contained in a regulatory region of the polyglutamine disease-related gene, and the second gRNA specifically binds to a sequence contained in the poly(CAG) tract of the polyglutamine disease-related gene.

In some embodiments, the composition comprises one or more nucleic acid encoding at least:
(a) a first fusion protein comprising, optionally from N-terminus to the C-terminus, DNMT3A, DNMT3L, a DNA-binding domain, and a KRAB domain;
(b) a second fusion protein comprising, optionally from N-terminus to the C-terminus, DNMT3A, DNMT3L, a dCas protein, and a KRAB domain,
wherein the composition further comprises a gRNA or a nucleic acid encoding the gRNA, and wherein the DNA-binding domain specifically binds to a sequence contained in a regulatory region of the polyglutamine disease-related gene, and the gRNA specifically binds to a sequence contained in the poly(CAG) tract of the polyglutamine disease-related gene.

In some embodiments, the composition comprises one or more nucleic acid encoding at least:
(a) a first fusion protein comprising, optionally from N-terminus to the C-terminus, DNMT3A, DNMT3L, a DNA-binding domain, and a KRAB domain;
(b) a second fusion protein comprising, optionally from N-terminus to the C-terminus, DNMT3A, DNMT3L, a dCas protein, and a KRAB domain,
wherein the composition further comprises a gRNA or a nucleic acid encoding the gRNA, and wherein the gRNA specifically binds to a sequence contained in a regulatory region of the polyglutamine disease-related gene, and the DNA-binding domain specifically binds to a sequence contained in the poly(CAG) tract of the polyglutamine disease-related gene.

In some embodiments, the composition comprises one or more nucleic acid encoding at least:
(a) a first fusion protein comprising, optionally from N-terminus to the C-terminus, DNMT3A, DNMT3L, a ZFP, and a KRAB domain;
(b) a second fusion protein comprising, optionally from N-terminus to the C-terminus, DNMT3A, DNMT3L, a dCas protein, and a KRAB domain,
wherein the composition further comprises a gRNA or a nucleic acid encoding the gRNA, and wherein the ZFP specifically binds to a sequence contained in a regulatory region of the polyglutamine disease-related gene, and the gRNA specifically binds to a sequence contained in the poly(CAG) tract of the polyglutamine disease-related gene.

In some embodiments, the composition comprises one or more nucleic acid encoding at least:
(a) a first fusion protein comprising, optionally from N-terminus to the C-terminus, DNMT3A, DNMT3L, a ZFP, and a KRAB domain;
(b) a second fusion protein comprising, optionally from N-terminus to the C-terminus, DNMT3A, DNMT3L, a dCas protein, and a KRAB domain,
wherein the composition further comprises a gRNA or a nucleic acid encoding the gRNA, and wherein the gRNA specifically binds to a sequence contained in a regulatory region of the polyglutamine disease-related gene, and the ZFP specifically binds to a sequence contained in the poly(CAG) tract of the polyglutamine disease-related gene.

In any of the above embodiments, the fusion protein may further comprise one or more linker between one or more domain. For example, a fusion protein of the present invention may be in accordance with the following structure:
N-DNMT3A- Linker-DNMT3L-Linker-PBD-Linker-KRAB-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-H3-linker-DNMT3L-Linker-PBD-Linker-KRAB-C,
   wherein the N indicates the N-terminus, the C indicates the C-terminus, H3 is a histone H3 tail, and PBD is a polynucleotide-binding domain (e.g., ZFP or dCas protein). Alternatively, a fusion protein of the present invention may be in accordance with the following structure:
N-H3-linker-DNMT3L-Linker-PBD-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-DNMT3L-Linker-PBD-Linker-KRAB-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-DNMT3A-linker-DNMT3L-Linker-PBD-C,
   wherein the N indicates the N-terminus, the C indicates the C-terminus, and PBD is a polynucleotide-binding domain (e.g., ZFP or dCas protein).

In any embodiment of the present invention, one or more nucleic acid may encode one or more fusion protein comprising, from N-terminus to C-terminus, SEQ ID NO: 110 or a sequence having at least 70, 75, 80, 85, 90, 95, 97, 98 or 99% sequence identity to SEQ ID NO: 110, a polynucleotide-binding domain, and SEQ ID NO: 111 or a sequence having at least 70, 75, 80, 85, 90, 95, 97, 98 or 99% sequence identity to SEQ ID NO: 111. The polynucleotide-binding domain may, for example, be a ZFP or dCas protein.

### Construct containing, from N-terminus to C-terminus: DNMT3A, linker, DNMT3L, and linker

NHDQEFDPPKVYPPVPAEKRKPIRVLSLFDGIATGLLVLKDLGIQVDRYIASEVCEDSITVGMV
RHQGKIMYVGDVRSVTQKHIQEWGPFDLVIGGSPCNDLSIVNPARKGLYEGTGRLFFEFYRLL
HDARPKEGDDRPFFWLFENVVAMGVSDKRDISRFLESNPVMIDAKEVSAAHRARYFWGNLP
GMNRPLASTVNDKLELQECLEHGRIAKFSKVRTITTRSNSIKQGKDQHFPVFMNEKEDILWCT
EMERVFGFPVHYTDVSNMSRLARQRLLGRSWSVPVIRHLFAPLKEYFACVSSGNSNANSRGP
SFSSGLVPLSLRGSHMAAIPALDPEAEPSMDVILVGSSELSSSVSPGTGRDLIAYEVKANQRNIE
DICICCGSLQVHTQHPLFEGGICAPCKDKFLDALFLYDDDGYQSYCSICCSGETLLICGNPDCT
RCYCFECVDSLVGPGTSGKVHAMSNWVCYLCLPSSRSGLLQRRRKWRSQLKAFYDRESENP
LEMFETVPVWRRQPVRVLSLFEDIKKELTSLGFLESGSDPGQLKHVVDVTDTVRKDVEEWGP
FDLVYGATPPLGHTCDRPPSWYLFQFHRLLQYARPKPGSPRPFFWMFVDNLVLNKEDLDVAS
RFLEMEPVTIPDVHGGSLQNAVRVWSNIPAIRSRHWALVSEEELSLLAQNKQSSKLAAKWPT
KLVKNCFLPLREYFKYFSTELTSSLGGPSSGAPPPSGGSPAGSPTSTEEGTSESATPESGPGTSTE
PSEGSAPGSPAGSPTSTEEGTSTEPSEGSAPGTSTEPSE (SEQ ID NO: 110)

### Construct containing, from N-terminus to C-terminus: linker and KRAB domain

PKKKRKVGVDGSSGSETPGTSESATPESRTLVTFKDVFVDFTREEWKLLDTAQQIVYRNVML
ENYKNLVSLGYQLTKPDVILRLEKGEEPWLV (SEQ ID NO: 111)

In some embodiments, the histone fragment that is capable of recruiting an endogenously expressed DNA methyltransferase comprises SEQ ID NO: 113 or a sequence having at least 70, 75, 80, 85, 90, 95, 97, 98 or 99% sequence identity to SEQ ID NO: 113.

### H3 histone tail

ARTKQTARKSTGGKAPRKQLATKAARKSAP (SEQ ID NO: 113)

In some embodiments, the one or more nucleic acid encodes SEQ ID NO: 114 or a sequence having at least 70, 75, 80, 85, 90, 95, 97, 98 or 99% sequence identity to SEQ ID NO: 114.

### Construct containing, from N-terminus to C-terminus: H3 histone tail, linker and DNMT3L

ARTKQTARKSTGGKAPRKQLATKAARKSAPGGASSGAGSSSGGSAAGSGSGGASSGAASSSA
GSAAGSGSSPVEIYKTVSAWKRQPIRVLSLFGNIDKELKSLGFLESGSHSEGGTLKYVEDVTN
VVRRDVEKWGPFDLLYGSTQPLGGSCDRCPGWYMFQFHRILQYARPRQESQPFFWIFMDNL
LLTEDDQMTTVRFLQTEAVTLRDVRSRVLQNAVRVWSNIPGLKSKHEALSPKEEESLQGHVR
TRSKLAAPKVDPLVKNCLLPLREYFKYFSQSSLPL (SEQ ID NO: 114)

In some embodiments, the first DNA-binding domain is a ZFP. In some embodiments, the second DNA-binding domain is a ZFP. In some embodiments, the first DNA-binding domain and the second DNA-binding domain are ZFPs.

In any of the above embodiments, the polyglutamine disease-related gene is *HTT* or *ATXN2.* In some embodiments, the polyglutamine disease-related gene is *HTT.*

In some embodiments, the sequence contained in the regulatory region of *HTT* to which the DNA-binding domain (e.g., ZFP) specifically binds to comprises any one of SEQ ID NOs: 35-66. Exemplary ZFPs that can bind SEQ ID NOs: 35-66 are provided in Table 4. In some embodiments, the DNA-binding domain comprises any one of SEQ ID NOs: 4-34 or a sequence that has at least 70, 75, 80, 85, 90, 95, 97, 98 or 99% sequence identity with any one of SEQ ID NOs: 4-34 that specifically binds to the same sequence.

**Table 4: ZFPs targeting the promoter/enhancer region of HTT and their binding site**

| **ZFP sequence** | **Binding site of ZFP sequence** |
|---|---|
| LEGIHGVPAAMAERPFQCRICMRNFSRSDH LSQHIRTHTGEKPFACDICGRKFAQNATRT KHTKIHTGSQKPFQCRICMRNFSDRSNLSR HIRTHTGEKPFACDICGRKFAARWYLDKHT KIHTGSQKPFQCRICMRNFSDRSALSRHIR THTGEKPFACDICGRKFAQSSDLTRHTKIH LRQKDAAR (SEQ ID NO: 4) | CCGCAGGGCTGTCCGGGT (SEQ ID NO: 35) |
| LEGIHGVPAAMAERPFQCRICMRNFSRSDT LSQHIRTHTGEKPFACDICGRKFAHNSHRT KHTKIHTGSQKPFQCRICMRNFSRSDNLSA HIRTHTGEKPFACDICGRKFAQNRDRKNHT KIHTGSQKPFQCRICMRNFSQSGHLARHIR THTGEKPFACDICGRKFARKWTLQGHTKIH LRQKDAAR (SEQ ID NO: 5) | AGGGCTGTCCGGGTGAGT (SEQ ID NO: 36) |
| LEGIHGVPAAMAERPFQCRICMRNFSTSSN RKTHIRTHTGEKPFACDICGRKFARSDDLS RHTKIHTGSQKPFQCRICMRNFSLRHHLTR HIRTHTGEKPFACDICGRKFARRDTLLDHT KIHTGSQKPFQCRICMRNFSWRSCRSAHIR THTGEKPFACDICGRKFADSSHRTRHTKIH LRQKDAAR (SEQ ID NO: 6) | GGCTGTCCGGGTGAGTAT (SEQ ID NO: 37) |
| LEGIHGVPAAMAERPFQCRICMRNFSRSDT LSAHIRTHTGEKPFACDICGRKFADKRTRT THTKIHTGSQKPFQCRICMRNFSRSDNLAR HIRTHTGEKPFACDICGRKFAQWNYRGSHT KIHTGSQKPFQCRICMRNFSDRSHLTRHIR THTGEKPFACDICGRKFADRSYRNTHTKIH LRQKDAAR (SEQ ID NO: 7) | CTCGGCTCAGAGTCCACG (SEQ ID NO: 38) |
| LEGIHGVPAAMAERPFQCRICMRNFSRSDA LSEHIRTHTGEKPFACDICGKKFATSSSRK KHTKIHTGSQKPFQCRICMRNFSDRSDLSR HIRTHTGEKPFACDICGRKFARRDTLRSHT KIHTGSQKPFQCRICMRNFSRSDHLSTHIR THTGEKPFACDICGRKFAQSAHRITHTKIH LRQKDAAR (SEQ ID NO: 8) | CCGGCTGTCGCCCCGCTC (SEQ ID NO: 39) |
| LEGIHGVPAAMAERPFQCRICMRNFSLRHH LTRHIRTHTGEKPFACDICGRKFAQSADRK KHTKIHTGSQKPFQCRICMRNFSYDYGRYT HIRTHTGEKPFACDICGRKFADRSHLARHT KIHTGSQKPFQCRICMRNFSDRSNLSRHIR THTGEKPFACDICGRKFARQDSRSQHTKIH LRQKDAAR (SEQ ID NO: 9) | CTCCAGGCGTCGGCGGGG (SEQ ID NO: 40) |
| LEGIHGVPAAMAERPFQCRICMRNFSHNHD LRNHIRTHTGEKPFACDICGRKFATSGNLT RHTKIHTGSQKPFQCRICMRNFSRSDHLSR HIRTHTGEKPFACDICGRKFARSDDLTRHT KIHTGSQKPFQCRICMRNFSRSDDLSKHIR THTGEKPFACDICGRKFARSADLSRHTKIH LRQKDAAR (SEQ ID NO: 10) | GCGTCGGCGGGGGATCCT (SEQ ID NO: 41) |
| LEGIHGVPAAMAERPFQCRICMRNFSTNYG RTEHIRTHTGEKPFACDICGRKFATSGHLS RHTKIHTGSQKPFQCRICMRNFSRSDVLST HIRTHTGEKPFACDICGRKFAVRSRLRRHT KIHTGSQKPFQCRICMRNFSERGTLARHIR THTGEKPFACDICGRKFAQSSDLRRYTKIH LRQKDAAR (SEQ ID NO: 11) | GCTGCCGGGACGGGTCCA (SEQ ID NO: 42) |
| LEGIHGVPAAMAERPFQCRICMRNFSRSAH LSRHIRTHTGEKPFACDICGRKFADRSDLS RHTKIHTGSQKPFQCRICMRNFSHRWHLQT HIRTHTGEKPFACDICGRKFAASSNRIKHT KIHTGSQKPFQCRICMRNFSDRSNLSRHIR THTGEKPFACDICGRKFARSDHLSRHTKIH LRQKDAAR (SEQ ID NO: 12) | GGGGACTGCCGTGCCGGG (SEQ ID NO: 43) |
| LEGIHGVPAAMAERPFQCRICMRNFSGVKG LTVHIRTHTGEKPFACDICGRKFADRSNLT RHTKIHTGSQKPFQCRICMRNFSRSHSLLR HIRTHTGEKPFACDICGRKFAHRKSLSRHT KIHTGSQKPFQCRICMRNFSRSDTLSEHIR THTGEKPFACDICGRKFAKLCNRKCHTKIH LRQKDAAR (SEQ ID NO: 13) | CAGCCGGCCGTGGACTCT (SEQ ID NO: 44) |
| LEGIHGVPAAMAERPFQCRICMRNFSDTGD LRRHIRTHTGEKPFACDICGRKFADRSNLT RHTKIHTGSQKPFQCRICMRNFSRSDDLTR HIRTHTGEKPFACDICGRKFAHRRSRDQHT KIHTGSQKPFQCRICMRNFSDSGGLSKHIR THTGEKPFACDICGRKFAQSGHLSRHTKIH LRQKDAAR (SEQ ID NO: 14) | GGATCCCCCGCCGACGCC (SEQ ID NO: 45) |
| LEGIHGVPAAMAERPFQCRICMRNFSRSAH LSRHIRTHTGEKPFACDICGRKFADRSNLT RHTKIHTGSQKPFQCRICMRNFSRSDALST HIRTHTGEKPFACDICGRKFADRSTRTKHT KIHTGSQKPFQCRICMRNFSRSAHLSRHIR THTGEKPFACDICGRKFADRSNLTRHTKIH LRQKDAAR (SEQ ID NO: 15) | GACGGGGCCATGGACGGG (SEQ ID NO: 46) |
| LEGIHGVPAAMAERPFQCRICMRNFSRSDN LSEHIRTHTGEKPFACDICGRKFATSANLS RHTKIHTGSQKPFQCRICMRNFSRSDHLSA HIRTHTGEKPFACDICGRKFARSANLTRHT KIHTGSQKPFQCRICMRNFSRSDDLTRHIR THTGEKPFACDICGRKFAMSYQLGRHTKIH LRQKDAAR (SEQ ID NO: 16) | GGGGCGGAGCGGGATAGG (SEQ ID NO: 47) |
| LEGIHGVPAAMAERPFQCRICMRNFSRSDN LSTHIRTHTGEKPFACDICGRKFARSTHRT QHTKIHTGSQKPFQCRICMRNFSWKCQLPI HIRTHTGEKPFACDICGRKFADRSNLTRHT KIHTGSQKPFQCRICMRNFSRSAHLSRHIR THTGEKPFACDICGRKFADRSNLTRHTKIH LRQKDAAR (SEQ ID NO: 17) | GACGGGGACATTAGGCAG (SEQ ID NO: 48) |
| LEGIHGVPAAMAERPFQCRICMRNFSRSDH LSRHIRTHTGEKPFACDICGRKFAQSSDLR RHTKIHTGSQKPFQCRICMRNFSTSGNLTR HIRTHTGEKPFACDICGRKFADRSHLARHT KIHTGSQKPFQCRICMRNFSRSDHLSEHIR THTGEKPFACDICGRKFARSAHLSRHTKIH LRQKDAAR (SEQ ID NO: 18) | GGGCGGGGCGATGCTGGG (SEQ ID NO: 49) |
| LEGIHGVPAAMAERPFQCRICMRNFSRSDN LARHIRTHTGEKPFACDICGRKFADRSHLA RHTKIHTGSQKPFQCRICMRNFSHKQHRDA HIRTHTGEKPFACDICGRKFARSANLTRHT KIHTGSQKPFQCRICMRNFSRSDNLSAHIR THTGEKPFACDICGRKFARSDHRITHTKIH LRQKDAAR (SEQ ID NO: 19) | AGGTAGGGGCGTGGCGAG (SEQ ID NO: 50) |
| LEGIHGVPAAMAERPFQCRICMRNFSRSDN LARHIRTHTGEKPFACDICGRKFARSDHLT QHTKIHTGSQKPFQCRICMRNFSQSGSLTR HIRTHTGEKPFACDICGRKFARSDALTQHT KIHTGSQKPFQCRICMRNFSSSGHLPPHIR THTGEKPFACDICGRKFADRSNLTRHTKIH LRQKDAAR (SEQ ID NO: 20) | GACTGCATGGTAAGGGAG (SEQ ID NO: 51) |
| LEGIHGVPAAMAERPFQCRICMRNFSRSDN LARHIRTHTGEKPFACDICGRKFADRSNLT RHTKIHTGSQKPFQCRICMRNFSRSDHLSE HIRTHTGEKPFACDICGRKFAQSNHRKTHT KIHTGSQKPFQCRICMRNFSRSADLTRHIR THTGEKPFACDICGRKFARSDDLTRHTKIH LRQKDAAR (SEQ ID NO: 21) | GCGGCGAGGCGGGACGAG (SEQ ID NO: 52) |
| LEGIHGVPAAMAERPFQCRICMRNFSQSGN LARHIRTHTGEKPFACDICGRKFAWLSSLG IHTKIHTGSQKPFQCRICMRNFSRPYTLRL HIRTHTGEKPFACDICGRKFARRSDLKRHT KIHTGSQKPFQCRICMRNFSQRSNLVRHIR THTGEKPFACDICGRKFADRSHLARHTKIH LRQKDAAR (SEQ ID NO: 22) | GGCGAAGCTGTGTGTGAA (SEQ ID NO: 53) |
| LEGIHGVPAAMAERPFQCRICMRNFSRSAH LSRHIRTHTGEKPFACDICGRKFARSDDLT RHTKIHTGSQKPFQCRICMRNFSDRSHLTR HIRTHTGEKPFACDICGRKFAQSGNLARHT KIHTGSQKPFQCRICMRNFSDRSHLTRHIR THTGEKPFACDICGRKFARRDTLLDHTKIH LRQKDAAR (SEQ ID NO: 23) | CCGGGCGAAGGCGCGGGG (SEQ ID NO: 54) |
| LEGIHGVPAAMAERPFQCRICMRNFSLQCC LYHHIRTHTGEKPFACDICGRKFADRSNLT RHTKIHTGSQKPFQCRICMRNFSQSSDLSR HIRTHTGEKPFACDICGRKFAHRKSLSRHT KIHTGSQKPFQCRICMRNFSRSDTLSAHIR THTGEKPFACDICGRKFAAQCCLFHHTKIH LRQKDAAR (SEQ ID NO: 24) | AGCAAGGCCGCTGACAGC (SEQ ID NO: 55) |
| LEGIHGVPAAMAERPFQCRICMRNFSRSDH LSTHIRTHTGEKPFACDICGRKFAQSSDLR RHTKIHTGSQKPFQCRICMRNFSTNQNLTV HIRTHTGEKPSACDICGRKFATSGHLSRHT KIHTGSQKPFQCRICMRNFSRSDHLSEHIR THTGEKPFACDICGRKFADRSNRKTHTKIH LRQKDAAR (SEQ ID NO: 25) | CACCGGGGCAATGAATGG (SEQ ID NO: 56) |
| LEGIHGVPAAMAERPFQCRICMRNFSQSGH LARHIRTHTGEKPFACDICGRKFADKRDRR THTKIHTGSQKPFQCRICMRNFSDRSALSR YIRTHTGEKPFACDICGRKFAQSGDLTRHT KIHTGSQKPFQCRICMRNFSRSDSLSVHIR THTGEKPFACDICGRKFADRSHLARHTKIH LRQKDAAR (SEQ ID NO: 26) | GGCACGGCAGTCCCCGGA (SEQ ID NO: 57) |
| LEGIHGVPAAMAERPFQCRICMRNFSRSDD LSRHIRTHTGEKPFACDICGRKFARNDNRT KHTKIHTGSQKPFQCRICMRNFSRSDDLTR HIRTHTGEKPFACDICGRKFARSDDRKTHT KIHTGSQKPFQCRICMRNFSRSADLTRHIR THTGEKPFACDICGRKFAQSSDLRRHTKIH LRQKDAAR (SEQ ID NO: 27) | ACGCTGCGCCGGCGGAGGCG (SEQ ID NO: 58) |
| LEGIHGVPAAMAERPFQCRICMRNFSRSDH LSEHIRTHTGEKPFACDICGRKFAQNAHRK THTKIHTGSQKPFQCRICMRNFSQSSDLSR HIRTHTGEKPFACDICGRKFAHRSTRNRHT KIHTGSQKPFQCRICMRNFSQSSDLSRHIR THTGEKPFACDICGRKFAHRSTRNRHTKIH LRQKDAAR (SEQ ID NO: 28) | GCTGCTGCTGCTGGAAGG (SEQ ID NO: 59) |
| LEGIHGVPAAMAERPFQCRICMRNFSRSDN LARHIRTHTGEKPFACDICGRKFAWRGDRV KHTKIHTGSQKPFQCRICMRNFSDRSNLSR HIRTHTGEKPFACDICGRKFATSGSLTRHT KIHTGSQKPFQCRICMRNFSERGTLARHIR THTGEKPFACDICGRKFARSDDRKTHTKIH LRQKDAAR (SEQ ID NO: 29) | CCGGCCGTGGACTCTGAG (SEQ ID NO: 60) |
| LEGIHGVPAAMAERPFQCRICMRNFSRSDA LSRHIRTHTGEKPFACDICGRKFADRSHLA RHTKIHTGSQKPFQCRICMRNFSRSDHLSR HIRTHTGEKPFACDICGRKFAQSSDLTRHT KIHTGSQKPFQCRICMRNFSTSGNLTRHIR THTGEKPFACDICGRKFADRSHLARHTKIH LRQKDAAR (SEQ ID NO: 30) | GGCGATGCGGGGGGCGTG (SEQ ID NO: 61) |
| LEGIHGVPAAMAERPFQCRICMRNFSQSGH LQRHIRTHTGEKPFACDICGRKFATSGNLT RHTKIHTGSQKPFQCRICMRNFSQSGDLTR HIRTHTGEKPFACDICGRKFADRSHLARHT KIHTHPRAPIPKPFQCRICMRNFSRSDVLS THIRTHTGEKPFACDICGRKFAVRSRLRRH TKIHLRQKDAAR (SEQ ID NO: 31) | GGGACGGGTCCAaGATGGA (SEQ ID NO: 62) |
| LEGIHGVPAAMAERPFQCRICMRNFSRSDN LARHIRTHTGEKPFACDICGRKFAWRGDRV KHTKIHTGSQKPFQCRICMRNFSDRSDLSR HIRTHTGEKPFACDICGRKFARSDALARHT KIHTGSQKPFQCRICMRNFSERGTLARHIR THTGEKPFACDICGRKFARSDDRKTHTKIH LRQKDAAR (SEQ ID NO: 32) | CCGGCCGTGGACTCTGAG (SEQ ID NO: 63) |
| LEGIHGVPAAMAERPFQCRICMRNFSDRST LRQHIRTHTGEKPFACDICGRKFADRSDLS RHTKIHTGSQKPFQCRICMRNFSQSSTRAR HIRTHTGEKPFACDICGRKFARSDTLSEHT KIHTGSQKPFQCRICMRNFSHRRSRWGHIR THLRQKDAAR (SEQ ID NO: 33) | GTCCCGGCAGCCCCC (SEQ ID NO: 64) |
| LEGIHGVPAAMAERPFQCRICMRNFSDRSD LSRHIRTHTGEKPFACDICGRKFARRDTLR SHTKIHTGSQKPFQCRICMRNFSRSDHLST HIRTHTGEKPFACDICGRKFAQSAHRITHT KIHTGSQKPFQCRICMRNFSQSGDLTRHIR THTGEKPFACDICGRKFADRSHLARHTKIH LRQKDAAR (SEQ ID NO: 34) | GGTCCAAGATGGACGGCC (SEQ ID NO: 65) |

In some embodiments, the sequence contained in the poly(CAG) tract of *HTT* to which the DNA-binding domain (e.g., ZFP) specifically binds to comprises SEQ ID NO: 99 or SEQ ID NO: 105. Exemplary ZFPs that can bind to a sequence comprising SEQ ID NO: 99 or SEQ ID NO: 105 are provided in Table 5. In some embodiments, the DNA-binding domain comprises any one of SEQ ID NOs: 66-93 or a sequence that has at least 70, 75, 80, 85, 90, 95, 97, 98 or 99% sequence identity with any one of SEQ ID NOs: 66-93 that specifically binds to the same sequence. In some embodiments, the DNA-binding domain comprises any one of SEQ ID NOs: 66-77, 79 and 82-93 or a sequence that has at least 70, 75, 80, 85, 90, 95, 97, 98 or 99% sequence identity with any one of SEQ ID NOs: 66-77, 79 and 82-93 that specifically binds to the same sequence. In some embodiments, the DNA-binding domain comprises SEQ ID NO: 74 or SEQ ID NO: 82 or a sequence that has at least 70, 75, 80, 85, 90, 95, 97, 98 or 99% sequence identity with SEQ ID NO: 74 or SEQ ID NO: 82 that specifically binds to the same sequence.

**Table 5: ZFPs targeting the poly(CAG) tract of pathogenic HTT alleles and the binding site of said ZFPs**

| **ID** | **ZFP sequence** | **Binding site of ZFP sequence** |
|---|---|---|
| HTT_EXP_1 | MAPKKKRKVGIHGVPAAMAERPFQCRICMR NFSQSSDLSRHIRTHTGEKPFACDICGRKF AQWSTRKRHTKIHTGSQKPFQCRICMRNFS QSSDLSRHIRTHTGEKPFACDICGRKFAQW STRKRHTKIHTGSQKPFQCRICMRNFSQSS DLSRHIRTHTGEKPFACDICGRKFAQWSTR KRHTKIHLRQKDAARGSGMDAKSLTAW (SEQ ID NO: 66) | GCAGCAGCAGCAGCAGCA (SEQ ID NO: 94) |
| HTT_EXP_2 | MAPKKKRKVGIHGVPAAMAERPFQCRICMR NFSQSSDLSRHIRTHTGEKPFACDICGRKF AQWSTRKRHTKIHTGSQKPFQCRICMRNFS QSSDLSRHIRTHTGEKPFACDICGRKFAQW STRKRHTKIHTGEKPFQCRICMRKFAQSGD LTRHTKIHLRQKDAARGSGMDAKSLTAW (SEQ ID NO: 67) | GCAGCAGCAGCAGCA (SEQ ID NO: 95) |
| HTT_EXP_3 | MAPKKKRKVGIHGVPAAMAERPFQCRICMR NFSQSSDLSRHIRTHTGEKPFACDICGRKF AQWSTRKRHTKIHTGSQKPFQCRICMRKFA QSGDLTRHTKIHTGEKPFQCRICMRNFSQS SDLSRHIRTHTGEKPFACDICGRKFAQWST RKRHTKIHLRQKDAARGSGMDAKSLTAW (SEQ ID NO: 68) | GCAGCAGCAGCAGCA (SEQ ID NO: 95) |
| HTT_EXP_4 | MAPKKKRKVGIHGVPAAMAERPFQCRICMR KFAQSGDLTRHTKIHTGEKPFQCRICMRNF SQSSDLSRHIRTHTGEKPFACDICGRKFAQ WSTRKRHTKIHTGSQKPFQCRICMRNFSQS SDLSRHIRTHTGEKPFACDICGRKFAQWST RKRHTKIHLRQKDAARGSGMDAKSLTAW (SEQ ID NO: 69) | GCAGCAGCAGCAGCA (SEQ ID NO: 95) |
| HTT_EXP_5 | MAPKKKRKVGIHGVPAAMAERPFQCRICMR NFSRSDNLSEHIRTHTGEKPFACDICGRKF AKRCNLRCHTKIHTHPRAPIPKPFQCRICM RNFSQSSDLSRHIRTHTGEKPFACDICGRK FAQWSTRKRHTKIHTGSQKPFQCRICMRNF SQSSDLSRHIRTHTGEKPFACDICGRKFAQ WSTRKRHTKIHLRQKDAARGSGMDAKSLTA W (SEQ ID NO: 70) | GCAGCAGCAGCAgCAGCAG (SEQ ID NO: 96) |
| HTT_EXP_6 | MAPKKKRKVGIHGVPAAMAERPFQCRICMR NFSRSDNLSEHIRTHTGEKPFACDICGRKF AKRCNLRCHTKIHTGSQKPFQCRICMRNFS RSDNLSEHIRTHTGEKPFACDICGRKFAKR CNLRCHTKIHTHPRAPIPKPFQCRICMRNF SQSSDLSRHIRTHTGEKPFACDICGRKFAQ WSTRKRHTKIHLRQKDAARGSGMDAKSLTA W (SEQ ID NO: 71) | GCAGCAgCAGCAGCAGCAG (SEQ ID NO: 96) |
| HTT_EXP_7 | MAPKKKRKVGIHGVPAAMAERPFQCRICMR NFSRSDNLSEHIRTHTGEKPFACDICGRKF AKRCNLRCHTKIHTHPRAPIPKPFQCRICM RNFSQSSDLSRHIRTHTGEKPFACDICGRK FAQWSTRKRHTKIHTGEKPFQCRICMRKFA QSGDLTRHTKIHLRQKDAARGSGMDAKSLT AW (SEQ ID NO: 72) | GCAGCAGCAgCAGCAG (SEQ ID NO: 97) |
| HTT_EXP_8 | MAPKKKRKVGIHGVPAAMAERPFQCRICMR NFSRSDNLSEHIRTHTGEKPFACDICGRKF AKRCNLRCHTKIHTHPRAPIPKPFQCRICM RKFAQSGDLTRHTKIHTGEKPFQCRICMRN FSQSSDLSRHIRTHTGEKPFACDICGRKFA QWSTRKRHTKIHLRQKDAARGSGMDAKSLT AW (SEQ ID NO: 73) | GCAGCAGCAgCAGCAG (SEQ ID NO: 97) |
| HTT_EXP_9 | MAPKKKRKVGIHGVPAAMAERPFQCRICMR NFSQSSDLSRHIRTHTGEKPFACDICGRKF AQWSTRKRHTKIHTPNPHRRTDPSHKPFQC RICMRNFSRSDNLSEHIRTHTGEKPFACDI CGRKFAKRCNLRCHTKIHTGSQKPFQCRIC MRNFSQSSDLSRHIRTHTGEKPFACDICGR KFAQWSTRKRHTKIHLRQKDAARGSGMDAK SLTAW (SEQ ID NO: 74) | GCAGCAgCAGCAGcaGCAGCA (SEQ ID NO: 98) |
| HTT_EXP_10 | MAPKKKRKVGIHGVPAAMAERPFQCRICMR NFSQSGDLTRHIRTHTGEKPFACDICGRKF AQSGDLTRHTKIHTGSQKPFQCRICMRNFS RSDNLSEHIRTHTGEKPFACDICGRKFAKR CNLRCHTKIHTHPRAPIPKPFQCRICMRNF SQSGDLTRHIRTHTGEKPFACDICGRKFAQ SGDLTRHTKIHLRQKDAARGSGMDAKSLTA W (SEQ ID NO: 75) | GCAGCAgCAGCAGcaGCAGCA (SEQ ID NO: 98) |
| HTT_EXP_11 | MAPKKKRKVGIHGVPAAMAERPFQCRICMR NFSRSDNLSEHIRTHTGEKPFACDICGRKF ARLWNRKQHTKIHTHPRAPIPKPFQCRICM RNFSQSSDLSRHIRTHTGEKPFACDICGRK FAQWSTRKRHTKIHLRQKDAARGSGMDAKS LTAW (SEQ ID NO: 76) | GCAGCAgCAGCAG (SEQ ID NO: 99) |
| HTT_EXP_12 | MAPKKKRKVGIHGVPAAMAERPFQCRICMR NFSRSDNLSEHIRTHTGEKPFACDICGRKF AKRCNLRCHTKIHTHPRAPIPKPFQCRICM RNFSQSGDLTRHIRTHTGEKPFACDICGRK FAQSGDLTRHTKIHLRQKDAARGSGMDAKS LTAW (SEQ ID NO: 77) | GCAGCAgCAGCAG (SEQ ID NO: 99) |
| HTT_EXP_13 | MAPKKKRKVGIHGVPAAMAERPFQCRICMR NFSRSDNLSEHIRTHTGEKPFACDICGRKF AKRCNLRCHTKIHTGSQKPFQCRICMRNFS RSDNLSEHIRTHTGEKPFACDICGRKFAKR CNLRCHTKIHTGSQKPFQCRICMRNFSRSD NLSEHIRTHTGEKPFACDICGRKFAKRCNL RCHTKIHLRQKDAARGSGMDAKSLTAW (SEQ ID NO: 78) | CAGCAGCAGCAGCAGCAG (SEQ ID NO: 100) |
| HTT_EXP_14 | MAPKKKRKVGIHGVPAAMAERPFQCRICMR NFSQSSDLSRHIRTHTGEKPFACDICGRKF AQWSTRKRHTKIHTGSQKPFQCRICMRNFS QSSDLSRHIRTHTGEKPFACDICGRKFAQW STRKRHTKIHTPNPHRRTDPSHKPFQCRIC MRNFSRSDNLSEHIRTHTGEKPFACDICGR KFAKRCNLRCHTKIHLRQKDAARGSGMDAK SLTAW (SEQ ID NO: 79) | CAGCAGcaGCAGCAGCAGCA (SEQ ID NO: 101) |
| HTT_EXP_15 | MAPKKKRKVGIHGVPAAMAERPFQCRICMR NFSQSSDLSRHIRTHTGEKPFACDICGRKF AQWSTRKRHTKIHTPNPHRRTDPSHKPFQC RICMRNFSRSDNLSEHIRTHTGEKPFACDI CGRKFAKRCNLRCHTKIHTGSQKPFQCRIC MRNFSRSDNLSEHIRTHTGEKPFACDICGR KFAKRCNLRCHTKIHLRQKDAARGSGMDAK SLTAW (SEQ ID NO: 80) | CAGCAGCAGCAGcaGCAGCA (SEQ ID NO: 101) |
| HTT_EXP_16 | MAPKKKRKVGIHGVPAAMAERPFQCRICMR NFSRSDNLSEHIRTHTGEKPFACDICGRKF AKRCNLRCHTKIHTGSQKPFQCRICMRNFS QSSDLSRHIRTHTGEKPFACDICGRKFAQW STRKRHTKIHTGSQKPFQCRICMRNFSRSD NLSEHIRTHTGEKPFACDICGRKFAKRCNL RCHTKIHLRQKDAARGSGMDAKSLTAW (SEQ ID NO: 81) | CAGCAGcaGCAGCAgCAGCAG (SEQ ID NO: 102) |
| HTT_EXP_17 | MAPKKKRKVGIHGVPAAMAERPFQCRICMR NFSRSDNLSEHIRTHTGEKPFACDICGRKF AKRCNLRCHTKIHTHPRAPIPKPFQCRICM RNFSQSGDLTRHIRTHTGEKPFACDICGRK FAQSGDLTRHTKIHTPNPHRRTDPSHKPFQ CRICMRNFSRSDNLSEHIRTHTGEKPFACD ICGRKFAKRCNLRCHTKIHLRQKDAARGSG MDAKSLTAW (SEQ ID NO: 82) | CAGCAGcaGCAGCAgCAGCAG (SEQ ID NO: 102) |
| HTT_EXP_18 | MAPKKKRKVGIHGVPAAMAERPFQCRICMR NFSQSSDLSRHIRTHTGEKPFACDICGRKF AHRSTRNRHTKIHTGSQKPFQCRICMRNFS QSSDLSRHIRTHTGEKPFACDICGRKFAHR STRNRHTKIHTGSQKPFQCRICMRNFSQSS DLSRHIRTHTGEKPFACDICGRKFAHRSTR | GCTGCTGCTGCTGCTGCT (SEQ ID NO: 103) |
| | NRHTKIHLRQKDAARGSGMDAKSLTAW (SEQ ID NO: 83) | |
| HTT_EXP_19 | MAPKKKRKVGIHGVPAAMAERPFQCRICMR NFSRSDTLSEHIRTHTGEKPFACDICGRKF ARRWTLVGHTKIHTHPRAPIPKPFQCRICM RNFSQSSDLSRHIRTHTGEKPFACDICGRK FAHRSTRNRHTKIHTGSQKPFQCRICMRNF SQSSDLSRHIRTHTGEKPFACDICGRKFAH RSTRNRHTKIHLRQKDAARGSGMDAKSLTA W (SEQ ID NO: 84) | GCTGCTGCTGCTgCTGCTG (SEQ ID NO: 104) |
| HTT_EXP_20 | MAPKKKRKVGIHGVPAAMAERPFQCRICMR NFSQSSDLSRHIRTHTGEKPFACDICGRKF AHRSTRNRHTKIHTGSQKPFQCRICMRNFS QSSDLSRHIRTHTGEKPFACDICGRKFAHR STRNRHTKIHTGEKPFQCRICMRKFAQSSD LSRHTKIHLRQKDAARGSGMDAKSLTAW (SEQ ID NO: 85) | GCTGCTGCTGCTGCT (SEQ ID NO: 105) |
| HTT_EXP_21 | MAPKKKRKVGIHGVPAAMAERPFQCRICMR NFSQSSDLSRHIRTHTGEKPFACDICGRKF AHRSTRNRHTKIHTGSQKPFQCRICMRKFA QSSDLSRHTKIHTGEKPFQCRICMRNFSQS SDLSRHIRTHTGEKPFACDICGRKFAHRST RNRHTKIHLRQKDAARGSGMDAKSLTAW (SEQ ID NO: 86) | GCTGCTGCTGCTGCT (SEQ ID NO: 105) |
| HTT_EXP_22 | MAPKKKRKVGIHGVPAAMAERPFQCRICMR KFAQSSDLSRHTKIHTGEKPFQCRICMRNF SQSSDLSRHIRTHTGEKPFACDICGRKFAH RSTRNRHTKIHTGSQKPFQCRICMRNFSQS SDLSRHIRTHTGEKPFACDICGRKFAHRST RNRHTKIHLRQKDAARGSGMDAKSLTAW (SEQ ID NO: 87) | GCTGCTGCTGCTGCT (SEQ ID NO: 105) |
| HTT_EXP_23 | MAPKKKRKVGIHGVPAAMAERPFQCRICMR NFSRSDTLSEHIRTHTGEKPFACDICGRKF ARRWTLVGHTKIHTGSQKPFQCRICMRNFS RSDTLSEHIRTHTGEKPFACDICGRKFARR WTLVGHTKIHTHPRAPIPKPFQCRICMRNF SQSSDLSRHIRTHTGEKPFACDICGRKFAH RSTRNRHTKIHLRQKDAARGSGMDAKSLTA W (SEQ ID NO: 88) | GCTGCTgCTGCTGCTGCTG (SEQ ID NO: 104) |
| HTT_EXP_24 | MAPKKKRKVGIHGVPAAMAERPFQCRICMR NFSRSDTLSEHIRTHTGEKPFACDICGRKF ARRWTLVGHTKIHTHPRAPIPKPFQCRICM RNFSQSSDLSRHIRTHTGEKPFACDICGRK FAHRSTRNRHTKIHTGEKPFQCRICMRKFA QSSDLSRHTKIHLRQKDAARGSGMDAKSLT AW (SEQ ID NO: 89) | GCTGCTGCTgCTGCTG (SEQ ID NO: 106) |
| HTT_EXP_25 | MAPKKKRKVGIHGVPAAMAERPFQCRICMR NFSRSDTLSEHIRTHTGEKPFACDICGRKF ARRWTLVGHTKIHTHPRAPIPKPFQCRICM RKFAQSSDLSRHTKIHTGEKPFQCRICMRN | GCTGCTGCTgCTGCTG (SEQ ID NO: 106) |
| | FSQSSDLSRHIRTHTGEKPFACDICGRKFA HRSTRNRHTKIHLRQKDAARGSGMDAKSLT AW (SEQ ID NO: 90) | |
| HTT_EXP_26 | MAPKKKRKVGIHGVPAAMAERPFQCRICMR NFSRSDTLSEHIRTHTGEKPFACDICGRKF ARRWTLVGHTKIHTGSQKPFQCRICMRNFS RSDTLSEHIRTHTGEKPFACDICGRKFARR WTLVGHTKIHTGSQKPFQCRICMRNFSRSD TLSEHIRTHTGEKPFACDICGRKFARRWTL VGHTKIHLRQKDAARGSGMDAKSLTAW (SEQ ID NO: 91) | CTGCTGCTGCTGCTGCTG (SEQ ID NO: 107) |
| HTT_EXP_27 | MAPKKKRKVGIHGVPAAMAERPFQCRICMR NFSQSSDLSRHIRTHTGEKPFACDICGRKF AHRSTRNRHTKIHTGSQKPFQCRICMRNFS QSSDLSRHIRTHTGEKPFACDICGRKFAHR STRNRHTKIHTPNPHRRTDPSHKPFQCRIC MRNFSRSDTLSEHIRTHTGEKPFACDICGR KFARRWTLVGHTKIHLRQKDAARGSGMDAK SLTAW (SEQ ID NO: 92) | CTGCTGctGCTGCTGCTGCT (SEQ ID NO: 108) |
| HTT_EXP_28 | MAPKKKRKVGIHGVPAAMAERPFQCRICMR NFSRSDTLSEHIRTHTGEKPFACDICGRKF ARRWTLVGHTKIHTHPRAPIPKPFQCRICM RNFSQSSDLSRHIRTHTGEKPFACDICGRK FAHRSTRNRHTKIHTPNPHRRTDPSHKPFQ CRICMRNFSRSDTLSEHIRTHTGEKPFACD ICGRKFARRWTLVGHTKIHLRQKDAARGSG MDAKSLTAW (SEQ ID NO: 93) | CTGCTGctGCTGCTgCTGCTG (SEQ ID NO: 109) |

In any of the above embodiments, the composition comprises two, three or more gRNAs that specifically bind to two, three or more sequences contained in a regulatory region of the polyglutamine disease-related gene.

In some embodiments, the polyglutamine disease-related gene is *HTT.* Exemplary target sequences are provided in Table 6. In some embodiments, the gRNA that specifically binds to a sequence contained in a regulatory region of the polyglutamine disease-related gene specifically binds to any one of the target sequences provided in Table 6. In some embodiments, the composition comprises two, three or more gRNAs (or one or more nucleic acid encoding the gRNAs) that specifically bind to two, three, or more sequences contained in a regulatory region of the polyglutamine disease-related gene, wherein each gRNA specifically binds to a different target sequence provided in Table 6.

**Table 6: Target sequences found in a regulatory region of HTT**

| **ID** | **Target.sequence** | **Genomic.location** | **SEQ ID NO** |
|---|---|---|---|
| HTT_369 | GACATTAGGCAGGCCGGCTG | 3074186 | 115 |
| HTT_288 | GACGGGGACATTAGGCAGGC | 3074192 | 116 |
| HTT_444 | TGGGGACGGGGACATTAGGC | 3074196 | 117 |
| HTT_373 | ATGCTGGGGACGGGGACATT | 3074200 | 118 |
| HTT_534 | GCGGGGCGATGCTGGGGACG | 3074208 | 119 |
| HTT_520 | GGCGGGGCGATGCTGGGGAC | 3074209 | 120 |
| HTT_622 | GGGCGGGGCGATGCTGGGGA | 3074210 | 121 |
| HTT_696 | GGCGGGGCGGGGCGATGCTG | 3074214 | 122 |
| HTT_733 | GGGCGGGGCGGGGCGATGCT | 3074215 | 123 |
| HTT_745 | GGGGCGGGGCGGGGCGATGC | 3074216 | 124 |
| HTT_720 | GGCGAGACGGGGGCGGGGCG | 3074225 | 125 |
| HTT_729 | GGGCGAGACGGGGGCGGGGC | 3074226 | 126 |
| HTT_715 | GGGGCGAGACGGGGGCGGGG | 3074227 | 127 |
| HTT_716 | GGCGGGGCGAGACGGGGGCG | 3074230 | 128 |
| HTT_726 | GGGCGGGGCGAGACGGGGGC | 3074231 | 129 |
| HTT_751 | GGGGCGGGGCGAGACGGGGG | 3074232 | 130 |
| HTT_630 | TGAGGGGCGGGGCGAGACGG | 3074235 | 131 |
| HTT_579 | CTGAGGGGCGGGGCGAGACG | 3074236 | 132 |
| HTT_644 | CCCGTCTCGCCCCGCCCCTC | 3074237 | 133 |
| HTT_619 | GCCTGAGGGGCGGGGCGAGA | 3074238 | 134 |
| HTT_363 | GTCTCGCCCCGCCCCTCAGG | 3074240 | 135 |
| HTT_587 | GGGAGGCCGCCTGAGGGGCG | 3074246 | 136 |
| HTT_551 | AGGGAGGCCGCCTGAGGGGC | 3074247 | 137 |
| HTT_569 | CAGGGAGGCCGCCTGAGGGG | 3074248 | 138 |
| HTT_521 | CAGCAGGGAGGCCGCCTGAG | 3074251 | 139 |
| HTT_351 | ACAGCAGGGAGGCCGCCTGA | 3074252 | 140 |
| HTT_618 | CACAGCAGGGAGGCCGCCTG | 3074253 | 141 |
| HTT_667 | CGGGGCGGGGCACAGCAGGG | 3074263 | 142 |
| HTT_585 | CTCCCTGCTGTGCCCCGCCC | 3074264 | 143 |
| HTT_639 | GGCCGGGGCGGGGCACAGCA | 3074266 | 144 |
| HTT_625 | AGGCCGGGGCGGGGCACAGC | 3074267 | 145 |
| HTT_700 | GGCGTGGCGAGGCCGGGGCG | 3074276 | 146 |
| HTT_714 | GGGCGTGGCGAGGCCGGGGC | 3074277 | 147 |
| HTT_712 | GGGGCGTGGCGAGGCCGGGG | 3074278 | 148 |
| HTT_487 | GTAGGGGCGTGGCGAGGCCG | 3074281 | 149 |
| HTT_596 | GGTAGGGGCGTGGCGAGGCC | 3074282 | 150 |
| HTT_531 | AGGTAGGGGCGTGGCGAGGC | 3074283 | 151 |
| HTT_613 | GGTGAGGTAGGGGCGTGGCG | 3074287 | 152 |
| HTT_561 | GGCGTGGTGAGGTAGGGGCG | 3074292 | 153 |
| HTT_480 | CGGGGGGCGTGGTGAGGTAG | 3074297 | 154 |
| HTT_533 | GCGGGGGGCGTGGTGAGGTA | 3074298 | 155 |
| HTT_583 | TGCGGGGGGCGTGGTGAGGT | 3074299 | 156 |
| HTT_421 | GCGATGCGGGGGGCGTGGTG | 3074303 | 157 |
| HTT_621 | GCGTGGCGATGCGGGGGGCG | 3074308 | 158 |
| HTT_629 | GGGGGGCGTGGCGATGCGGG | 3074313 | 159 |
| HTT_574 | CGGGGGGCGTGGCGATGCGG | 3074314 | 160 |
| HTT_572 | GCGGGGGGCGTGGCGATGCG | 3074315 | 161 |
| HTT_376 | TGCGGGGGGCGTGGCGATGC | 3074316 | 162 |
| HTT_184 | ATGCGGGGGGCGTGGCGATG | 3074317 | 163 |
| HTT_488 | GGGAGGCGTGGCGATGCGGG | 3074330 | 164 |
| HTT_329 | AGGGAGGCGTGGCGATGCGG | 3074331 | 165 |
| HTT_208 | AAGGGAGGCGTGGCGATGCG | 3074332 | 166 |
| HTT_110 | TAAGGGAGGCGTGGCGATGC | 3074333 | 167 |
| HTT_218 | GTAAGGGAGGCGTGGCGATG | 3074334 | 168 |
| HTT_186 | ACTGCATGGTAAGGGAGGCG | 3074342 | 169 |
| HTT_39 | GCGGGACTGCATGGTAAGGG | 3074347 | 170 |
| HTT_99 | GGGGCGGGACTGCATGGTAA | 3074350 | 171 |
| HTT_295 | CGGGGCGGGACTGCATGGTA | 3074351 | 172 |
| HTT_544 | AGGGACGGGGCGGGACTGCA | 3074356 | 173 |
| HTT_680 | GGACGAGGAAGGGACGGGGC | 3074365 | 174 |
| HTT_693 | GGGACGAGGAAGGGACGGGG | 3074366 | 175 |
| HTT_570 | GGCGGGACGAGGAAGGGACG | 3074369 | 176 |
| HTT_564 | AGGCGGGACGAGGAAGGGAC | 3074370 | 177 |
| HTT_689 | GAGGCGGGACGAGGAAGGGA | 3074371 | 178 |
| HTT_215 | CGGCGAGGCGGGACGAGGAA | 3074375 | 179 |
| HTT_452 | GCGGCGAGGCGGGACGAGGA | 3074376 | 180 |
| HTT_107 | TGTCGCGGCGAGGCGGGACG | 3074380 | 181 |
| HTT_66 | GTGAAGTGTCGCGGCGAGGC | 3074386 | 182 |
| HTT_23 | TGTGAAGTGTCGCGGCGAGG | 3074387 | 183 |
| HTT_34 | GTGTGTGAAGTGTCGCGGCG | 3074390 | 184 |
| HTT_361 | AAGCTGTGTGTGAAGTGTCG | 3074395 | 185 |
| HTT_404 | GGTGAGACTGTAATGGGGTG | 3074420 | 186 |
| HTT_50 | GGCGTGGTGAGACTGTAATG | 3074425 | 187 |
| HTT_109 | GGGCGTGGTGAGACTGTAAT | 3074426 | 188 |
| HTT_138 | GGGGCGTGGTGAGACTGTAA | 3074427 | 189 |
| HTT_543 | AACGGAGAGGGGACGGGGCG | 3074441 | 190 |
| HTT_334 | GGCTCAACGGAGAGGGGACG | 3074446 | 191 |
| HTT_425 | GGGCTCAACGGAGAGGGGAC | 3074447 | 192 |
| HTT_466 | GGGGCTCAACGGAGAGGGGA | 3074448 | 193 |
| HTT_198 | GCGCGGGGCTCAACGGAGAG | 3074452 | 194 |
| HTT_225 | GGCGCGGGGCTCAACGGAGA | 3074453 | 195 |
| HTT_60 | AGGCGCGGGGCTCAACGGAG | 3074454 | 196 |
| HTT_106 | GGCGAAGGCGCGGGGCTCAA | 3074459 | 197 |
| HTT_88 | TTGAGCCCCGCGCCTTCGCC | 3074462 | 198 |
| HTT_270 | TGAGCCCCGCGCCTTCGCCC | 3074463 | 199 |
| HTT_354 | GCCCCGCGCCTTCGCCCGGG | 3074466 | 200 |
| HTT_249 | CCCCGCGCCTTCGCCCGGGT | 3074467 | 201 |
| HTT_156 | CCCGCGCCTTCGCCCGGGTG | 3074468 | 202 |
| HTT_211 | GCCCCACCCGGGCGAAGGCG | 3074469 | 203 |
| HTT_111 | GCAGCGCCCCACCCGGGCGA | 3074474 | 204 |
| HTT_707 | GACAGCGCAGCGCCCCACCC | 3074480 | 205 |
| HTT_182 | TGACAGCGCAGCGCCCCACC | 3074481 | 206 |
| HTT_589 | GTGGGGCGCTGCGCTGTCAG | 3074485 | 207 |
| HTT_422 | GTCAGCGGCCTTGCTGTGTG | 3074500 | 208 |
| HTT_436 | AGGTTCTGCCTCACACAGCA | 3074508 | 209 |
| HTT_442 | GCTGTGTGAGGCAGAACCTG | 3074512 | 210 |
| HTT_494 | CTGTGTGAGGCAGAACCTGC | 3074513 | 211 |
| HTT_348 | TGTGTGAGGCAGAACCTGCG | 3074514 | 212 |
| HTT_192 | GTGTGAGGCAGAACCTGCGG | 3074515 | 213 |
| HTT_448 | GAGGCAGAACCTGCGGGGGC | 3074519 | 214 |
| HTT_431 | AGGCAGAACCTGCGGGGGCA | 3074520 | 215 |
| HTT_504 | GGCAGAACCTGCGGGGGCAG | 3074521 | 216 |
| HTT_611 | AGAACCTGCGGGGGCAGGGG | 3074524 | 217 |
| HTT_615 | GAACCTGCGGGGGCAGGGGC | 3074525 | 218 |
| HTT_636 | CAGCCCGCCCCTGCCCCCGC | 3074528 | 219 |
| HTT_701 | CTGCGGGGGCAGGGGCGGGC | 3074529 | 220 |
| HTT_506 | GCAGGGGCGGGCTGGTTCCC | 3074537 | 221 |
| HTT_385 | CTGGTTCCCTGGCCAGCCAT | 3074548 | 222 |
| HTT_462 | ACTCTGCCAATGGCTGGCCA | 3074554 | 223 |
| HTT_381 | GACTCTGCCAATGGCTGGCC | 3074555 | 224 |
| HTT_128 | CTGCGGACTCTGCCAATGGC | 3074560 | 225 |
| HTT_269 | CAGCCATTGGCAGAGTCCGC | 3074561 | 226 |
| HTT_126 | TAGCCTGCGGACTCTGCCAA | 3074564 | 227 |
| HTT_153 | ATTGGCAGAGTCCGCAGGCT | 3074566 | 228 |
| HTT_267 | TTGGCAGAGTCCGCAGGCTA | 3074567 | 229 |
| HTT_299 | TGATTGACAGCCCTAGCCTG | 3074577 | 230 |
| HTT_82 | GCTAGGGCTGTCAATCATGC | 3074583 | 231 |
| HTT_185 | GGGCTGTCAATCATGCTGGC | 3074587 | 232 |
| HTT_17 | GTCAATCATGCTGGCCGGCG | 3074592 | 233 |
| HTT_600 | CGGCGGAGGCGGGGCCACGC | 3074606 | 234 |
| HTT_311 | CGGCGTGGCCCCGCCTCCGC | 3074607 | 235 |
| HTT_407 | TGGCCCCGCCTCCGCCGGCG | 3074612 | 236 |
| HTT_446 | ACGCTGCGCCGGCGGAGGCG | 3074635 | 237 |
| HTT_409 | GACGCTGCGCCGGCGGAGGC | 3074636 | 238 |
| HTT_321 | AGACGCTGCGCCGGCGGAGG | 3074637 | 239 |
| HTT_252 | GCCTCCGCCGGCGCAGCGTC | 3074639 | 240 |
| HTT_247 | CCTCCGCCGGCGCAGCGTCT | 3074640 | 241 |
| HTT_137 | CGTCCCAGACGCTGCGCCGG | 3074643 | 242 |
| HTT_44 | TTGCGTCCCAGACGCTGCGC | 3074646 | 243 |
| HTT_272 | GGCGCAGCGTCTGGGACGCA | 3074648 | 244 |
| HTT_179 | GTCTGGGACGCAAGGCGCCG | 3074656 | 245 |
| HTT_74 | TCTGGGACGCAAGGCGCCGT | 3074657 | 246 |
| HTT_79 | CTGGGACGCAAGGCGCCGTG | 3074658 | 247 |
| HTT_96 | TGGGACGCAAGGCGCCGTGG | 3074659 | 248 |
| HTT_518 | CAAGGCGCCGTGGGGGCTGC | 3074666 | 249 |
| HTT_509 | AAGGCGCCGTGGGGGCTGCC | 3074667 | 250 |
| HTT_586 | CGCCGTGGGGGCTGCCGGGA | 3074671 | 251 |
| HTT_547 | GCCGTGGGGGCTGCCGGGAC | 3074672 | 252 |
| HTT_465 | ACCCGTCCCGGCAGCCCCCA | 3074673 | 253 |
| HTT_41 | CTGCCGGGACGGGTCCAAGA | 3074682 | 254 |
| HTT_40 | CGTCCATCTTGGACCCGTCC | 3074685 | 255 |
| HTT_78 | CGGGACGGGTCCAAGATGGA | 3074686 | 256 |
| HTT_19 | TCCAAGATGGACGGCCGCTC | 3074695 | 257 |
| HTT_46 | ACCTGAGCGGCCGTCCATCT | 3074696 | 258 |
| HTT_428 | AGGTAAAAGCAGAACCTGAG | 3074709 | 259 |
| HTT_308 | TGAATGGGGCTCTGGGCCGC | 3074729 | 260 |
| HTT_279 | GGGGCAATGAATGGGGCTCT | 3074736 | 261 |
| HTT_370 | CGGGGCAATGAATGGGGCTC | 3074737 | 262 |
| HTT_503 | CAGAGCCCCATTCATTGCCC | 3074738 | 263 |
| HTT_232 | CAGCACCGGGGCAATGAATG | 3074743 | 264 |
| HTT_141 | TCAGCACCGGGGCAATGAAT | 3074744 | 265 |
| HTT_248 | CTCAGCACCGGGGCAATGAA | 3074745 | 266 |
| HTT_76 | TTCATTGCCCCGGTGCTGAG | 3074748 | 267 |
| HTT_233 | CGCGGCGCCGCTCAGCACCG | 3074755 | 268 |
| HTT_114 | TCGCGGCGCCGCTCAGCACC | 3074756 | 269 |
| HTT_158 | CTCGCGGCGCCGCTCAGCAC | 3074757 | 270 |
| HTT_90 | TGCTGAGCGGCGCCGCGAGT | 3074761 | 271 |
| HTT_42 | CGGCGCCGCGAGTCGGCCCG | 3074768 | 272 |
| HTT_199 | GGAGGCCTCGGGCCGACTCG | 3074773 | 273 |
| HTT_134 | GCGAGTCGGCCCGAGGCCTC | 3074775 | 274 |
| HTT_229 | CGAGTCGGCCCGAGGCCTCC | 3074776 | 275 |
| HTT_239 | GAGTCGGCCCGAGGCCTCCG | 3074777 | 276 |
| HTT_310 | CGGCAGTCCCCGGAGGCCTC | 3074784 | 277 |
| HTT_313 | ACGGCAGTCCCCGGAGGCCT | 3074785 | 278 |
| HTT_230 | GCCTCCGGGGACTGCCGTGC | 3074790 | 279 |
| HTT_227 | CCTCCGGGGACTGCCGTGCC | 3074791 | 280 |
| HTT_150 | CCGGGGACTGCCGTGCCGGG | 3074794 | 281 |
| HTT_394 | CGGGGACTGCCGTGCCGGGC | 3074795 | 282 |
| HTT_61 | TGGCGGTCTCCCGCCCGGCA | 3074804 | 283 |
| HTT_73 | TGCCGGGCGGGAGACCGCCA | 3074807 | 284 |
| HTT_85 | CGCCATGGCGGTCTCCCGCC | 3074809 | 285 |
| HTT_165 | GGAGACCGCCATGGCGACCC | 3074816 | 286 |
| HTT_420 | GACCCTGGAAAAGCTGATGA | 3074831 | 287 |
| HTT_286 | GAAGGACTTGAGGGACTCGA | 3074854 | 288 |
| HTT_511 | CTGCTGCTGGAAGGACTTGA | 3074863 | 289 |
| HTT_552 | GCTGCTGCTGGAAGGACTTG | 3074864 | 290 |
| HTT_728 | CTGCTGCTGCTGCTGCTGGA | 3074872 | 291 |
| HTT_1 | GCTGCTGCTGCTGCTGCTGC | 3074876 | 292 |
| HTT_770 | TGAGGAGGCGGCGGCGGCGG | 3074952 | 293 |
| HTT_740 | AGCTGAGGAGGCGGCGGCGG | 3074955 | 294 |
| HTT_711 | GGAAGCTGAGGAGGCGGCGG | 3074958 | 295 |
| HTT_731 | TGAGGAAGCTGAGGAGGCGG | 3074961 | 296 |
| HTT_724 | GGCTGAGGAAGCTGAGGAGG | 3074964 | 297 |
| HTT_722 | GGCGGCTGAGGAAGCTGAGG | 3074967 | 298 |
| HTT_669 | GGCGGCGGCTGAGGAAGCTG | 3074970 | 299 |
| HTT_556 | GCTTCCTCAGCCGCCGCCGC | 3074975 | 300 |
| HTT_657 | TGTGCCTGCGGCGGCGGCTG | 3074979 | 301 |
| HTT_559 | AGCGGCTGTGCCTGCGGCGG | 3074985 | 302 |
| HTT_550 | AGCAGCGGCTGTGCCTGCGG | 3074988 | 303 |
| HTT_638 | GGCAGCAGCGGCTGTGCCTG | 3074991 | 304 |
| HTT_687 | GGCTGCGGCTGAGGCAGCAG | 3075003 | 305 |
| HTT_756 | GGCGGGGGCGGCTGCGGCTG | 3075012 | 306 |
| HTT_607 | AGCCGGGCCGGGTGGCGGCG | 3075040 | 307 |
| HTT_623 | CAGCCGGGCCGGGTGGCGGC | 3075041 | 308 |
| HTT_571 | ACAGCCGGGCCGGGTGGCGG | 3075042 | 309 |
| HTT_594 | GCCGCCACCCGGCCCGGCTG | 3075044 | 310 |
| HTT_576 | GCCACAGCCGGGCCGGGTGG | 3075045 | 311 |
| HTT_527 | TCAGCCACAGCCGGGCCGGG | 3075048 | 312 |
| HTT_471 | ACCCGGCCCGGCTGTGGCTG | 3075050 | 313 |
| HTT_542 | TCCTCAGCCACAGCCGGGCC | 3075051 | 314 |
| HTT_558 | CTCCTCAGCCACAGCCGGGC | 3075052 | 315 |
| HTT_489 | GCGGCTCCTCAGCCACAGCC | 3075056 | 316 |
| HTT_374 | AGCGGCTCCTCAGCCACAGC | 3075057 | 317 |
| HTT_37 | CAAACTCACGGTCGGTGCAG | 3075075 | 318 |
| HTT_20 | CGCTGCACCGACCGTGAGTT | 3075076 | 319 |
| HTT_45 | GCTGCACCGACCGTGAGTTT | 3075077 | 320 |
| HTT_22 | AGCGGGCCCAAACTCACGGT | 3075083 | 321 |
| HTT_98 | CTGCAGCGGGCCCAAACTCA | 3075087 | 322 |
| HTT_515 | CCCGCTGCAGCTCCCTGTCC | 3075100 | 323 |
| HTT_537 | GCCGGGACAGGGAGCTGCAG | 3075101 | 324 |
| HTT_565 | GCTGCAGCTCCCTGTCCCGG | 3075103 | 325 |
| HTT_479 | CTGCAGCTCCCTGTCCCGGC | 3075104 | 326 |
| HTT_365 | TCCCTGTCCCGGCGGGTCCC | 3075111 | 327 |
| HTT_344 | GCCTGGGACCCGCCGGGACA | 3075112 | 328 |
| HTT_260 | AGCCTGGGACCCGCCGGGAC | 3075113 | 329 |
| HTT_206 | TCCCGGCGGGTCCCAGGCTA | 3075117 | 330 |
| HTT_124 | GCCGTAGCCTGGGACCCGCC | 3075118 | 331 |
| HTT_190 | CGCCGTAGCCTGGGACCCGC | 3075119 | 332 |
| HTT_287 | CGGCGGGTCCCAGGCTACGG | 3075120 | 333 |
| HTT_294 | GGCGGGTCCCAGGCTACGGC | 3075121 | 334 |
| HTT_169 | GCGGGTCCCAGGCTACGGCG | 3075122 | 335 |
| HTT_347 | GTCCCAGGCTACGGCGGGGA | 3075126 | 336 |
| HTT_67 | CGCCATCCCCGCCGTAGCCT | 3075128 | 337 |
| HTT_352 | CCAGGCTACGGCGGGGATGG | 3075129 | 338 |
| HTT_368 | GGCGGTAACCCTGCAGCCTG | 3075147 | 339 |
| HTT_245 | GCGGTAACCCTGCAGCCTGC | 3075148 | 340 |
| HTT_293 | TAACCCTGCAGCCTGCGGGC | 3075152 | 341 |
| HTT_325 | TCGCCGGCCCGCAGGCTGCA | 3075155 | 342 |
| HTT_356 | GTCGCCGGCCCGCAGGCTGC | 3075156 | 343 |
| HTT_27 | GGTTCGTGTCGCCGGCCCGC | 3075163 | 344 |
| HTT_86 | GGGCCGGCGACACGAACCCC | 3075168 | 345 |
| HTT_130 | GGGCCGGGGGTTCGTGTCGC | 3075171 | 346 |
| HTT_483 | CTCTGTCTCTGCGGGGCCGG | 3075184 | 347 |
| HTT_219 | ACTCTGTCTCTGCGGGGCCG | 3075185 | 348 |
| HTT_281 | CACTCTGTCTCTGCGGGGCC | 3075186 | 349 |
| HTT_397 | TCACTCTGTCTCTGCGGGGC | 3075187 | 350 |
| HTT_231 | TGGGTCACTCTGTCTCTGCG | 3075191 | 351 |
| HTT_445 | CTGGGTCACTCTGTCTCTGC | 3075192 | 352 |
| HTT_655 | GCTGGGTCACTCTGTCTCTG | 3075193 | 353 |
| HTT_562 | CTCATGGGCTCTGGGTTGCT | 3075210 | 354 |
| HTT_437 | CCAGCAACCCAGAGCCCATG | 3075211 | 355 |
| HTT_440 | CAGCAACCCAGAGCCCATGA | 3075212 | 356 |
| HTT_416 | GGGTGTCCCTCATGGGCTCT | 3075218 | 357 |
| HTT_117 | CGGGTGTCCCTCATGGGCTC | 3075219 | 358 |
| HTT_290 | AGGGGGCGGGTGTCCCTCAT | 3075225 | 359 |
| HTT_375 | GAGGGGGCGGGTGTCCCTCA | 3075226 | 360 |
| HTT_408 | GAGGGACACCCGCCCCCTCC | 3075230 | 361 |
| HTT_353 | AGGGACACCCGCCCCCTCCT | 3075231 | 362 |
| HTT_350 | GGGACACCCGCCCCCTCCTG | 3075232 | 363 |
| HTT_367 | ACCCGCCCCCTCCTGGGGCG | 3075237 | 364 |
| HTT_522 | GCCTCGCCCCAGGAGGGGGC | 3075238 | 365 |
| HTT_628 | GGCCTCGCCCCAGGAGGGGG | 3075239 | 366 |
| HTT_417 | GAAGGCCTCGCCCCAGGAGG | 3075242 | 367 |
| HTT_555 | GGAAGGCCTCGCCCCAGGAG | 3075243 | 368 |
| HTT_463 | GGGAAGGCCTCGCCCCAGGA | 3075244 | 369 |
| HTT_484 | GGGGAAGGCCTCGCCCCAGG | 3075245 | 370 |
| HTT_541 | GTGGGGGAAGGCCTCGCCCC | 3075248 | 371 |
| HTT_603 | AGCGGGGCTGAAGTGGGGGA | 3075260 | 372 |
| HTT_582 | AGGGAGCGGGGCTGAAGTGG | 3075264 | 373 |
| HTT_595 | GAGGGAGCGGGGCTGAAGTG | 3075265 | 374 |
| HTT_474 | TGAGGGAGCGGGGCTGAAGT | 3075266 | 375 |
| HTT_560 | GTGAGGGAGCGGGGCTGAAG | 3075267 | 376 |
| HTT_426 | TTCAGCCCCGCTCCCTCACT | 3075271 | 377 |
| HTT_539 | TCAGCCCCGCTCCCTCACTT | 3075272 | 378 |
| HTT_277 | AAGACCCAAGTGAGGGAGCG | 3075276 | 379 |
| HTT_102 | TCTTCCCTTGTCCTCTCGCG | 3075295 | 380 |
| HTT_200 | CTTCCCTTGTCCTCTCGCGA | 3075296 | 381 |
| HTT_120 | TTCCCTTGTCCTCTCGCGAG | 3075297 | 382 |
| HTT_38 | CTCCCCTCGCGAGAGGACAA | 3075299 | 383 |
| HTT_58 | CCTTGTCCTCTCGCGAGGGG | 3075300 | 384 |
| HTT_306 | GCTCTGCCTCCCCTCGCGAG | 3075306 | 385 |
| HTT_573 | GAGGGGAGGCAGAGCCTTGT | 3075314 | 386 |
| HTT_493 | AGGGGAGGCAGAGCCTTGTT | 3075315 | 387 |
| HTT_470 | GGGGAGGCAGAGCCTTGTTG | 3075316 | 388 |
| HTT_194 | ATTCAGGACAGGCCCCAACA | 3075328 | 389 |
| HTT_212 | GGCCTGTCCTGAATTCACCG | 3075337 | 390 |
| HTT_160 | GCCTGTCCTGAATTCACCGA | 3075338 | 391 |
| HTT_94 | CCTGTCCTGAATTCACCGAG | 3075339 | 392 |
| HTT_113 | TGACTCCCCTCGGTGAATTC | 3075344 | 393 |
| HTT_51 | GAATTCACCGAGGGGAGTCA | 3075347 | 394 |
| HTT_331 | GCTGAGGCCGTGACTCCCCT | 3075354 | 395 |
| HTT_461 | TGCGAAGGGCGAGAGGGCTG | 3075370 | 396 |
| HTT_207 | TCAGCCCTCTCGCCCTTCGC | 3075372 | 397 |
| HTT_115 | GCATCCTGCGAAGGGCGAGA | 3075376 | 398 |
| HTT_63 | CGCATCCTGCGAAGGGCGAG | 3075377 | 399 |
| HTT_59 | AACTCTTCGCATCCTGCGAA | 3075384 | 400 |
| HTT_29 | CAACTCTTCGCATCCTGCGA | 3075385 | 401 |
| HTT_43 | CTTCGCAGGATGCGAAGAGT | 3075386 | 402 |
| HTT_132 | TTCGCAGGATGCGAAGAGTT | 3075387 | 403 |
| HTT_145 | TCGCAGGATGCGAAGAGTTG | 3075388 | 404 |
| HTT_276 | TTATTTGCGAGAAACCAGGG | 3075428 | 405 |
| HTT_265 | TATTTGCGAGAAACCAGGGC | 3075429 | 406 |
| HTT_81 | ATTTGCGAGAAACCAGGGCG | 3075430 | 407 |
| HTT_103 | TTTGCGAGAAACCAGGGCGG | 3075431 | 408 |
| HTT_151 | AGTTAAAAGAACCCCCGCCC | 3075442 | 409 |
| HTT_149 | ACTGCGTTGTGAAGAGAACT | 3075462 | 410 |
| HTT_53 | GCGTTGTGAAGAGAACTTGG | 3075465 | 411 |
| HTT_349 | AAGTGGCACTGAGCAAATCT | 3075490 | 412 |
| HTT_473 | CTCAGACTAGAAGAGGGAAG | 3075507 | 413 |
| HTT_284 | CTTCCCTCTTCTAGTCTGAG | 3075510 | 414 |
| HTT_430 | TTCCCTCTTCTAGTCTGAGA | 3075511 | 415 |
| HTT_322 | TTCCCTCTCAGACTAGAAGA | 3075513 | 416 |
| HTT_324 | CTTCCCTCTCAGACTAGAAG | 3075514 | 417 |
| HTT_383 | CTTCTAGTCTGAGAGGGAAG | 3075517 | 418 |
| HTT_645 | TTCTAGTCTGAGAGGGAAGA | 3075518 | 419 |
| HTT_647 | AGTCTGAGAGGGAAGAGGGC | 3075522 | 420 |
| HTT_593 | GTCTGAGAGGGAAGAGGGCT | 3075523 | 421 |
| HTT_654 | TCTGAGAGGGAAGAGGGCTG | 3075524 | 422 |
| HTT_670 | CTGAGAGGGAAGAGGGCTGG | 3075525 | 423 |
| HTT_710 | AGGGAAGAGGGCTGGGGGCG | 3075530 | 424 |
| HTT_692 | GGGAAGAGGGCTGGGGGCGC | 3075531 | 425 |
| HTT_21 | GGGGCGCGGGACACTTCGAG | 3075544 | 426 |
| HTT_31 | GCGCGGGACACTTCGAGAGG | 3075547 | 427 |
| HTT_205 | CGGGACACTTCGAGAGGAGG | 3075550 | 428 |
| HTT_312 | GGGACACTTCGAGAGGAGGC | 3075551 | 429 |
| HTT_159 | GGACACTTCGAGAGGAGGCG | 3075552 | 430 |
| HTT_183 | CTTCGAGAGGAGGCGGGGTT | 3075557 | 431 |
| HTT_605 | GAGGAGGCGGGGTTTGGAGC | 3075563 | 432 |
| HTT_632 | GGTTTGGAGCTGGAGAGATG | 3075573 | 433 |
| HTT_546 | GTTTGGAGCTGGAGAGATGT | 3075574 | 434 |
| HTT_554 | TTTGGAGCTGGAGAGATGTG | 3075575 | 435 |
| HTT_601 | TTGGAGCTGGAGAGATGTGG | 3075576 | 436 |
| HTT_652 | CTGGAGAGATGTGGGGGCAG | 3075582 | 437 |
| HTT_68 | TTAGGACGCCTCGGCGGGAG | 3075619 | 438 |
| HTT_413 | GGACGCCTCGGCGGGAGTGG | 3075622 | 439 |
| HTT_237 | GACGCCTCGGCGGGAGTGGC | 3075623 | 440 |
| HTT_123 | ACGCCTCGGCGGGAGTGGCG | 3075624 | 441 |
| HTT_513 | CTGCCCCGCCACTCCCGCCG | 3075627 | 442 |
| HTT_545 | CTCGGCGGGAGTGGCGGGGC | 3075628 | 443 |
| HTT_475 | TCGGCGGGAGTGGCGGGGCA | 3075629 | 444 |
| HTT_666 | CGGCGGGAGTGGCGGGGCAG | 3075630 | 445 |
| HTT_697 | GGCGGGAGTGGCGGGGCAGG | 3075631 | 446 |
| HTT_706 | GCGGGAGTGGCGGGGCAGGG | 3075632 | 447 |
| HTT_705 | CGGGAGTGGCGGGGCAGGGG | 3075633 | 448 |
| HTT_754 | GGGAGTGGCGGGGCAGGGGG | 3075634 | 449 |
| HTT_735 | GGAGTGGCGGGGCAGGGGGG | 3075635 | 450 |
| HTT_762 | GTGGCGGGGCAGGGGGGGGG | 3075638 | 451 |
| HTT_744 | TGGCGGGGCAGGGGGGGGGC | 3075639 | 452 |
| HTT_721 | CAGGGGGGGGGCGGGGAGTG | 3075647 | 453 |
| HTT_746 | AGGGGGGGGGCGGGGAGTGA | 3075648 | 454 |
| HTT_89 | GGGAGTGAGGGCGCGTCCAA | 3075660 | 455 |
| HTT_28 | GGAGTGAGGGCGCGTCCAAT | 3075661 | 456 |
| HTT_435 | TAGGAAAAGAAATCTCCCAT | 3075676 | 457 |
| HTT_449 | TTAAAACAGCCTGAGATTTG | 3075706 | 458 |
| HTT_255 | TAGGAAGAGCCTCAAATCTC | 3075715 | 459 |
| HTT_309 | GAGGCTCTTCCTACATTGTC | 3075725 | 460 |
| HTT_323 | ATGAAATGTCCTGACAATGT | 3075734 | 461 |
| HTT_526 | TTTCATTTAGTTCATGATCA | 3075751 | 462 |
| HTT_168 | CATTTAGTTCATGATCACGG | 3075754 | 463 |
| HTT_220 | AGATGAGCAGATCTCTTAGG | 3075797 | 464 |
| HTT_464 | CTTAGATGAGCAGATCTCTT | 3075800 | 465 |
| HTT_64 | CTGCTCATCTAAGCCTAAGT | 3075810 | 466 |
| HTT_25 | CTAAGCCTAAGTTGGTCTGC | 3075818 | 467 |
| HTT_65 | AAACGCCTGCAGACCAACTT | 3075823 | 468 |
| HTT_171 | GCAGGCGTTTGAATGAGTTG | 3075836 | 469 |
| HTT_371 | GTTGTGGTTGCCAAGTAAAG | 3075852 | 470 |
| HTT_32 | CGTAAGTTCACCACTTTACT | 3075862 | 471 |
| HTT_48 | AGTAAAGTGGTGAACTTACG | 3075865 | 472 |
| HTT_663 | ATGAAATTATCTTAAATATT | 3075894 | 473 |
| HTT_125 | TAAAACCAACACGTTGCTGA | 3075956 | 474 |
| HTT_101 | AAAACCAACACGTTGCTGAT | 3075957 | 475 |
| HTT_55 | AAACCAACACGTTGCTGATG | 3075958 | 476 |
| HTT_49 | CCAACACGTTGCTGATGGGG | 3075961 | 477 |
| HTT_77 | GATGGGGAGGTTAATTGCCG | 3075974 | 478 |
| HTT_36 | ATGGGGAGGTTAATTGCCGA | 3075975 | 479 |
| HTT_93 | TAATTGCCGAGGGATGAATG | 3075985 | 480 |
| HTT_343 | TGTACACCTCATTCATCCCT | 3075991 | 481 |
| HTT_180 | GGCAAGCCTGACTGGAATAC | 3076019 | 482 |
| HTT_129 | CGTATTCTGGCAAGCCTGAC | 3076027 | 483 |
| HTT_155 | CAGTCAGGCTTGCCAGAATA | 3076028 | 484 |
| HTT_204 | AGTCAGGCTTGCCAGAATAC | 3076029 | 485 |
| HTT_24 | GTCAGGCTTGCCAGAATACG | 3076030 | 486 |
| HTT_75 | TCAGGCTTGCCAGAATACGG | 3076031 | 487 |
| HTT_47 | CAGGCTTGCCAGAATACGGG | 3076032 | 488 |
| HTT_33 | TCTGCGGACCCCCCGTATTC | 3076040 | 489 |
| HTT_80 | CGGGGGGTCCGCAGACTCCG | 3076048 | 490 |
| HTT_54 | GGGGGGTCCGCAGACTCCGT | 3076049 | 491 |
| HTT_485 | TGAGATGCCCACGGAGTCTG | 3076056 | 492 |
| HTT_403 | TGGCACATCTGAGATGCCCA | 3076065 | 493 |
| HTT_268 | ATCTCAGATGTGCCAGTGAA | 3076073 | 494 |
| HTT_256 | TCTCAGATGTGCCAGTGAAA | 3076074 | 495 |
| HTT_266 | GCAAACAGAAACCCTTTCAC | 3076085 | 496 |
| HTT_434 | GGGTTTCTGTTGCTTGTTCT | 3076144 | 497 |
| HTT_581 | GGTTTCTGTTGCTTGTTCTT | 3076145 | 498 |
| HTT_641 | GTTTCTGTTGCTTGTTCTTG | 3076146 | 499 |
| HTT_263 | GAGACCATTAAAACATCTAG | 3076192 | 500 |
| HTT_224 | AAAACATCTAGCGGAACCCC | 3076201 | 501 |
| HTT_217 | CGGAACCCCAGGACTTTCCC | 3076212 | 502 |
| HTT_455 | GACTTCCAGGGAAAGTCCTG | 3076217 | 503 |
| HTT_379 | AGACTTCCAGGGAAAGTCCT | 3076218 | 504 |
| HTT_502 | CAGACTTCCAGGGAAAGTCC | 3076219 | 505 |
| HTT_193 | ACTCGACACACAGACTTCCA | 3076229 | 506 |
| HTT_273 | CACTCGACACACAGACTTCC | 3076230 | 507 |
| HTT_148 | CTGTGTGTCGAGTGTACAGT | 3076239 | 508 |
| HTT_84 | TCGAGTGTACAGTAGGAGTT | 3076246 | 509 |
| HTT_240 | GTAGGAGTTAGGAAGTACTC | 3076257 | 510 |
| HTT_140 | GAAGTACTCTGGTGCAGTTC | 3076268 | 511 |
| HTT_512 | AATACTGAGAGGTAAGAGAA | 3076291 | 512 |
| HTT_167 | ATCGGAAATAGAATACTGAG | 3076302 | 513 |
| HTT_69 | TCAGTATTCTATTTCCGATC | 3076306 | 514 |
| HTT_144 | CATCTGGGACACATCCAGAT | 3076320 | 515 |
| HTT_56 | CGATCTGGATGTGTCCCAGA | 3076321 | 516 |
| HTT_187 | GATGTGTCCCAGATGGCATT | 3076328 | 517 |
| HTT_364 | ATTCTTACCAAATGCCATCT | 3076335 | 518 |
| HTT_228 | TATTCTTACCAAATGCCATC | 3076336 | 519 |
| HTT_610 | AGTTTAATTAGAAAATGATT | 3076435 | 520 |
| HTT_320 | AGCAGTGTTAGCTTATTTGT | 3076460 | 521 |
| HTT_649 | TATTTGTTGGAATAAAATTT | 3076473 | 522 |
| HTT_557 | TTAGGAATAAATTATTCTAA | 3076491 | 523 |
| HTT_566 | GAATAAATTATTCTAAAGGA | 3076495 | 524 |
| HTT_427 | AAAGCACTACAGTAAATTTC | 3076588 | 525 |
| HTT_131 | ATCTTGTGTAAACACTAAAC | 3076651 | 526 |
| HTT_441 | TCTTGTGTAAACACTAAACA | 3076652 | 527 |
| HTT_264 | ACACTAAACAGGGCTCCTGA | 3076662 | 528 |
| HTT_214 | CACTAAACAGGGCTCCTGAT | 3076663 | 529 |
| HTT_482 | AAACAGGGCTCCTGATGGGA | 3076667 | 530 |
| HTT_390 | TCTGAAGCAGCCCAGCAGAA | 3076694 | 531 |
| HTT_656 | AAATCAGCATAAGAAAGAGA | 3076740 | 532 |
| HTT_709 | ATTTGCTTATCTTCAAAATA | 3076775 | 533 |
| HTT_661 | TTTGCTTATCTTCAAAATAT | 3076776 | 534 |
| HTT_459 | TTCCTTTGCTGCCTTGACAA | 3076810 | 535 |
| HTT_451 | CTCCTTTGTCAAGGCAGCAA | 3076812 | 536 |
| HTT_469 | AAAATCTATCTCCTTTGTCA | 3076821 | 537 |
| HTT_682 | CTTATTTAAAAAATTTAATC | 3076875 | 538 |
| HTT_686 | TTAAAAAATTTAATCAGGAC | 3076880 | 539 |
| HTT_500 | AAATTTAATCAGGACTGGCA | 3076885 | 540 |
| HTT_283 | TTTAATCAGGACTGGCAAGG | 3076888 | 541 |
| HTT_767 | CACCTTTAATCCGAGCACTT | 3076915 | 542 |
| HTT_778 | ACCTTTAATCCGAGCACTTT | 3076916 | 543 |
| HTT_769 | TCCCAAAGTGCTCGGATTAA | 3076917 | 544 |
| HTT_780 | TTTAATCCGAGCACTTTGGG | 3076919 | 545 |
| HTT_787 | CCGAGCACTTTGGGAGGCCT | 3076925 | 546 |
| HTT_817 | AGCACTTTGGGAGGCCTAGG | 3076928 | 547 |
| HTT_796 | CTCAGGTGATTCGTCCACCT | 3076942 | 548 |
| HTT_750 | CTAGGTGGACGAATCACCTG | 3076943 | 549 |
| HTT_779 | TGGACGAATCACCTGAGGTC | 3076948 | 550 |
| HTT_825 | GGTCTCAAACTCCTGACCTC | 3076959 | 551 |
| HTT_822 | TCAGGAGTTTGAGACCAGCC | 3076966 | 552 |
| HTT_799 | TGAGACCAGCCTGGCTAACA | 3076975 | 553 |
| HTT_783 | TTTCACCATGTTAGCCAGGC | 3076980 | 554 |
| HTT_792 | AGGGTTTCACCATGTTAGCC | 3076984 | 555 |
| HTT_801 | ATACAAAAATTAGCTGGTCA | 3077020 | 556 |
| HTT_797 | CAAAAATTAGCTGGTCATGG | 3077023 | 557 |
| HTT_813 | TGCCTGTAATCCAAGCTACC | 3077050 | 558 |
| HTT_800 | GCCTGTAATCCAAGCTACCT | 3077051 | 559 |
| HTT_764 | TCCCAGGTAGCTTGGATTAC | 3077052 | 560 |
| HTT_808 | TGTAATCCAAGCTACCTGGG | 3077054 | 561 |
| HTT_786 | CCAAGCTACCTGGGAGGCTG | 3077060 | 562 |
| HTT_805 | GCTACCTGGGAGGCTGAGGC | 3077064 | 563 |
| HTT_816 | GCAGGAAAATCGCTTGAACC | 3077082 | 564 |
| HTT_807 | CAGGAAAATCGCTTGAACCC | 3077083 | 565 |
| HTT_776 | GAAAATCGCTTGAACCCGGG | 3077086 | 566 |
| HTT_815 | CACTGCAGACTCTGCCTCCC | 3077100 | 567 |
| HTT_812 | TCACTGCAGACTCTGCCTCC | 3077101 | 568 |
| HTT_819 | CACGCCACTGCACTCCAGCC | 3077132 | 569 |
| HTT_821 | ACGCCACTGCACTCCAGCCT | 3077133 | 570 |
| HTT_829 | TCACCCAGGCTGGAGTGCAG | 3077136 | 571 |
| HTT_820 | TCTCGCTCTGTCACCCAGGC | 3077146 | 572 |
| HTT_784 | AGAGTCTCGCTCTGTCACCC | 3077150 | 573 |
| HTT_535 | ACAAATTCTGTAATTACAAA | 3077232 | 574 |
| HTT_608 | CAAATTCTGTAATTACAAAA | 3077233 | 575 |
| HTT_694 | ACTCAGAGGAAAGGGGTGGA | 3077274 | 576 |
| HTT_640 | AGGTACTCAGAGGAAAGGGG | 3077278 | 577 |
| HTT_282 | GTTAGGTACTCAGAGGAAAG | 3077281 | 578 |
| HTT_414 | AGTTAGGTACTCAGAGGAAA | 3077282 | 579 |
| HTT_399 | AAGTTAGGTACTCAGAGGAA | 3077283 | 580 |
| HTT_118 | GGACAAAGTTAGGTACTCAG | 3077288 | 581 |
| HTT_432 | TTGTTCTTGGGGACAAAGTT | 3077298 | 582 |
| HTT_478 | TGAAATAGTGCTTGTTCTTG | 3077309 | 583 |
| HTT_388 | CTGAAATAGTGCTTGTTCTT | 3077310 | 584 |
| HTT_318 | ACTGAAATAGTGCTTGTTCT | 3077311 | 585 |
| HTT_143 | TATATCTGGCAGGATACATG | 3077335 | 586 |
| HTT_197 | ATGAACAGGTTATATCTGGC | 3077345 | 587 |
| HTT_271 | CAATATGAACAGGTTATATC | 3077349 | 588 |
| HTT_181 | ATCTATCTTACAATATGAAC | 3077359 | 589 |
| HTT_759 | GTAGTCTCACATTGTCACCC | 3077443 | 590 |
| HTT_795 | TCTCACATTGTCACCCAGGC | 3077447 | 591 |
| HTT_794 | GTATCACTGCACTCCAGCCT | 3077460 | 592 |
| HTT_771 | TGTATCACTGCACTCCAGCC | 3077461 | 593 |
| HTT_810 | GGAGTGCAGTGATACAATCT | 3077468 | 594 |
| HTT_788 | CACTGCAGTCTCTGCCTCCC | 3077493 | 595 |

In some embodiments, the gRNA that specifically binds to a sequence contained in a regulatory region of the polyglutamine disease-related gene specifically binds to any one of the target sequences provided in Table 7. In some embodiments, the composition comprises two, three or more gRNAs (or one or more nucleic acid encoding the gRNAs) that specifically bind to two, three, or more sequences contained in a regulatory region of the polyglutamine disease-related gene, wherein each gRNA specifically binds to a different target sequence provided in Table 7. In a preferred embodiment, the gRNAs specifically bind to:
(a) SEQ ID NOs: 246, 256 and 264;
(b) SEQ ID NOs: 248, 264 and 259;
(c) SEQ ID NOs: 246 and 264; or
(d) SEQ ID NOs: 248 and 264.

**Table 7: Target sequences found in the promoter/enhancer region of HTT**

| **ID** | **Target.sequence** | **Genomic.location** | **Strand** | **Region** | **SEQ ID NO** |
|---|---|---|---|---|---|
| HTT_74 | TCTGGGACGCAAGGCGCCGT | chr4:3074657 | + | Region 2 | 246 |
| HTT_78 | CGGGACGGGTCCAAGATGGA | chr4:3074686 | + | Region 2 | 256 |
| HTT_96 | TGGGACGCAAGGCGCCGTGG | chr4:3074659 | + | Region 2 | 248 |
| HTT_169 | GCGGGTCCCAGGCTACGGCG | chr4:3075122 | + | Region 2 | 335 |
| HTT_232 | CAGCACCGGGGCAATGAATG | chr4:3074743 | - | Region 2 | 264 |
| HTT_272 | GGCGCAGCGTCTGGGACGCA | chr4:3074648 | + | Region 2 | 244 |
| HTT_428 | AGGTAAAAGCAGAACCTGAG | chr4:3074709 | - | Region 2 | 259 |
| HTT_436 | AGGTTCTGCCTCACACAGCA | chr4:3074508 | - | Region 2 | 209 |
| HTT_550 | AGCAGCGGCTGTGCCTGCGG | chr4:3074988 | - | Region 2 | 303 |
| HTT_687 | GGCTGCGGCTGAGGCAGCAG | chr4:3075003 | - | Region 2 | 305 |
| HTT_722 | GGCGGCTGAGGAAGCTGAGG | chr4:3074967 | - | Region 2 | 298 |

In any of the above embodiments, the composition comprises two, three or more gRNAs (or one or more nucleic acid encoding the gRNAs) that specifically bind to two, three, or more sequences contained in the poly(CAG) tract of the polyglutamine disease-related gene. These gRNAs may specifically bind to two, three or more sequences selected from Table 8.

In some embodiments, the polyglutamine disease-related gene is *HTT.* Exemplary target sequences are provided in Table 8. In some embodiments, the gRNA that specifically binds to a sequence contained in the poly(CAG) tract of *HTT* specifically binds to a sequence provided in Table 8. In some embodiments, the composition comprises two, three or more gRNAs (or one or more nucleic acid encoding the gRNAs) that specifically bind to two, three, or more sequences contained in the poly(CAG) tract of *HTT,* wherein each gRNA specifically binds to a different target sequence provided in Table 8.

**Table 8: Target sequences specific for the poly(CAG) tract**

| **ID** | **Target.sequence** | **Strand** | **SEQ ID NO** |
|---|---|---|---|
| F1 | CAGCAGCAGCAGCAGCAGCA | + | 596 |
| F2 | AGCAGCAGCAGCAGCAGCAG | + | 597 |
| F3 | GCAGCAGCAGCAGCAGCAGC | + | 598 |
| R1 | CTGCTGCTGCTGCTGCTGCT | - | 599 |
| R2 | TGCTGCTGCTGCTGCTGCTG | - | 600 |
| R3 | GCTGCTGCTGCTGCTGCTGC | - | 601 |

### Compositions comprising nucleic acids encoding at least two or more TRDs

In some embodiments, the composition of the present invention comprises one or more nucleic acid encoding at least a TRD that increases repressive histone modifications, a TRD that increases DNA methylation, and a polynucleotide-binding domain, wherein the TRDs are operably linked to the polynucleotide-binding domain when expressed, and wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In some embodiments, the composition of the present invention comprises one or more nucleic acid encoding at least a fusion protein comprising a TRD that increases repressive histone modifications, a TRD that increases DNA methylation, and a polynucleotide-binding domain, wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In some embodiments, the TRD that increases repressive histone modifications increases histone methylation. In some embodiments, the TRD that increases repressive histone modifications is a KRAB domain. In some embodiments, the TRD that increases DNA methylation is DNMT3L or a DNA methyltransferase (e.g., DNMT3A). In some embodiments, the TRD that increases DNA methylation is DNMT3L. In some embodiments, DNMT3L is fused to a histone fragment that recruits an endogenously expressed DNA methyltransferase.

In some embodiments, the composition of the present invention comprises one or more nucleic acid encoding at least a KRAB domain, DNMT3L, and a polynucleotide-binding domain, wherein the KRAB domain and DNMT3L are operably linked to the polynucleotide-binding domain when expressed, and wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In some embodiments, the composition of the present invention comprises one or more nucleic acid encoding at least a fusion protein comprising a KRAB domain, DNMT3L, and a polynucleotide-binding domain, wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In some embodiments, the composition of the present invention comprises one or more nucleic acid encoding at least a KRAB domain, DNMT3A, DNMT3L, and a polynucleotide-binding domain, wherein the KRAB domain, DNMT3A and DNMT3L are operably linked to the polynucleotide-binding domain when expressed, and wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In some embodiments, the composition of the present invention comprises one or more nucleic acid encoding at least a fusion protein comprising a KRAB domain, DNMT3L, DNMT3A and a polynucleotide-binding domain, wherein:
(a) the polynucleotide-binding domain is a DNA-binding domain (e.g., a ZFP or TALE) that specifically binds to a sequence contained in a polyglutamine disease-related gene, or
(b) the polynucleotide-binding domain is a dCas protein and the composition further comprises:
   (i) a gRNA that specifically binds to a sequence contained in a polyglutamine disease-related gene, or
   (ii) a nucleic acid encoding a gRNA that specifically binds to a sequence contained in a polyglutamine disease-related gene.

In any embodiment of the invention, the DNMT3A domain may be replaced with a histone fragment that recruits an endogenous DNA methyltransferase, such as a histone H3 tail. Embodiments involving a histone fragment may, in some instances, not comprise a DNA methyltransferase such as DNMT3A. Embodiments involving a histone fragment may, in some instances, not comprise a DNA methyltransferase and/or a KRAB domain. Compositions encoding for a histone fragment that recruits an endogenous DNA methyltransferase may not need to encode a further DNA methyltransferase such as DNMT3A because such compositions can rely on the endogenously produced DNA methyltransferases instead. Thus, in some embodiments, the composition does not comprise a nucleic acid encoding a DNA methyltransferase.

In any of the above embodiments, the fusion protein may further comprise one or more linker between one or more domain. For example, a fusion protein of the present invention may be in accordance with the following structure:
N-DNMT3A- Linker-DNMT3L-Linker-PBD-Linker-KRAB-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-H3-linker-DNMT3L-Linker-PBD-Linker-KRAB-C,
   wherein the N indicates the N-terminus, the C indicates the C-terminus, H3 is a histone H3 tail, and PBD is a polynucleotide-binding domain (e.g., ZFP or dCas protein). Alternatively, a fusion protein of the present invention may be in accordance with the following structure:
N-H3-linker-DNMT3L-Linker-PBD-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-DNMT3L-Linker-PBD-Linker-KRAB-C,
   wherein the N indicates the N-terminus, the C indicates the C-terminus, and PBD is a polynucleotide-binding domain (e.g., ZFP or dCas protein). Alternatively, a fusion protein of the present invention may be in accordance with the following structure:
N-DNMT3A-linker-DNMT3L-Linker-PBD-C,
   wherein the N indicates the N-terminus, the C indicates the C-terminus, and PBD is a polynucleotide-binding domain (e.g., ZFP or dCas protein).

In any embodiment of the present invention, one or more nucleic acid may encode one or more fusion protein comprising, from N-terminus to C-terminus, SEQ ID NO: 110 or a sequence having at least 70, 75, 80, 85, 90, 95, 97, 98 or 99% sequence identity to SEQ ID NO: 110, a polynucleotide-binding domain, and SEQ ID NO: 111 or a sequence having at least 70, 75, 80, 85, 90, 95, 97, 98 or 99% sequence identity to SEQ ID NO: 111. The polynucleotide-binding domain may, for example, be a ZFP or dCas protein.

In some embodiments, the polyglutamine disease-related gene is *HTT.* In some embodiments, the polyglutamine disease-related gene is *ATXN2.* The DNA-binding domain or gRNA may be in accordance with any one of the DNA-binding domains or gRNAs disclosed above in the section entitled *"Compositions targeting the regulatory region and the poly(CAG) tract".*

### Targeting poly(CAG) tracts with dCas and gRNAs

In some embodiments, the composition of the present invention comprises:
(a) one or more nucleic acid encoding at least one or more TRD and a dCas protein, wherein the one or more TRD is operably linked to the dCas protein when expressed,
(b) a gRNA that specifically binds to a sequence contained in the poly(CAG) tract of a polyglutamine disease-related gene, or a nucleic acid encoding the gRNA.

In some embodiments, the composition of the present invention comprises:
(a) one or more nucleic acid encoding at least a fusion protein comprising one or more TRD and a dCas protein,
(b) a gRNA that specifically binds to a sequence contained in the poly(CAG) tract of a polyglutamine disease-related gene, or a nucleic acid encoding the gRNA.

In some embodiments, the polyglutamine disease-related gene is *HTT.* In some embodiments, the polyglutamine disease-related gene is *SCA2.* In some embodiments, the gRNA that specifically binds to a sequence contained in the poly(CAG) tract of *HTT* specifically binds to a target sequence provided in Table 8. In some embodiments, the composition comprises two, three or more gRNAs (or one or more nucleic acid encoding the gRNAs) that specifically bind to two, three, or more sequences contained in the poly(CAG) tract of *HTT*, wherein each gRNA specifically binds to a different target sequence provided in Table 8.

The engineered transcription repressor constructs may be in accordance with any embodiment disclosed in the present application including those embodiments in any previous section of the present application.

### Expression vectors and pharmaceutical compositions

The different encoded components may be present on a single polynucleotide chain or may be provided in two or more separate polynucleotide chains. For example, a nucleic acid encoding a gRNA may be present on the same polynucleotide chain as the one or more nucleic acid encoding the polynucleotide-binding domain and/or one or more TRD or on separate polynucleotide chains. The expression of any one or more of the nucleic acids involves at least one suitable promoter known in the art.

In some embodiments, the one or more nucleic acid of the composition is comprised in one or more mRNA, or one or more viral vector.

The one or more mRNA may be formulated in a suitable delivery vehicle such as a lipid nanoparticle (see, for example, Tuma et al., 2023. Biochemistry. 62(24):3533-3547), or polymer-based carrier (see, for example, Yang et al., 2023. Adv Healthc Mater. 12(15):e2202688).

A viral vector may be a non-replicating viral vector or replicating viral vector. The viral vector is preferably non-replicating. For example, the viral vector may be a recombinant adeno-associated virus (AAV) such as AAV9 or AAV-PHP.eB (see, for example, Kimura & Harashima, 2022. Neural Regen Res. 17(4): 785-787).

Compositions of the present invention may allow a pathogenic allele of a polyglutamine-disease related gene to be downregulated for 20 or more days (e.g., 30 or more, 35 or more, 40 or more, 45 or more, or 50 or more days). Such compositions can be delivered using available mRNA technology without needing frequent administration.

In some embodiments, the compositions of the present invention downregulate the expression of a pathogenic allele of a polyglutamine disease-related gene for a longer number of days than a fusion protein comprising (i) the same DNA-binding domain and a human KOX1 KRAB domain, or (ii) the same dCas protein (which is complexed to the same gRNA) and a human KOX1 KRAB domain when delivered into a cell using an mRNA.

The compositions of the present invention may be pharmaceutical compositions. Pharmaceutical compositions may further comprise a pharmaceutically acceptable carrier and/or pharmaceutically acceptable diluent.

### Uses of the compositions

In a further aspect, any one of the compositions of the present invention is for use as a medicament. The present invention provides a method of administering a composition of the present invention to a subject. For example, the invention provides a method of treating a subject having a polyglutamine disease, the method comprising administering a therapeutically effective amount of any one of the compositions of the present invention to the subject.

The present invention also provides a method of reducing the expression of a pathogenic allele of a polyglutamine disease-related gene, the method comprising contacting a biological system with a composition of the present invention. The biological system may be a cell, organoid, organ or subject. When the biological system is a cell or organoid, the method may be performed *ex vivo.*

In some embodiments, any one of the compositions of the present invention can be used to treat a polyglutamine disease. The polyglutamine disease may be any one of the diseases provided in Table 1. Targeting any one of the polyglutamine disease-related genes in Table 1 allows for the treatment of the disease indicated in the same row. In some embodiments, the polyglutamine disease is Huntington's disease and the polyglutamine disease-related gene is *HTT.*

In some embodiments, the subject is classified as belonging to Stage 0, Stage 1, or Stage 2 of the Huntington's Disease Integrated Staging System (HD-ISS). The HD-ISS characterizes individuals for research purposes from birth, starting at Stage 0 (i.e., individuals with the Huntington's disease genetic mutation without any detectable pathological change) by using a genetic definition of Huntington's disease. Huntington's disease progression is then marked by measurable indicators of underlying pathophysiology (Stage 1), a detectable clinical phenotype (Stage 2), and then decline in function (Stage 3). Individuals can be precisely classified into stages based on thresholds of stage-specific landmark assessments (see Tabrizi et al., 2022. Lancet Neurol. 21(7):632-644). Without being bound to a particular theory, treating subjects at an earlier stage of disease progression will allow for more effective therapies.

In another embodiment, the polyglutamine disease is a Spinocerebellar Ataxia and the polyglutamine disease-related gene is *ATXN1*, *ATXN2*, *ATXN3*, *CACNA1A*, *ATXN7* or *TBP.* In some embodiments, the polyglutamine disease is Spinocerebellar Ataxia Type 2 and the polyglutamine disease-related gene is *ATXN2.*

In some embodiments, the polypeptides (and, optionally, one or more gRNA) encoded by the one or more nucleic acid are expressed transiently (e.g., for 1 to 14, 15, 16, 17, 18, 19, 20, 21, 22, 23, 24, 25, 26, 27, 28, 29 or 30 days after administration of the composition). In some embodiments, expression cannot be measured, 15, 16, 17, 18, 19, 20, 21, 22, 23, 24, 25, 26, 27, 28, 29 or 30 days after administration of the composition of the invention.

The composition of the present invention can be administered via any suitable route (see, for example, Zhou et al., 2022. Front Mol Neurosci. 15: 988914). For example, a composition of the present invention can be administered via intrathecal injection, intravenously, intranasally or intracranially.

### Incorporation by reference

The entire contents of all references (including literature references, issued patents, published patent applications, and co-pending patent applications) cited throughout this application are hereby expressly incorporated by reference, in particular for the teaching that is referenced hereinabove.

### Examples

The present invention is further illustrated by the following Examples, which in no way should be construed as further limiting.

### Example 1: Bi-allelic silencing of HTT by epi-silencing using dCas protein

To rapidly assess epi-silencing activity of different types of epi-editors, we developed a HEK 293T cell line that faithfully reports for *HTT* expression. To this end, we exploited CRISPR-Cas9 to insert the tdTomato fluorescent transgene into the last coding exon of the *HTT* gene **(****Figure 1A**, **B**). After the gene targeting procedure, we generated single cell clones from the tdTomato-positive cell population and functionally validated targeted insertion in one of these clones (namely SCCE11) by disrupting the *HTT* gene with CRISPR-Cas9 **(****Figure 1C****).** This cell line is hereafter referred to as HEK 293T *HTT^{tdTomato}.*

We then developed an unbiased library approach to identify highly effective gRNAs for *HTT* epi-silencing. First, we selected the *HTT* regions to be targeted by the gRNA library. **Figure 2** shows the 5' of the *HTT* locus with the regions investigated in the screening experiment, which are represented as boxes below the schematic of the gene (TSS: Transcription Start Site of the *HTT* gene). A total of 1925 SpCas9 gRNAs were designed to recognize two CpG Islands (CGIs; region 1 and 2), one of which overlapping with the promoter/proximal enhancer of the *HTT* gene (n= 878 for region 1, n=708 for region 2, gRNAs), and two putative downstream regulatory elements (grey boxes; n=445 gRNAs). gRNAs predicted not to bind to the human genome (non-targeting gRNAs; n=100) were included in the final library as controls.

The LV-pooled screening was then performed as shown in **Figure 3**. Briefly, a Lentiviral Vector (LV) library expressing the BFP marker together with the 1925 gRNAs against *HTT* or the 100 control guides was prepared and used to transduce, at low Multiplicity of Infection (MOI), the HEK 293T *HTT^{tdTomato}* cell line. LV-positive cells were sorted according to BFP expression and then transfected with mRNA encoding for the tri-partite epi-editor DNMT3A:DNMT3L-dCas9-KRAB (abbreviated as 3A:h3L-dCas9-K). Twenty-one days after transfection, the BFP-positive, tdTomato-negative cells were sorted, and their genomic DNA extracted. Complexity of the gRNAs was then assessed by targeted deep sequencing (>300x coverage) followed by bioinformatic analysis (with the MAGeCK tool). HEK 293T *HTT^{tdTomato}* cells transduced with the LV library but not transfected with the epi-editor were used to control for input library complexity.

The results of this screening are reported in the dot plot of **Figure 4****,** which shows the normalized log₂ Fold-Change (log₂FC, Y axis) of the sequencing counts of each gRNA between cells treated or not with the epi-editor. X axis depicts position of the gRNA overlayed with the 4 investigated regions (representation on-scale with the *HTT* gene). Enriched gRNAs are in the area highlighted in grey. The highest gRNAs enrichment was observed at region 2, which coincides with the promoter/proximal enhancer of the *HTT* gene. The 11 top-performing gRNAs (black dots) from this region were chosen for further validation experiments and are provided in **Table 7.** To this end, we co-transfected HEK 293T *HTT^{tdTomato}* cells with the mRNA encoding for the tri-partite epi-editor (at 1mg dose) and the indicated gRNAs (1mg each) and then longitudinally monitored the cells for expression of *HTT^{tdTomato}* until day 21. At this time-point, we observed a wide range of epi-silencing, with 9 out of the 11 tested gRNAs being able to impose >10% of *HTT* epi-silenced cells **(****Figure 5****;** Y axis: percentage of HTT-negative cells). Top 6 gRNAs (indicated with filled-in bars in the histogram) were selected for further studies.

We then asked if gRNA combination can increase epi-silencing efficiency over individual gRNAs. To this end, we co-transfected *HTT^{tdTomato}* cells with triple gRNA combinations (2mg of each gRNA combination, in a 1:1:1 molar ratio among gRNAs) and the mRNA encoding for the tri-partite epi-editor (2 mg). Thirty-two days post treatment, we measured the percentage of *HTT^{tdTomato}*-negative cells *per* treatment condition by flow cytometry (data reported in the histogram of **Figure 6A****).** This experiment uncovered improved epi-silencing activity of several gRNA combinations, allowing to achieve >70% of *HTT^{tdTomato}*-negative cells in all treatments. We also asked if reduced gRNA combinations were beneficial for *HTT* epi-silencing. We thus co-transfected HEK 293T *HTT^{tdTomato}* cells with all possible double combinations of the 6 top-performing gRNAs from the validation experiment in **Figure 5** together with the mRNA encoding for the tri-partite epi-editor and assessed the percentage of *HTT^{tdTomato}*-negative by flow cytometry at day 16 post-treatment. The heatmap in **Figure 6B** shows that several gRNA pairs outperformed their parental counterparts (i.e., individual gRNAs, reported in the diagonal of the map), with clear indications of synergistic activity (e.g., 80.8% vs. 37.5% vs. 13.4% of *HTT^{tdTomato}*-negative cells for the pair gRNA _232+gRNA _96 vs. gRNA _232 alone vs. gRNA_96 alone, among others).

Based on the above results, we then tested epi-silencing of *HTT* in human primary fibroblasts from a healthy donor. To this end, we co-transfected the cells with 4 mg of the mRNA encoding for the dCas9-based tri-partite epi-editor and the 11 top-performing gRNAs from the screening. Genetic disruption of the *HTT* gene by conventional CRISPR-Cas9 or cells treated with the tri-partite epi-editor and an *HTT*-unrelated gRNA were used as controls. Eight days post-transfection, total mRNA was extracted from the cells and the levels of the *HTT* transcript were measured by RT-qPCR. The histogram in **Figure 8A** shows the levels of the *HTT* transcripts in the indicated conditions relative to untreated (UT) cells. These data show that all but one of the selected gRNAs were able to induce robust inhibition of *HTT* expression, at levels that were comparable or significantly higher than those obtained with conventional gene editing by CRISPR-Cas9.

We also assessed epi-silencing efficiency of *HTT* in iPSC-derived Neural Stem Cells (NSCs) from a healthy donor. To this end, NSCs were transfected with 4 mg of RNA containing the dCas9-based tri-partite epi-editor and the indicated gRNAs (in a 1:1 weight-to-weight ratio), either alone or in combination. Genetic disruption of the *HTT* gene by conventional CRISPR-Cas9 or cells treated with the tri-partite epi-editor and an *HTT*-unrelated gRNA were used as controls. Ten days post-transfection, cells were lysed and the *HTT* transcripts were measured using RT-qPCR. The histogram in **Figure 8B** shows the relative expression levels of *HTT* in treated *versus* untreated (UT) cells. Remarkably, all gRNA tested induced high levels of *HTT* repression, with gRNA combinations outperforming individual gRNAs. In this regard, all but one of the gRNA combinations resulted in nearly absent *HTT* expression, while the three individual gRNAs tested induced up to 75% reduction in *HTT* expression, a level comparable to gene disruption by CRISPR-Cas9.

Altogether, these data show that transient expression of epi-editors can lead to efficient and durable silencing of *HTT* in both primary fibroblasts and NSCs.

It is possible to expand the repertoire of gRNA to include other gRNAs from the initial screen and to test further double and triple gRNA combinations. These and the other gRNAs already tested can be applied with other architectures, such as bi-partite designs including, for example, KRAB and DNMT3L but no DNMT3A.

A similar cell line can be generated using *ATXN2* instead of *HTT* and gRNAs can be designed and identified as for *HTT.* This cell line can be named HEK 293T *ATXN2^{tdTomato}* and used to screen for suitable gRNAs that target the promoter/enhancer. These gRNAs could then also be tested in primary fibroblasts and/or NSCs using the methods above.

### Example 2: Bi-allelic silencing of HTT by epi-silencing using ZFPs

We designed a panel of 31 ZFP arrays targeting the promoter/enhancer region of *HTT* **(****Figure 7****, Table 4).** These ZFPs can be used to develop epi-editors according to the present invention. Efficacy of these reagents can be tested by transfecting HEK 293T *HTT^{tdTomato}* cells. Combinations of these ZFPs can also be tested.

A ZFP array targeting the promoter/enhancer region of *ATXN2* can also be generated tested using HEK 293T *ATXN2^{tdTomato}* cells.

### Example 3: Allele-selective epi-silencing using dCas protein

To assess the allele-selective epi-silencing activity of different types of epi-editors, we developed a panel of HEK 293T cell lines that report for the co-expression of the wild-type (WT) and the pathogenic expanded *HTT* alleles. To this end, by performing a two steps gene targeting procedure with TALENs and CRISPR-Cas9, we inserted pathogenic expansions (i.e., 30, 45, 65 or 81 CAG repetitions) and the tdTomato fluorescent transgene in one *HTT* allele and the Wasabi fluorescent transgene into the WT *HTT* allele, which harbors an 18 CAG repetition. A schematic of the cell lines engineering is reported in **Figure 9****.** These lines are named according to the length of the pathogenic expansion as: HEK-293T *HTT^{WT-Wasbi}*/*HTT^{30Q-tdTomato}*, HEK-293T *HTT^{WT-Wasbi}*/*HTT^{45Q-tdTomato}*, HEK-293T *HTT^{WT-Wasbi}*/*HTT^{65Q-tdTomato}* and HEK-293T *HTT^{WT-Wasbi}*/*HTT^{81Q-tdTomato}.* A line expressing tdTomato and Wasabi from WT alleles was also generated (referred as to the *HTT^{WT-Wasbi}*/*HTT^{WT-tdTomato}* cells).

To target the CAG expansion with CRISPR-dCas9 based epi-editors, we designed 3 gRNAs *per* DNA strand, each shifting of 1 nucleotide with respect to the others **(Table 8).** As some of these gRNAs lack a canonical SpCas9 PAM, we also built catalytically deactivated tri-partite epi-editors based of the near PAM-less SpG and SpRY SpCas9 variants (Walton et al., 2020. Science. 368(6488):290-296). We then co-transfected HEK-293T *HTT^{WT-Wasbi}*/*HTT^{81Q-tdTomato}* cells with a non-saturating dose of plasmids encoding for the editors and the individual gRNAs and measured epi-silencing efficiency of *HTT* by flow cytometry. The histogram in **Figure 10A** shows the percentage of single and double negative cells at day 16 post-treatment. Remarkably, at least one gRNA *per* SpCas9 variant showed selectivity towards the pathogenic expanded allele. Representative flow cytometry dots plots are shown in **Figure 10B****.** Overall, these data show, for the first time to our knowledge, that transient expression of epi-editors based on dCas9-based DBDs can lead to efficient and durable allele-selective silencing of pathogenically expanded *HTT* alleles.

Combinations of the best-performing gRNAs can be tested in this and other reporter cell lines. Different epi-editor architectures, including other all-in-one (i.e., bi- and tri-partite) as well as individual (i.e., double and triple combinations) designs, can also be effective. Genome wide analyses of cells treated with the best-performing epi-editors uncovers the most specific ones.

Similar cell lines can be generated for *ATXN2* and used to screen the identified gRNAs.

### Example 4: Allele-selective epi-silencing using ZFPs

We then designed 28 ZFP-based tri-partite epi-editors targeting the CAG expansion **(Table 5).** We tested allele-selectivity of these epi-editors in the HEK-293T *HTT^{WT-Wasbi}*/*HTT^{81Q-tdTomato}* cell line, transfecting a non-saturating dose of plasmid encoding for the epi-editors. **Figure 11A** shows the percentage of single or double negative cells for each ZFP-based epi-editor at day 16 post-treatment, as measured by flow cytometry. Several of the tested epi-editors showed an allelic-preference for the pathogenic expanded allele (i.e., higher percentage of tdTomato- vs. Wasabi-negative cells). Based on these results, we performed a validation experiment in which we assessed the epi-silencing activity of the tri-partite epi-editor equipped with ZFP_17 in *HTT^{WT-Wasbi}*/*HTT^{WT-tdTomato}* and in the two cell lines with pathogenic expansions, namely HEK-293T *HTT^{WT-Wasbi}*/*HTT^{65Q-tdTomato}* and HEK-293T *HTT^{WT-Wasbi}*/*HTT^{81Q-tdTomato}* **(****Figure 12****).** The cells were transfected with increasing doses of the mRNA encoding for the epi-editor and analyzed by flow cytometry 20 days post-transfection, when KRAB-mediated effects were absent. In HEK-293T *HTT^{WT-Wasbi}*/*HTT^{WT-tdTomato}* cells, the tri-partite epi-editor induced indiscriminate residual silencing of both alleles, with up to 9% of silencing of the pathogenic expanded allele and halved silencing of the WT allele. Up to 4% of the treated cells displayed bi-allelic silencing. On the other end, silencing activity of the epi-editor was markedly allele-specific in HEK-293T *HTT^{WT-Wasbi}*/*HHT^{65Q-tdTomato}* and HEK-293T *HTT^{WT-Wasbi}*/*HTT^{81Q-tdTomato}* cells. In these cell models, up to 60 and 80% of silencing of the pathogenic expanded *HTT* alleles was measured, with a range of 7-13% of bi-allelic silencing. Interestingly, no silencing was observed only at the WT allele in these two cell models at any epi-editor's dose. Overall, these data show, for the first time to our knowledge, that transient expression of epi-editors based on ZFP DBDs can lead to efficient and durable allele-selective silencing of pathogenically expanded *HTT* alleles.

Other tri-partite and bi-partite epi-editors' architectures, as well as double and triple epi-editors' combinations can be applied in this and in the other reporter cell lines. Genome wide analyses of cells treated with the best-performing epi-editors uncovers the most specific ones.

Similar cell lines can be generated for *ATXN2* and used to test the identified ZFPs.

### Example 5: combining allele-specific and bi-allelic epi-silencing

To further improve efficacy and specificity of allele-selective epi-silencing of *HTT* and *ATXN2,* we reasoned to combine promoter/enhancer and expansion targeting epi-editors, an approach that may benefit from the synergistic activity of the epi-editing platform. To this end, one can follow two strategies: (1) targeting of the pathogenic expansion with the top-performing epi-editors identified above and the promoter/enhancer with an epi-editor with minimal - if not absent - long-term epi-silencing activity; (2) targeting the promoter/enhancer and the pathogenic expansion with poorly active epi-editors, thus exploiting the synergistic characteristic of the epi-editing platform. By testing both strategies in *HTT* and *ATXN2* reporter cell lines, one can identify combinations able to impose efficient and durable gene silencing.

## Claims

1. A composition comprising one or more nucleic acid encoding at least a transcriptional repressor domain (TRD) and a polynucleotide-binding domain, wherein the TRD is operably linked to the polynucleotide-binding domain when expressed, and wherein:
(a) the polynucleotide-binding domain is a DNA-binding domain that specifically binds to a sequence contained in a polyglutamine disease-related gene, or
(b) the polynucleotide-binding domain is a dCas protein and the composition further comprises a gRNA that specifically binds to a sequence contained in a polyglutamine disease-related gene, or a nucleic acid encoding said gRNA.

2. The composition of claim 1, wherein the DNA-binding domain or gRNA specifically binds to:
(a) a sequence contained in the poly(CAG) tract of the polyglutamine disease-related gene; or
(b) a sequence contained in a regulatory region of the polyglutamine disease-related gene.

3. The composition of claim 1 or 2, wherein the TRD is a KRAB domain, DNMT3A or DNMT3L.

4. The composition of claim 3, wherein the TRD is a DNMT3L and the DNMT3L is also operably linked to a histone fragment when expressed, and wherein the histone fragment is capable of recruiting an endogenous DNA methyltransferase.

5. The composition of claim 1 or 2, wherein the one or more nucleic acid encodes two or more TRDs that are operably linked to the polynucleotide-binding domain when expressed, and wherein one or more TRD increases repressive histone modifications and one or more TRD increases DNA methylation.

6. The composition of claim 5, wherein the TRD that increases repressive histone modifications is a KRAB domain and the TRD that increases DNA methylation is DNMT3A or DNMT3L.

7. The composition of claim 6, wherein the TRD that increases DNA methylation is DNMT3L, wherein, optionally, the DNMT3L is also operably linked to a histone fragment when expressed, wherein the histone fragment is capable of recruiting an endogenously expressed DNA methyltransferase.

8. The composition of any one of claims 3-7, wherein the one or more nucleic acid further encodes DNMT3A, and DNMT3A is operably linked to the polynucleotide-binding domain when expressed.

9. The composition of any one of claims 1-8, wherein the one or more nucleic acid encodes at least a fusion protein comprising the polynucleotide-binding domain and the one or more TRD.

10. The composition of any one of claims 1-9, wherein the DNA-binding domain or gRNA specifically binds to a sequence contained in the poly(CAG) tract of the polyglutamine disease-related gene, and wherein:
(a) the one or more nucleic acid further encodes one or more additional TRD and a second polynucleotide-binding domain, wherein the one or more additional TRD is operably linked to the second polynucleotide-binding domain when expressed, and wherein the second polynucleotide-binding domain is a DNA-binding domain that specifically binds to a sequence contained in a regulatory region of the polyglutamine disease-related gene; and/or
(b) the composition further comprises a second gRNA that specifically binds to a sequence contained in a regulatory region of the polyglutamine disease-related gene or a nucleic acid encoding said gRNA.

11. The composition of claim 10(a), wherein the additional TRD is a KRAB domain, DNMT3A or DNMT3L.

12. The composition of claim 11, wherein the additional TRD is a DNMT3L and the DNMT3L is also operably linked to a histone fragment that recruits an endogenous DNA methyltransferase when expressed.

13. The composition of claim 10(a), wherein two or more additional TRDs are operably linked to the second polynucleotide-binding domain when expressed, and wherein one of the TRDs increases repressive histone modifications and one or more of the TRDs increases DNA methylation.

14. The composition of claim 13, wherein the additional TRD that increases repressive histone modifications is a KRAB domain and the additional TRD that increases DNA methylation is DNMT3A or DNMT3L.

15. The composition of claim 14, wherein the additional TRD that increases DNA methylation is DNMT3L, wherein, optionally, the DNMT3L is also operably linked to a histone fragment when expressed, wherein the histone fragment is capable of recruiting an endogenously expressed DNA methyltransferase.

16. The composition of any one of claims 11-15, wherein the one or more nucleic acid further encodes DNMT3A, and DNMT3A is operably linked to the second polynucleotide-binding domain when expressed.

17. The composition of any one of claims 10-16, wherein the one or more nucleic acid encodes at least:
(a) a fusion protein according to claim 9; and
(b) a second fusion protein comprising the second polynucleotide-binding domain and the one or more additional TRD.

18. The composition of any one of claims 1-3 and 10, wherein the one or more nucleic acid encodes:
(a) a fusion protein comprising a KRAB domain and dCas protein;
(b) a fusion protein comprising DNMT3L and a dCas protein; and/or
(c) a fusion protein comprising DNMT3A and a dCas protein.

19. The composition of any one of claims 1-18, wherein the DNA-binding domain is a zinc finger protein (ZFP) or a Transcription Activator-like Effector (TALE).

20. The composition of any one of claims 1-19 for use as a medicament.

21. The composition of any one of claims 1-19 for use in the treatment of a polyglutamine disease.

22. The composition for use of claim 21, wherein the polyglutamine disease-related gene is *HTT* and the polyglutamine disease is Huntington's disease.

23. The composition for use of claim 21, the polyglutamine disease-related gene is *ATXN1*, *ATXN2, ATXN3*, *CACNA1A*, *ATXN7*, or *TBP,* and the polyglutamine disease is Spinocerebellar ataxia.

24. The composition for use of claim 23, wherein the polyglutamine disease-related gene is *ATXN2* and the polyglutamine disease is Spinocerebellar ataxia Type 2.

25. The composition for use of any one of claims 20-24, wherein the composition is administered via intrathecal injection, intravenously, intranasally or intracranially.

26. An *ex vivo* method of reducing the expression of a pathogenic allele of a polyglutamine disease-related gene, the method comprising contacting a cell or organoid with the composition of any one of claims 1-19.
